(19) Europäisches Patentamt
European Patent Office
Office européen des brevets

(11) Veröffentlichungsnummer: **0 268 148 B1**

# (12) EUROPÄISCHE PATENTSCHRIFT

(45) Veröffentlichungstag der Patentschrift: **11.12.91**

(21) Anmeldenummer: **87116251.7**

(22) Anmeldetag: **04.11.87**

(51) Int. Cl.5: **C07D 233/64**, C07D 235/14, C07D 471/04, C07D 277/62, C07D 243/14, C07D 487/04, A61K 31/415, A61K 31/425, A61K 31/44, A61K 31/55

## (54) Tetrahydronaphthalinderivate und diese enthaltende Arzneimittel.

(30) Priorität: **14.11.86 CH 4565/86**

(43) Veröffentlichungstag der Anmeldung:
**25.05.88 Patentblatt 88/21**

(45) Bekanntmachung des Hinweises auf die Patenterteilung:
**11.12.91 Patentblatt 91/50**

(84) Benannte Vertragsstaaten:
**AT BE CH DE ES FR GB GR IT LI LU NL SE**

(56) Entgegenhaltungen:
**EP-A- 0 177 960**
**CH-A- 587 830**

**THE JOURNAL OF ORGANIC CHEMISTRY, Band 44, Nr. 5, 1979 E.J. FRONEFELD; A.J. PIKE "Cycloaddition Reaction with Methylnitrone" Seiten 835-839**

(73) Patentinhaber: **F. HOFFMANN-LA ROCHE AG**
**Postfach 3255**
**CH-4002 Basel(CH)**

(72) Erfinder: **Branca, Quirico, Dr.**
**Lenzgasse 2**
**CH-4056 Basel(CH)**
Erfinder: **Jaunin, Roland, Prof. Dr.**
**Lehenmattstrasse 308**
**CH-4052 Basel(CH)**
Erfinder: **Märkl, Hans Peter, Dr.**
**Leimenstrasse 45**
**CH-4051 Basel(CH)**
Erfinder: **Marti, Fränzi, Dr.**
**Baselstrasse 50**
**CH-4125 Riehen(CH)**
Erfinder: **Ramuz, Henri, Prof. Dr.**
**Rheinparkstrasse 3**
**CH-4127 Birsfelden(CH)**

(74) Vertreter: **Lederer, Franz, Dr. et al**
**Patentanwalt Dr. Franz Lederer Lucile-Grahn-Strasse 22**
**W-8000 München 80(DE)**

EP 0 268 148 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Tetrahydronaphthalinderivate. Im speziellen betrifft sie Tetrahydronaphthalinderivate der allgemeinen Formel

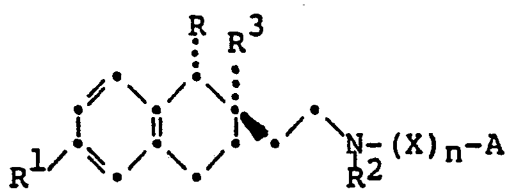

I

worin R nieder-Alkyl, $R^1$ Halogen, $R^2$ $C_1$-$C_{12}$-Alkyl,$R^3$ Hydroxy, nieder-Alkoxy, nieder-Alkylcarbonyloxy, nieder-Alkoxy-nieder-alkylcarbonyloxy, nieder-Alkylaminocarbonyloxy, Arylaminocarbonyloxy oder Aryl-nieder-alkylaminocarbonyloxy, X $C_1$-$C_{18}$-Alkylen, welches gegebenenfalls durch 1,4-Phenylen unterbrochen oder durch 1,4-Cyclohexylen unterbrochen oder verlängert sein kann, A 2-Benzimidazolyl, 1-Methyl-2-benzimidazolyl, 1-Dodecyl-2-benzimidazolyl, Benzimidazolonyl, 3-Methylbenzimidazolonyl, 3-Isopropylbenzimidazolonyl, 3-Butylbenzimidazolonyl, 3-Morpholinoäthylbenzimidazolonyl, 3-Benzylbenzimidazolonyl, 2-Pyridylmethylbenzimidazolonyl, 2-Imidazo-(4,5-c)pyridinyl, Imidazo(4,5-c)pyridinonyl, 2-Benzthiazolyl, 2,3,4,5-Tetrahydro-4-methylbenzodiazepin-2,5-dion-1-yl, 6-Chlor-2,3,11,11a-tetrahydro-pyrrolo(2,1-c)(1,4)-benzodiazepin-5,11-dion-10-yl, 5,6-Dimethyl-2-benzimidazolyl, 1-Methyl-4,5-diphenyl-2-imidazolyläthyl oder 4,5-Diphenyl-2-imidazolyläthyl und n die Zahl 0 oder 1 bedeuten,
in Form von Racematen und optischen Antipoden, sowie N-Oxyde und pharmazeutisch verwendbare Säureadditionssalze davon.

In EPA 0.177.960 sind Tetrahydronaphtalinderivate mit einer Phenylalkylmethylaminoalkylseitenkette in 2-Stellung und deren calciumantagonistische Eigenschaften bekannt. Die anmeldegemässen Verbindungen unterscheiden sich durch den Ersatz des Phenylrestes in der Seitenkette durch heterocyclische Reste sowie gegebenenfalls durch die Verlängerung der Kette und Einfügung von Phenylen- oder Cyclohexylengruppen als Kettenglieder in signifikanter Weise von den vorbekannten Verbindungen.

Diese Verbindungen sind neu und Zeichnen sich durch wertvolle pharmakodynamische Eigenschaften aus.

Gegenstand der Vorliegenden Erfindung sind Verbindungen der allgemeinen Formel I, N-Oxyde und pharmazeutisch verwendbare Säureadditionssalze davon als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der allgemeinen Formel I, N-Oxyden und pharmazeutisch verwendbaren Säureadditionssalzen davon bei der Bekämpfung bzw. Verhütung von Krankheiten bzw. bei der Verbesserung der Gesundheit, insbesondere bei der Bekämpfung bzw. Verhütung von Angina pectoris, Ischämie, Arrhythmien, Bluthochdruck und Herzinsuffizienz.

Der in der vorliegenden Beschreibung verwendete Ausdruck "nieder-Alkyl" - allein oder in Kombination - bedeutet geradkettige und verzweigte, gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sek.-Butyl, tert.-Butyl und dergleichen. Der Ausdruck "$C_1$-$C_{12}$-Alkyl" betrifft in ähnlicher Weise Alkylgruppen, worin der Alkylrest 1-12 Kohlenstoffatome aufweist. Der Ausdruck "nieder-Alkoxy" bedeutet nieder-Alkyläthergruppen, worin der Ausdruck "nieder-Alkyl" die obige Bedeutung hat. Der Ausdruck "Halogen" umfasst die vier Halogenatome Fluor, Chlor, Brom und Jod. Der Ausdruck "$C_1$-$C_{18}$-Alkylen" bezeichnet geradkettige oder verzweigte, gesättigte Reste mit 1-18 Kohlenstoffatomen, wie Methylen, Aethylen, Propylen, Methyläthylen, Butylen, 1,1-Dimethylpropylen, Pentamethylen, 1-Methylpentamethylen, Hexamethylen, Heptamethylen, Undecamethylen und dergleichen. Der Ausdruck "Aryl" bezeichnet gegebenenfalls durch Halogen, Trifluormethyl, nieder-Alkyl, nieder-Alkoxy, Nitro oder Amino ein- oder mehrfach substituiertes Phenyl. Der Ausdruck "Aryl-nieder-alkyl" bezeichnet geradkettige oder verzweigte nieder-Alkylgruppen, worin ein oder mehrere Wasserstoffatome durch Arylgruppen ersetzt sind, wie Benzyl, Phenäthyl und dergleichen. Der Ausdruck "Abgangsgruppe" bedeutet bekannte Gruppen mit Halogen, vorzugsweise Chlor oder Brom, Arylsulfonyloxy, wie etwa Tosyloxy, Brombenzolsulfonyloxy, Benzolsulfonyloxy oder Mesitylensulfonyloxy, oder Alkylsulfonyloxy, wie etwa Mesyloxy oder Trifluormethylsulfonyloxy.

Der Ausdruck "pharmazeutisch verwendbare Säureadditionssalze" umfasst Salze mit anorganischen oder organischen Säuren, wie Salzsäure, Bromwasserstoffsäure, Salpetersäure, Schwefelsäure, Phosphorsäure, Citronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Bevorzugt sind solche Verbindungen der Formel I, worin R Isopropyl bedeutet. $R^3$ bedeutet vorzugsweise Hydroxy, nieder-Alkylcarbonyloxy, besonders bevorzugt Isobutyryloxy, nieder-Alkoxy-nieder-alkylcarbonyloxy, besonders bevorzugt Methoxyacetyloxy, oder nieder-Alkylaminocarbonyloxy, besonders bevorzugt Butylaminocarbonyloxy. n bedeutet vorzugsweise die Zahl 1. Weiter sind solche Verbindungen der Formel I bevorzugt, worin $R^1$ Fluor bedeutet. Auch bevorzugt sind solche Verbindungen der Formel I, worin $R^2$ Methyl bedeutet. Ebenfalls bevorzugt sind die Verbindungen der Formel I, worin X $C_3$-$C_7$-Alkylen, besonders bevorzugt Propylen, Butylen, Pentamethylen oder Hexamethylen, bedeutet. A bedeutet vorzugsweise 2-Benzimidazolyl, 2-Benzthiazolyl, 1-Methyl-2-benzimidazolyl, 1-Dodecyl-2-benzimidazolyl, Benzimidazolonyl, 2,3,4,5-Tetrahydro-4-methylbenzodiazepin-2,5-dion-1-yl, 6-Chlor-2,3,11,11a-tetahydro-pyrrolo[2,1-c]-[1,4]benzodiazepin-5,11-dion-10-yl oder 1-Methyl-4,5-diphenyl-2-imidazolyl, besonders bevorzugt 2-Benzimidazolyl oder 2-Benzthiazolyl.

Aus dem obigen folgt, dass solche Verbindungen der Formel I ganz besonders bevorzugt sind, worin R Isopropyl, $R^3$ Hydroxy, Isobutyryloxy, Methoxyacetyloxy oder Butylaminocarbonyloxy, $R^1$ Fluor, $R^2$ Methyl, X Propylen, Butylen, Pentamethylen oder Hexamethylen, A 2-Benzimidazolyl oder 2-Benzthiazolyl und n die Zahl 1 bedeuten.

Ganz speziell bevorzugte Verbindungen der Formel I sind:

2-[2-[[3-(2-Benzimidazolyl)propyl]methylamino]äthyl]-6-fluor-1,2,3,4-tetrahydro-1α-isopropyl-2α-naphthylmethoxyacetat,

[1S,2S]-2-[2-[[5-(2-Benzthiazolyl)pentyl]methylamino]äthyl]-6-fluor-1,2,3,4-tetrahydro-1-isopropyl-2-naphthylmethoxyacetat und

[1S,2S]-2-[2-[[3-(2-Benzimidazolyl)propyl]methylamino]äthyl]-6-fluor-1,2,3,4-tetrahydro-1-isopropyl-2-naphthylmethoxyacetat.

Die Verbindungen der Formel I in Form von Racematen und optischen Antipoden, sowie N-Oxyde und pharmazeutisch verwendbare Säureadditionssalze davon können hergestellt werden, indem man

a) zur Herstellung von Verbindungen der Formel I, worin $R^3$ Hydroxy oder nieder-Alkoxy bedeutet und die übrigen Symbole die oben angegebene Bedeutung besitzen, eine Verbindung der allgemeinen Formel

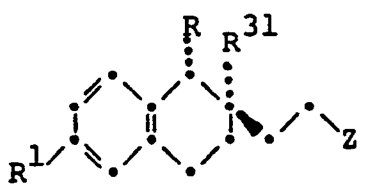

II

worin $R^{31}$ Hydroxy oder nieder-Alkoxy und Z eine Abgangsgruppe bedeuten und R und $R^1$ die oben angegebene Bedeutung besitzen,
mit einem Amin der allgemeinen Formel

$$\begin{array}{l} HN-(X)_n-A \\ \;| \\ R^2 \end{array} \qquad III$$

worin $R^2$, A, X und n die oben angegebene Bedeutung besitzen,
umsetzt, oder

b) zur Herstellung von Verbindungen der Formel I, worin $R^3$ nieder-Alkylcarbonyloxy oder nieder-Alkoxy-nieder-alkylcarbonyloxy bedeutet und die übrigen Symbole die oben angegebene Bedeutung besitzen, eine Verbindung der allgemeinen Formel

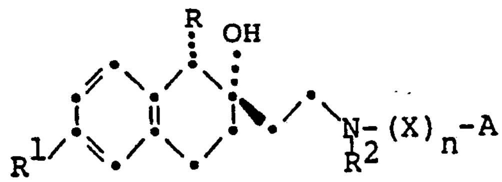

Ia

worin R, $R^1$, $R^2$, A, X und n die oben angegebene Bedeutung besitzen, mit einem eine nieder-Alkylcarbonyl-oder nieder-Alkoxy-nieder-alkylcarbonylgruppe abgebenden Acylierungsmittel umsetzt, oder

c) zur Herstellung von Verbindungen der Formel I, worin $R^3$ nieder-Alkylaminocarbonyloxy, Arylaminocarbonyloxy oder Aryl-nieder-alkylaminocarbonyloxy bedeutet und die übrigen Symbole die oben angegebene Bedeutung besitzen, eine Verbindung der obigen Formel Ia mit einem nieder-Alkyl-, Aryl-oder Aryl-nieder-alkyl-isocyanat umsetzt, und erwünschtenfalls

d) eine erhaltene Verbindung zum entsprechenden N-Oxydoxydiert, und/oder

e) ein erhaltenes Racemat in die optischen Antipoden auftrennt, und/oder

f) eine erhaltene Verbindung in ein pharmazeutisch verwendbares Säureadditionssalz überführt.

Eine Verbindung der Formel II wird nach an sich bekannten Methoden mit einem Amin der Formel III umgesetzt. Die Umsetzung erfolgt in Gegenwart oder Abwesenheit eines unter den Reaktionsbedingungen inerten organischen Lösungsmittels bei einer Temperatur zwischen etwa 20° und 150°C, vorzugsweise zwischen etwa 80° und 120°C. Bei dieser Umsetzung kommen Lösungsmittel, wie Dimethylformamid, Dimethylsulfoxyd, Alkohole, wie Isopropanol oder tert.-Butanol, Aether, wie Tetrahydrofuran oder Dioxan, aromatische Kohlenwasserstoffe, wie Benzol, Toluol oder Xylol, chlorierte Kohlenwasserstoffe, wie Methylenchlorid, Tetrachlorkohlenstoff oder Chlorbenzol, und dergleichen in Frage. Die Umsetzung erfolgt vorteilhafterweise in Gegenwart eines säurebindenden Mittels, wie eines tertiären Amins, wie Trimethylamin, Triäthylamin, Aethyldiisopropylamin oder 1,5-Diazabicyclo[4.3.0]non-5-en, wobei auch überschüssiges Amin der Formel III als säurebindendes Mittel dienen kann. Aus Zweckmässigkeitsgründen arbeitet man bei Atmosphärendruck, obwohl höhere Drucke ebenfalls angewendet werden können.

Die Acylierung einer Verbindung der Formel Ia erfolgt ebenfalls nach an sich bekannten Methoden. Geeignete Acylierungsmittel sind insbesondere aktivierte Säurederivate, wie Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0°C und der Rückflusstemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage.

Auch die Umsetzung einer Verbindung der Formel Ia mit einem Isocyanat erfolgt nach an sich bekannten Methoden in einem unter den Reaktionsbedingungen inerten organischen Lösungsmittel oder Lösungsmittelgemisch bei einer Temperatur zwischen etwa 50°C und dem Siedepunkt des Lösungsmittels bzw. Lösungsmittelgemisches, vorzugsweise zwischen etwa 80 und 120°C, in Gegenwart eines Katalysators, wie eines Zinn-(II)-salzes, z.B. Zinn-(II)-2-äthylhexanoat. Als Lösungsmittel kommen insbesondere aromatische Kohlenwasserstoffe, wie Benzol, Toluol oder Xylol, Aether wie Tetrahydrofuran oder Dioxan, und dergleichen in Frage.

Eine erhaltene Verbindung kann ebenfalls in an sich bekannter Wiese mittels eines Oxydationsmittels, wie Wasserstoffperoxyd oder einer Persäure, wie Peressigsäure oder Perbenzoesäure, in einem Lösungsmittel, wie einem Alkanol, z.B. Methanol oder Aethanol, und dergleichen bei einer Temperatur zwischen etwa 0° und 50°C, vorzugsweise bei Raumtemperatur, in das entsprechende N-Oxyd übergeführt werden.

Die Ausgangsstoffe der Formeln II und III sind bekannt oder können in Analogie zur Herstellung der bekannten Verbindungen erhalten werden. Je ein Verfahren zur Herstellung einer Verbindung der Formel III, worin A einen über ein Stickstoffatom bzw. ein Kohlenstoffatom gebundenen Heterocyclus bzw. di- oder trisubstituiertes, über eine Aethylengruppe gebundenes 2-Imidazolyl bedeutet, ist in den nachfolgenden Schemata I-III, worin Boc tert.-B utoxycarbonyl, Bz Benzyl und Ph Phenyl bedeuten, skizziert. Bezüglich der genauen Reaktionsbedingungen wird auf den Beispielteil verwiesen.

Schema I

$H_2N-(CH_2)_6-OH$ IV

$Boc-NH-(CH_2)_6OH$ V

$Boc-NH-(CH_2)_6-OSO_2CH_3$ VI

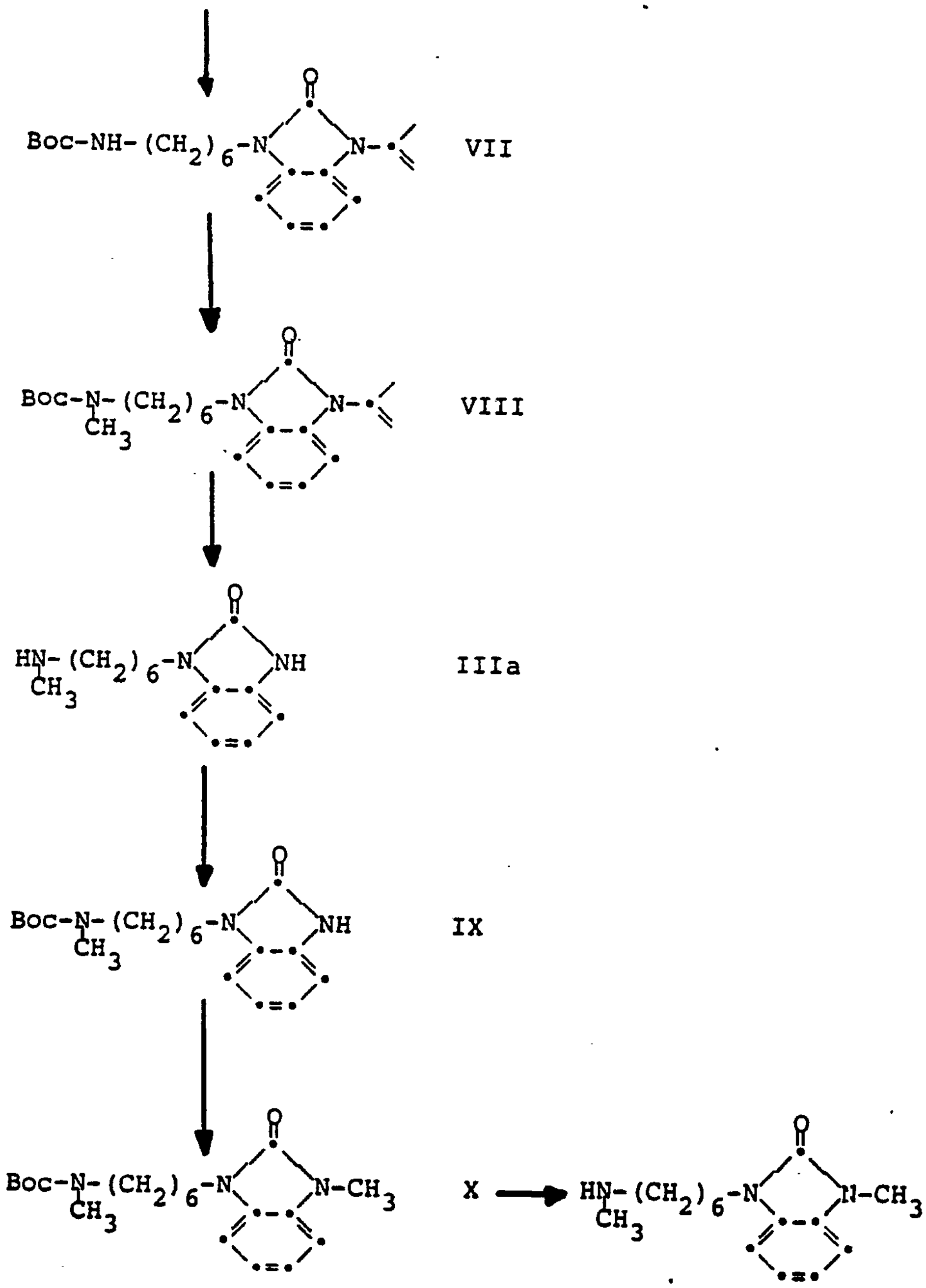

VII

VIII

IIIa

IX

X

IIIb

Schema II

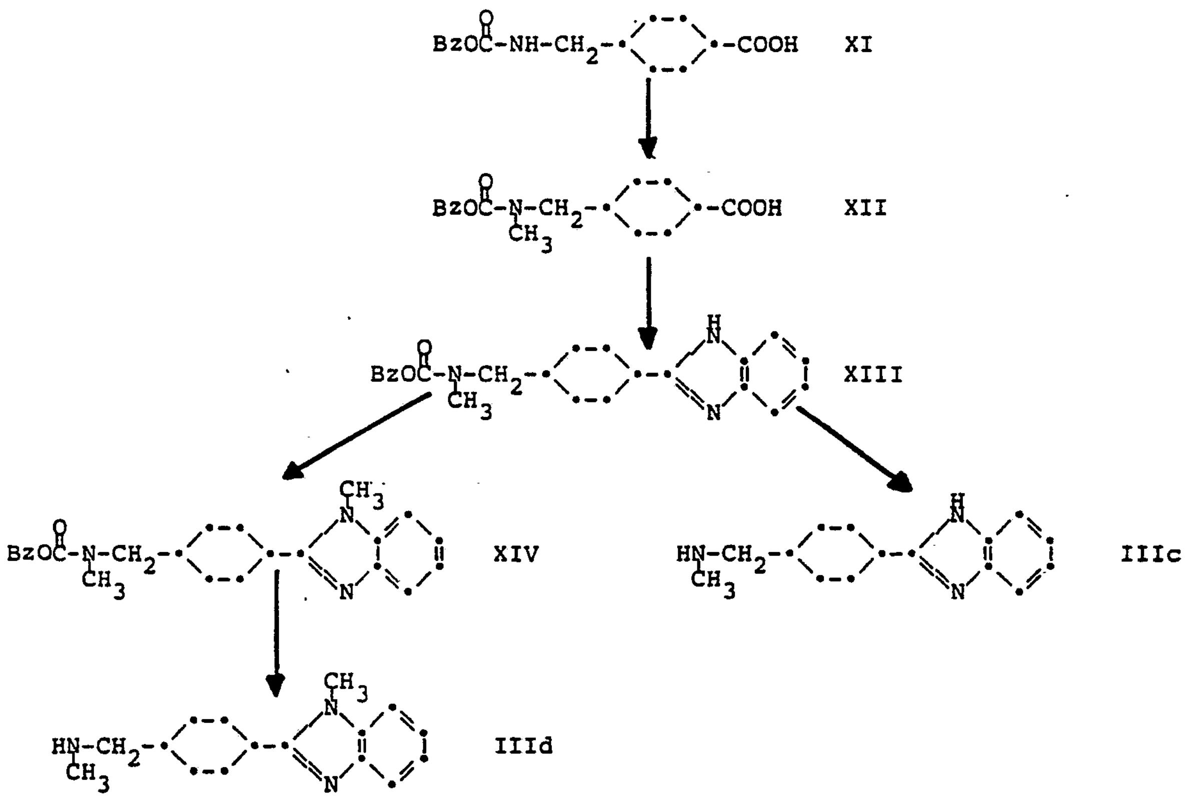

XI

XII

XIII

XIV

IIIc

IIId

Schema III

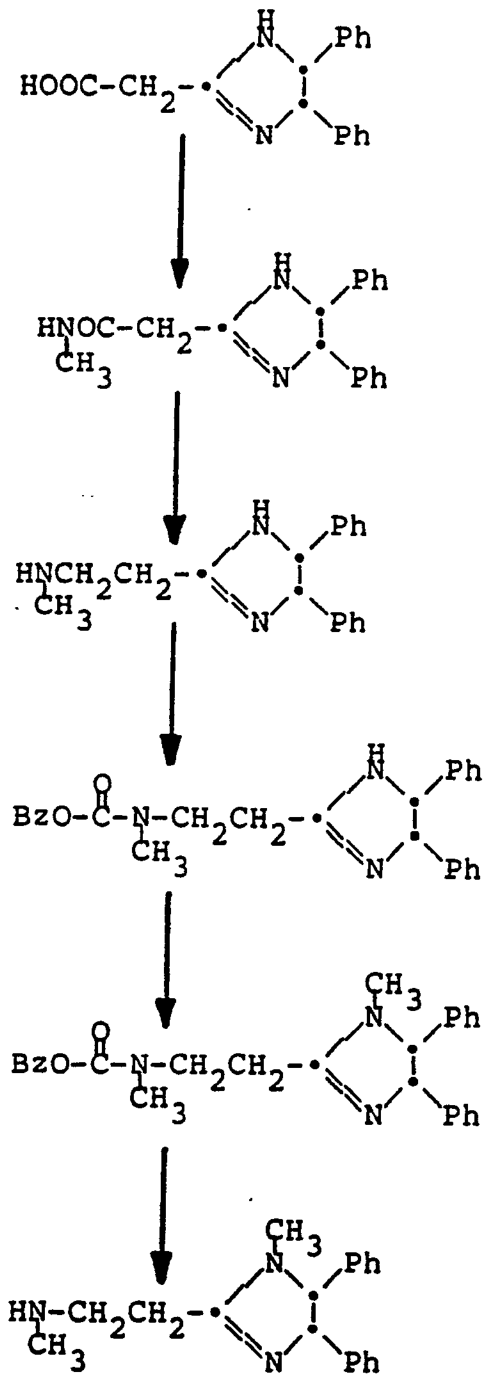

XV

XVI

IIIe

XVII

XVIII

IIIf

Die Verbindungen der allgemeinen Formel I enthalten mindestens ein asymmetrisches Zentrum (2-Stellung) und können deshalb als optische Antipoden oder als Racemate vorliegen. Verbindungen der Formel I, die mehr als ein asymmetrisches Zentrum enthalten, liegen in der durch die Formel I angegebenen relativen Konfiguration vor. Die Racemate der Formel I können nach an sich bekannten Methoden, z.B. durch Umsetzen mit einer optisch aktiven Säure und fraktionierte Kristallisation des erhaltenes Salzes in die optischen Antipoden aufgetrennt werden.

Die Verbindung der Formel I besitzen eine ausgeprägte Calcium-antagonistische Wirkung und können

deshalb als Arzneimittel verwendet werden, insbesondere für die Bekämpfung bzw. Verhütung von Angina pectoris, Ischämie, Arrhythmien, Bluthochdruck und Herzinsuffizienz.

Die Calcium-antagonistische Wirkung sowie die blutdrucksenkenden Eigenschaften der erfindungsgemässen Verbindungen können in den nachstehend beschriebenen Tests gezeigt werden:

A. $^3$H-Desmethoxyverapamil-Bindungsbestimmungen:

Die Bestimmung wird an partiell-gereinigten Membranen vom Meerschweinchen-Herz durchgeführt. Die Reaktionsmischung (0,3 ml) besteht aus 0,2-0,8 mg Membranprotein, 2,5 nM $^3$H-Desmethoxyverapamil und verschiedenen Konzentrationen der Prüfsubstanzen. Die Inkubation dauert 120 Minuten bei 37° C und wird durch Verdünnung mit dem Inkubationspuffer gestoppt; anschliessend erfolgt eine Filtration. Die filtergebundene Radioaktivität wird mit einem Szintillationszähler gemessen. Spezifische Bindung (d.h. rezeptorgebundene) wird als die Differenz zwischen total- und unspezifisch-gebundener Radioaktivität definiert. Die unspezifische Bindung wird in Gegenwart von einem Ueberschuss nichtradioaktiven Verapamils (10 μM) bestimmt.

Die Wirksamkeit (Potenz) einer Verbindung in diesem Test wird durch den $IC_{50}$-Wert definiert. $IC_{50}$ ist die Substanzkonzentration (in Mol/l), die eine halb-maximale Inhibition der spezifischen $^3$H-Desmethoxyverapamil-Bindung verursacht. Dieser Wert wird aus einer Konzentration-Bindungs-Kurve extrapoliert.

B. Isoliertes, perfundiertes Meerschweinchen-Herz nach Langendorff:

Meerschweinchen mit einem Gewicht von ungefähr 400 g werden mit Urethan (1 g/kg i.p.) betäubt, und das Herz wird rasch entfernt. Die Aorta wird kannuliert, und das Herz wird retrograd mit einer modifizierten Krebs-Henseleit-Lösung folgender Zusammensetzung in mM perfundiert: NaCl 114,7, KCl 4,7, $MgSO_4$ 1,2, $KH_2PO_4$ 1,5, $NaHCO_3$ 25, $CaCl_2$ 2,5 und Glukose 11,1. Die Lösung wird mit Oxycarbon (Gemisch von 95% Sauerstoff und 5% Kohlendioxyd) bei pH 7,3 und einer Temperatur von 37° C begast. Der Perfusionsdruck wird auf einem Wert von 90 cm $H_2O$ (8,83 kPa) konstantgehalten. Ein Miller-Mikrotipkatheter-Drucktransducer (PC-350) zur Messung des linksventrikulären Drucks wird in die linke Herzkammer eingeschoben. Der gesamte Koronararterienfluss wird in einen Trichter gesammelt und mit einem elektromagnetischen Flussmeter gemessen. Alle Messparameter werden auf einem Registrierapparat aufgezeichnet (Gould, Modell 2800). Nach einer 45-minütigen Adaptierung beginnt der Versuch. Substanzen werden mit einer Geschwindigkeit von 1% der gesamten Koronarflussrate infundiert. Eine komplette Konzentrations-Wirkungskurve ($10^{-10}$ bis $10^{-6}$ M) wird für jede Substanz erstellt. Die beiden wichtigsten Messparameter sind: (1) CBF: Coronary Blood Flow (in ml/min) - die Blutflussgeschwindigkeit durch die Koronararterien und (2) dp/dt: Rate of increase in left ventricular pressure (in mmHg/sec) - die Anstiegsgeschwindigkeit des linksventrikulären Druckes, als Mass für die Kontraktilitätskraft des Herzens; dieser Wert wird als % maximale Aenderung vom Ausgangswert (Δ%) pro verabreichter Dosis angegeben.

C. Hämodynamische Parameter am narkotisierten Hund:

Die 4 wichtigsten Messparameter (mit resp. Messeinheiten) der hämodynamischen Versuche sind: (1) CBF: Coronary Blood Flow (in ml/min) - die Blutflussgeschwindigkeit durch die Koronararterien; (2) HR: Heart Rate (in Schläge/min) -die Herzfrequenz; (3) BP: Blood Pressure (in mmHg) - der Blutdruck; und (4) dp/dt; Rate of increase in left ventricular pressure (in mmHg/sec) - die Anstiegsgeschwindigkeit des linksventrikulären Druckes, als Mass für die Kontraktilitätskraft des Herzens. Die Werte werden als % maximale Aenderung vom Ausgangswert (Δ%) pro verabreichter Dosis angegeben.

Dadurch bekommt man nicht nur ein Gesamtbild der Substanzwirkung, sondern auch eine Abschätzung über die potentielle Selektivität für einen bestimmten Teil des Kreislaufsystems im ganzen Organismus. Nach Verabreichung eines Anästhetikums, wird der Hund intubiert und künstlich beatmet. Blut pH, $pCO_2$, $pO_2$ und Hämoglobin werden mit einem Blut-Gas-Analysator stündlich gemessen. Der Blutdruck (systolisch und diastolisch) wird mit einer Sonde in der Aorta abdominalis gemessen. Die Herzfrequenz wird mittels eines Tachometers erfasst, der vom Druckpuls ausgelöst wird. Für die anderen Messungen muss das Herz zuerst freigelegt werden, um eine Sonde in den linken Ventrikel (Herzkammer) für die Druckmessungen (dp/dt) einsetzen zu können. Der Coronarblutfluss wird mit einer Fluss-Sonde an der linken Coronararterie (descendens) gemessen.

Die in diesen Tests erhaltenen Resultate sind in der folgenden Tabelle zusammengefasst:

Tabelle

| Verbindung | A | B | | C | | | | |
|---|---|---|---|---|---|---|---|---|
| | $IC_{50}$ [M] | CBF $IC_{50}$ [M] | dp/dt Δ % | CBF Δ % | HR Δ % | BP Δ % | dp/dt Δ % | Dosis mg/kg i.v. |
| A | $1{,}3 \cdot 10^{-7}$ | $4{,}7 \cdot 10^{-8}$ | 250 | 86 | - 7 | -22 | 25 | 0,3 |
| B | $6{,}3 \cdot 10^{-7}$ | $5{,}0 \cdot 10^{-8}$ | 216 | 36 | 0 | - 6 | 8 | 0,3 |
| C | $2{,}1 \cdot 10^{-7}$ | $1{,}7 \cdot 10^{-8}$ | 192 | 62 | - 9 | -16 | 15 | 0,3 |
| D | $3{,}2 \cdot 10^{-8}$ | $1{,}1 \cdot 10^{-9}$ | 162 | 22 | -25 | -21 | 6 | 0,03 |
| E | $1{,}0 \cdot 10^{-6}$ | $1{,}0 \cdot 10^{-5}$ | 164 | 57 | 0 | - 8 | 10 | 1 |
| F | $3{,}4 \cdot 10^{-8}$ | $2{,}8 \cdot 10^{-8}$ | 130 | 46 | - 2 | - 3 | 4 | 0,3 |
| G | $1{,}5 \cdot 10^{-7}$ | $1{,}8 \cdot 10^{-9}$ | 237 | 96 | -15 | -20 | 11 | 0,3 |
| H | $2{,}8 \cdot 10^{-8}$ | $2{,}4 \cdot 10^{-9}$ | 222 | 146 | -41 | -28 | 25 | 0,3 |
| I | $1{,}0 \cdot 10^{-7}$ | $2{,}2 \cdot 10^{-8}$ | 124 | 82 | - 9 | -14 | 18 | 0,3 |

A = [1S,2S]-2-[2-[[3-(2-Benzimidazolyl)propyl]methylamino]äthyl] -6-fluor-1,2,3,4-tetrahydro-1-isopropyl-2-naphthylmethoxyacetat

B = [1S,2S]-2-[2-[[7-(2-Benzimidazolyl)heptyl]methylamino]äthyl]-6-fluor-1,2,3,4-tetrahydro-1-isopropyl-2-naphthalinol

C = [1S,2S]-6-Fluor-1,2,3,4-tetrahydro-1-isopropyl-2-[2-[methyl-[5-(1-methyl-2-benzimidazolyl)-pentyl]amino]äthyl]-2-naphthylbutylcarbamat

D = [1S,2S]-6-Fluor-1,2,3,4-tetrahydro-1-isopropyl-2-[2-[methyl-[6-(2-oxo-1 benzimidazolinyl)hexyl]-amino]äthyl]-2-naphthylmethoxyac etat

E = [1S,2S]-2-[2-[[3-(2-Benzimidazolyl)propyl]methyl-N-oxidoamino]äthyl] -6-fluor-1,2,3,4-tetrahydro-1-isopropyl-2-naphthylmethoxyacetat

F = [1S,2S]-2-[2-[[7-(1-Dodecyl-2-benzimidazolyl)heptyl]methylamino]äthyl]--fluor-1,2,3,4k-tetrahydro-1-isopropyl-2-naphthylmethox yacetat

G = [1S,2S]-6-Fluor-1,2,3,4-tetrahydro-1-isopropyl-2-[2-[[3-(1-methyl-4,5-d phenylimidazol-2-yl)-propyl]methylamino]äthyl]-2-naphthylmethoxyacetat

H = [1S,2S]-2-[2-[[5-(2-Benzthiazolyl)pentyl]methylamino]äthyl] -6-fluor-1,2,3,4-tetrahydro-1-isopropyl-2-naphthylmethoxyacetat

I = [1S,2S]-6-Fluor-1,2,3,4-tetrahydro-1-isopropyl-2-[2-[[4-[(2-benzimidazolyl) methyl]benzyl]-methylamino]äthyl]-2-naphthylmethoxyacetat

Die Verbindungen der Formel I können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verbindungen der Formel I mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man Verbindungen der allgemeinen Formel I bei der Bekämpfung bzw. Verhütung von Angina pectoris, Ischämie, Arrhythmien, Bluthochdruck und Herz-insuffizienz verwenden. Die

Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 25 bis 150 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

Beispiel 1

Ein Gemisch von 5,4 g (28,7 mMol) 2-[3-Methylamino)propyl]benzimidazol, 11,4 g (28,7 mMol) 2-(6-Fluor-1,2,3,4-tetrahydro-2-hydroxy-1α-isopropyl -2β-naphthyl)äthyl-p-toluolsulfonat und 3,74 g (28,7 mMol) Hünig-Base wird 30 Minuten auf 120° erhitzt. Das Gemisch wird darauf auf Eiswasser gegossen und mit Methylenchlorid extrahiert. Nach dem Trocknen der organischen Phase über Magnesiumsulfat wird das Lösungsmittel abgedampft, und der Rückstand an Silicagel mit einem 6:1-Gemisch von Methylenchlorid und Methanol als Eluierungsmittel chromatographiert. Man erhält dabei 6,2 g (49%) [1S,2S]-2-[2-[[3-(2-Benzimidazolyl)propyl]methylamino]äthyl] -6- fluor-1,2,3,4-tetrahydro-1-isopropyl-2-napthalinol,

$$[\alpha]^{20}_{589} = = +41,2°$$

(c= 0,8%; Methanol).

Das als Ausgangsmaterial eingesetzte 2-[3-(Methylamino)propyl]benzimidazol wurde wie folgt hergestellt:

22,8 g (91 mMol) 4-[1-(Benzyloxy)-N-methylformamido]buttersäure werden in 200 ml Tetrahydrofuran gelöst. Das Gemisch wird abgekühlt, und 13 ml (128 mMol) Triäthylamin und 12 ml (91,5 mMol) Chlorameisensäureisobutylester werden bei -15° zugetropft. Nach 2,5 Stunden werden 10,3 g (95 mMol) o-Phenylendiamin in 85 ml Tetrahydrofuran bei -10° innerhalb von 30 Minuten zugegeben. Nach 1-stündigem Rühren bei Raumtemperatur wird das Lösungsmittel unter vermindertem Druck abgedampft. Darauf wird Wasser zugegeben und mit Essigester extrahiert. Die organische Phase wird mit gesättigter wässriger Natriumbicarbonatlösung und gesättigter wässriger Kochsalzlösung gewaschen. Nach dem Trocknen über Magnesiumsulfat und Abdampfen des Lösungsmittels erhält man 27,05 g eines Rohrprodukts, welches an Silicagel mit Essigester als Eluierungsmittel chromatographiert wird. Dabei erhält man 20,1 g (71%) Benzyl [3-[(2-aminophenyl)carbamoyl]propyl]methylcarbamat.

MS: $M^+$ 341.

20,1 g (59 mMol) Benzyl [3-[(2-aminophenyl)-carbamoyl]propyl]methylcarbamat werden in 450 ml Toluol gelöst und mit 7 g (37 mMol) p-Toluolsulfonsäure versetzt. Das Reaktionsgemisch wird danach während 2 Stunden zum Rückfluss erhitzt, wobei das gebildete Wasser mittels eines Wasserabscheiders aus dem Reaktionsgemisch entfernt wird. Nach dem Eindampfen und Lösen des Rückstandes in Essigester wird zweimal mit gesättigter wässriger Natriumbicarbonatlösung und zweimal mit gesättigter wässriger Kochsalzlösung gewaschen. Die organische Phase wird über Magnesiumsulfat getrocknet und eingedampft. Chromatographie des Rohprodukts an Silicagel mit Essigester als Eluierungsmittel liefert 11 g (58%) Benzyl [3-(2-benzimidazolyl)propyl]methylcarbamat, Smp. 83-86°.

11,0 g (34 mMol) Benzyl [3-(2-benzimidazolyl)propyl]methylcarbamat werden in 150 ml Methanol in Gegenwart von 2,5 g Palladium auf Kohle (5%ig) als Katalysator mit Wasserstoff reduziert. Dabei erhält man 5,45 g (85%) 2-[3-(Methylamino)propyl]benzimidazol, Smp. 134-136°.

Beispiel 2

6,2 g (14,6 mMol) [1S,2S]-2-[2-[[3-(2-Benzimidazolyl)propyl]methylamino]äthyl] -6-fluor-1,2,3,4-tetrahydro-1--isopropyl-2-naphthalinol werden in 50 ml Chloroform gelöst. Dazu gibt man bei 0° 2,5 ml (15 mMol) N-Aethyldiisopropylamin und 5 ml (55 mMol) Methoxyacetylchlorid. Das Reaktionsgemisch wird über Nacht bei Raumtemperatur gerührt und danach mit 100 ml IN Natronlauge versetzt und mit Chloroform extrahiert. Nach dem Trocknen über Magnesiumsulfat und Abdampfen des Lösungsmittels wird der Rückstand an Silicagel mit einem 6:1-Gemisch von Methylenchlorid und Methanol chromatographiert. Dabei werden 6,2 g eines Oels erhalten, welche in 30 ml Aethanol gelöst und mit 15 ml mit Chlorwasserstoffsäure gesättigtem Aether versetzt werden. Darauf wird das Reaktionsgemisch eingedampft, und der Rückstand aus Aethanol/Diäthyläther kristallisiert. Man erhält dabei 5,4 g (65%) [1S,2S]-2-[2-[[3-(2-Benzimidazolyl)-

propyl]methylamino]äthyl] -6-fluor-1,2,3,4-tetrahydro-1-isopropyl -2-naphthylmethoxyacetat-dihydrochlorid, Smp. 128°.

Beispiel 3

Ein Gemisch von 4,2 g (10,35 mMol) 2-(6-Fluor-1,2,3,4-tetrahydro-2-hydroxy-1α-isopropyl -2β-naphthyl)-äthyl-p-toluolsulfonat und 4,5 g (20,7 mMol) 2-[5-(Methylamino)-pentyl]benzimidazol wird 30 Minuten auf 100° erhitzt. Danach werden zunächst 100 ml Chloroform, dann nach dem Abkühlen 100 ml Aether und schliesslich 100 ml 1N wässrige Salzsäure zugegeben. Nach 30-minütigem Rühren des Reaktionsgemisches wird mit konzentrierter wässriger Natronlauge basisch gestellt, und die organische Phase abdekantiert, getrocknet und eingedampft. Nach dem Chromatographieren an Silicagel mit einem 6:1-Gemisch von Methylenchlorid und Methanol erhält man 2,7 g (58,2%) [1S,2S]-2-[2-[[5-(2-Benzimidazolyl)pentyl]-methylamino]äthyl] -6-fluor-1,2,3,4-tetrahydro-1-isopropyl-2-naphthalinol,

$$[\alpha]^{20}_{589} = = +36,8°$$

(c= 0,25; Methanol).

Beispiel 4

6 g (13,2 mMol) [1S,2S]-2-[2-[[5-(2-Benzimidazolyl)pentyl]methylamino]äthyl] -6-fluor-1,2,3,4-tetrahydro-1-isopropyl-2-naphthalinol, 20 ml Methoxyessigsäureanhydrid und 1,05 g (13,3 mMol) Pyridin werden unter Rühren auf 70° erhitzt. Nach 2 Stunden wird abgekühlt und mit 500 ml 3N Natronlauge und 500 ml Methylenchlorid versetzt und kräftig gerührt. Die organische Phase wird über Magnesiumsulfat getrocknet und eingedampft. Der Rückstand wird in Aethanol gelöst und mit 16 ml mit Chlorwasserstoffsäure gesättigtem Aether versetzt. Nach dem Eindampfen und Kristallisieren aus Aethanol/Aether erhält man 6,2 g (78,5%) [1S,2S]-2-[2-[[5-(2-Benzimidazolyl)pentyl]methylamino]äthyl] -6-fluor-1,2,3,4-tetrahydro-1-isopropyl -2-naphthylmethoxyacetat-dihydrochlorid, Smp. 196-198°.

Beispiel 5

In analoger Weise wie in den Beispielen 1 und 3 beschrieben wurden die folgenden Verbindungen hergestellt:

- Ausgehend von 2-(6-Fluor-1,2,3,4-tetrahydro-2-hydroxy-1α-isopropyl -2β-naphthyl)äthyl-p-toluolsulfonat und 2-[4-(Methylamino)butyl]benzimidazol das [1S,2S]-2-[2-[[4 -(2-Benzimidazolyl)butyl]-methylamino]äthyl] -6-fluor-1,2,3,4-tetrahydro-1-isopropyl-2-naphthalinol, MS: $M^+$ 437;
- ausgehend von 2-(6-Fluor-1,2,3,4-tetrahydro-2-hydroxy-1α-isopropyl -2β-naphthyl)äthyl-p-toluolsulfonat und 2-[7-(Methylamino)heptyl]benzimidazol das [1S,2S]-2-[2-[[7 -(2-Benzimidazolyl)heptyl]-methylamino]äthyl] -6-fluor-1,2,3,4-tetrahydro-1-isopropyl-2-naphthalinol -dihydrochlorid,

$$[\alpha]^{20}_{589} = +32,9°$$

(c= 1%; Methanol);
- ausgehend von 2-(6-Fluor-1,2,3,4-tetrahydro-2-hydroxy-1α-isopropyl -2β-naphthyl)äthyl-p-toluolsulfonat und 2-[11-(Methylamino)undecyl]benzimidazol das [1S,2S]-2-[2-[[11 -(2-Benzimidazolyl)undecyl]-methylamino]äthyl]-6-fluor-1,2,3,4-tetrahydro-1-isopropyl-2-naphthalinol;
- ausgehend von 2-(6-Fluor-1,2,3,4-tetrahydro-2-hydroxy-1α-isopropyl -2β-naphthyl)äthyl-p-toluolsulfonat und 5,6-Dimethyl-2-[7-(methylamino)heptyl]benzimidazol das [1S,2S]-2-[2-[[7-(5,6-Dimethyl -2-benzimidazolyl)heptyl]methylamino]äthyl] -6-fluor-1,2,3,4-tetrahydro-1-isopropyl-2-naphthalinol,

$$[\alpha]^{20}_{589} = +33,6°$$

(c= 0,5%; Methanol);
- ausgehend von 2-(6-Fluor-1,2,3,4-tetrahydro-2-hydroxy--1α-isopropyl -2β-naphthyl)äthyl-p-toluolsulfonat und 2-[5-(Dodecylamino)pentyl]benzimidazol das [1S,2S]-2-[2-[[5 -(2-Benzimidazolyl)pentyl]-dodecylamino]äthyl] -6-fluor-1,2,3,4-tetrahydro-1-isopropyl-2-naphthalinol, MS: $M^+$ 606;

- ausgehend von 2-(6-Fluor-1,2,3,4-tetrahydro-2-hydroxy--1α-isopropyl -2β-naphthyl)äthyl-p-toluolsulfonat und 2-[7-(Methylamino)heptyl-1H-imidazo[4,5-c]pyridin das [1S,2S]-6-Fluor-1,2,3,4-tetrahydro-1-isopropyl-2-[2-[[7-(1H-imidazo[4,5-c]pyridin-2-yl)heptyl]methylamino]äthyl] -2-naphthalinol, MS: $M^+$ 480.

Die als Ausgangsstoffe eingesetzten Benzimidazolderivate wurden in analoger Weise wie in Beispiel 1 beschrieben hergestellt.

Beispiel 6

In analoger Weise wie in den Beispielen 2 und 4 beschrieben wurden durch Methoxyacetylierung der entsprechenden Hydroxyderivate die folgenden Verbindungen hergestellt:

- [1S,2S]-2-[2-[[4 -(2-Benzimidazolyl)butyl]methylamino]äthyl]-6-fluor -1,2,3,4-tetrahydro-1-isopropyl-2-naphthylmethoxyacetat-dihydrochlorid,

$$[\alpha]^{20}_{589} = +28,6°$$

(c= 1%; Methanol);

- [1S,2S]-2-[2-[[7 -(2-Benzimidazolyl)heptyl]methylamino]äthyl]-6-fluor -1,2,3,4-tetrahydro-1-isopropyl -2-naphthylmethoxyacetat-dihydrochlorid,

$$[\alpha]^{20}_{589} = +25,4°$$

(c= 1%; methanol);

- [1S,2S]-2-[2-[[11 -(2-Benzimidazolyl)undecyl]methylamino]äthyl]-6-fluor -1,2,3,4-tetrahydro-1-isopropyl -2-naphthylmethoxyacetat-dihydrochlorid,

$$[\alpha]^{20}_{589} = +23,7°$$

(c= 1%; Methanol);

- [1S,2S]-2-[2-[[7 -(5,6-Dimethyl-2-benzimidazolyl)heptyl]methylamino]äthyl] -6-fluor-1,2,3,4-tetrahydro-1-isopropyl -2-naphthylmethoxyacetat-hydrochlorid, (1:1,85),

$$[\alpha]^{20}_{589} = +26,5°$$

(c= 1%; Methanol);

- [1S,2S]-2-[2-[[5 -(2-Benzimidazolyl)pentyl]dodecylamino]äthyl] -6-fluor-1,2,3,4-tetrahydro-1-isopropyl -2-naphthylmethoxyacetat-dihydrochlorid,

$$[\alpha]^{20}_{589} = +22,0°$$

(c= 0,25%; Methanol);

- [1S,2S]-6-Fluor-1,2,3,4-tetrahydro-1-isopropyl -2-[2-[[7-(1H-imidazo[4,5-c]pyridin -2-yl)heptyl]methyl amino]äthyl]-2-naphthylmethoxyacetat -dihydrochlorid, Smp. 112-115°.

Beispiel 7

0,79 g (3,8 mMol) 2-[3-(Methylamino)propyl]benzthiazol, 1,54 g (3,8 mMol) 2-(6-Fluor-1,2,3,4-tetrahydro-2-hydroxy-1α-isopropyl-2β -naphthyl)äthyl]-p-toluolsulfonat und 0,49 g (3,8 mMol) Hünig-Base werden 2,5 Stunden bei 120° gerührt. Nach dem Abkühlen und Lösen des Niederschlags mit wenig Methylenchlorid wird die Reaktionslösung an Silicagel mit einem 12:1-Gemisch von Methylenchlorid und Methanol chromatographiert. Dabei erhält man 1,12 g (76%) [1S,2S]-2-[2-[[3-(2-Benzthiazolyl)propyl]methylamino]äthyl] -6-fluor-1,2,3,4-tetrahydro-1-isopropyl-2-naphthalinol, MS: $M^+$ 440.

In analoger Weise wie oben beschrieben wurden die folgenden Verbindungen hergestellt:

- Ausgehend von 2-(6-Fluor-1,2,3,4-tetrahydro-2-hydroxy-1α-isopropyl-2β -naphthyl)äthyl]-p-toluolsulfonat und 2-[5-(Methylamino)pentyl]benzthiazol das [1S,2S]-2-[2-[[5 -(2-Benzthiazolyl)pentyl]-methylamino]äthyl] -6-fluor-1,2,3,4-tetrahydro-1-isopropyl-2-naphthalinol, MS: $M^+$ 468;
- ausgehend von 2-(6-Fluor-1,2,3,4-tetrahydro-2-hydroxy-1α-isopropyl-2β -naphthyl)äthyl]-p-toluolsulfonat und 2-[7-(Methylamino)heptyl]benzthiazol das [1S,2S]-2-[2-[[7-(2-Benzthiazolyl)heptyl]-methylamino]äthyl] -6-fluor-1,2,3,4-tetrahydro-1-isopropyl-2-naphthalinol, MS: $M^+$ 496.

Das als Ausgangsstoff eingesetzte 2-[3-(Methylamino)propyl]benzthiazol wurde wie folgt hergestellt:

5,0 g (19,9 mMol) 4-[1-(Benzyloxy)-N-methylformamido]buttersäure werden in 175 ml Tetrahydrofuran gelöst. Zu der auf -20° gekühlten Lösung werden 2,95 ml (2,1 g; 24 mMol) Triäthylamin und 2,95 ml (22 mMol) Chlorameisensäureisobutylester gegeben. Das Reaktionsgemisch wird danach 1 Stunde bei dieser Temperatur gerührt. Dann werden 2,45 g (19,6 mMol) 2-Aminothiophenol zugegeben und das Reaktionsgemisch 20 Stunden bei Raumtemperatur gerührt. Darauf werden 250 ml Wasser zugegeben und wird mit Essigester extrahiert. Die organische Phase wird über Magnesiumsulfat getrocknet und unter vermindertem Druck eingedampft. Nach Chromatographie an Silicagel unter Verwendung eines 1:1-Gemisches von Essigester und Hexan erhält man 1,7 g (25,1%) Benzyl [3-(2-benzthiazolyl)propyl]methylcarbamat als Oel, MS: $M^+$ 340.

1,7 g (4,99 mMol) Benzyl [3-(2-benzthiazolyl)propyl]methylcarbamat werden in 40%igem Bromwasserstoff in Essigsäure bei 0° gelöst und 20 Stunden bei Raumtemperatur gerührt. Darauf werden 60 ml Aether zugegeben, und nach 1,5 Stunden der gebildete Niederschlag abfiltriert. Nach Waschen des kristallinen Niederschlags mit Aether und Trocknen erhält man 1,71 g (93,1%) 2-[3-(Methylamino)propyl]benzthiazol-dihydrobromid, Smp. 196-197°.

In analoger Weise wie oben beschrieben wurden die folgenden Verbindungen hergestellt:

- 2-[5-(Methylamino)pentyl]benzthiazol, MS: $M^+$ 234;
- 2-[7-(Methylamino)heptyl]benzthiazol, MS: $M^+$ 262.

Beispiel 8

1,12 g (2,54 mMol) [1S,2S]-2-[2-[[3-(2-Benzthiazolyl)propyl]methylamino]äthyl] -6-fluor-1,2,3,4-tetrahydro-1-isopropyl-2-naphthalinol werden in 0,2 g Pyridin gelöst. Dazu werden 5 ml Methoxyessigsäureanhydrid gegeben. Das Reaktionsgemisch wird 2 Stunden auf 60° erwärmt. Danach gibt man 100 ml 1N Natronlauge bei 0° zu und extrahiert mit 100 ml Essigester. Die organische Phase wird mit Magnesiumsulfat getrocknet, filtriert und unter vermindertem Druck eingedampft. Der Rückstand wird an Silicagel unter Verwendung eines 30:1-Gemisches von Methylenchlorid und Methanol chromatographiert. Man erhält dabei 0,9 g eines öligen Produktes, welches in Essigester gelöst und mit 2 ml mit Chlorwasserstoffsäure gesättigtem Aether versetzt wird. Nach dem Eindampfen auf 20 ml werden 40 ml Aether zugeführt und das Reaktionsgemisch 1 Stunde gerührt. Der ausgefallene Niederschlag wird abfiltriert und getrocknet. Man erhält dabei 0,9 g (64,5%) [1S,2S]-2-[2-[[3-(2-Benzthiazolyl)propyl]methylamino]äthyl] -6-fluor-1,2,3,4-tetrahydro-1-isopropyl -2-naphthylmethoxyacetat-dihydrochlorid, Smp. 130-134°.

In analoger Wiese wie oben beschrieben wurden durch Methoxyacetylierung der entsprechenden Hydroxyderivate die folgenden Verbindungen hergestellt:

- [1S,2S]-2-[2-[[5 -(2-Benzthiazolyl)pentyl]methylamino-äthyl] -6-fluor-1,2,3,4-tetrahydro-1-isopropyl -2-naphthylmethoxyacetat-hydrochlorid (5:8),

$$[\alpha]^{20}_{589} = +27{,}4°$$

(c = 0,5%; Methanol);

- [1S,2S]-2-[2-[[7 -(2-Benzthiazolyl)heptyl]methylamino]äthyl] -6-fluor-1,2,3,4-tetrahydro-1-isopropyl -2-napthylmethoxyacetat-hydrochlorid (4:5),

$$[\alpha]_{589}^{20} = +25,8°$$

(c = 1%; Methanol).

Beispiel 9

In analoger Weise wie in den Beispielen 1 und 4 beschrieben wurde ausgehend von der (S)-6-[1-(Benzyloxy)-N-methylformamido]heptansäure via das [1S,2S]-2-[2-[[(S)-5-(2-Benzimidazolyl)-1-methylpentyl]methylamino]äthyl]-6-fluor-1,2,3,4-tetrahydro-1-isopropyl-2-naphthalinol das [1S,2S]-2-[2-[[(S)-5-(2-Benzimidazolyl)-1-methylpentyl]-methylamino]äthyl] -6-fluor-1,2,3,4-tetrahydro-1-isopropyl -2-naphthylmethoxyacetat-dihydrochlorid hergestellt,

$$[\alpha]_{589}^{20} = +20,0°$$

(c = 0,7%; Methanol).

Die als Ausgangsstoff eingesetzte (S)-6-[1-(Benzyloxy)-N-methylformamido]heptansäure wurde wie folgt hergestellt:

200 g (1,39 Mol) 6-Oxoheptansäure werden in 1,2 l Methylenchlorid gelöst. Bei -20° werden 14 ml konzentrierte Schwefelsäure zugegeben. Dann kondensiert man bei -40° 0,6 l (6,3 Mol) Isobutylen und lässt es darauf in den Reaktionskolben destillieren. Danach lässt man das Reaktionsgemisch 6 Tage bei Raumtemperatur unter Rückfluss des Reagens reagieren. Danach gibt man einen Liter gesättigte wässrige Natriumbicarbonatlösung unter Rühren zu. Die wässrige Phase wird mit Methylenchlorid extrahiert. Die vereinigten organischen Phasen werden über Magnesiumsulfat getrocknet und unter vermindertem Druck eingedampft. Dabei erhält man 268,0 g (1,338 Mol; 96,4%) tert.-Butyl-6-oxoheptanoat, welche zusammen mit 162,1 g (1,338 Mol) (S)-(-)-1-Phenyl-äthylamin und 5,8 g (30,5 mMol) p-Toluolsulfonsäure in 1,9 l Toluol unter gleichzeitiger Wasserabscheidung 12 Stunden zum Rückfluss erhitzt werden. Nach dem Eindampfen des Lösungsmittels erhält man 395,5 g (1,3 Mol; 97,4%) tert.-Butyl-(E/Z)-6-[[(R)-α-methylbenzyl]imino]-heptanoat, welche in 7 l Methanol gelöst werden. Dazu gibt man 43 g Raney-Nickel und hydriert bei 10 bar während 24 Stunden. Danach filtriert man und dampft das Lösungsmittel ein. Die erhaltenen 378,5 g eines Oels werden in 1,1 l Essigester gelöst und bei 0° mit 130 ml 10N äthanolischer Salzsäure versetzt. Nach 1-stündigem Rühren bei 0° werden die gebildeten Kristalle abfiltriert und getrocknet. Durch dreimaliges Umkristallisieren der erhaltenen 282 g Kristalle aus Essigester erhält man 172,7 g (38,9%) tert.-Butyl-(S)-6-[-[(S)-α-methylbenzyl]amino]hexanoat -hydrochlorid, Smp. 154-156°.

160 g (0,648 Mol) des obigen Hydrochlorids werden in 2,4 l Aethanol gelöst und in Gegenwart von 20 g Palladium auf Kohle (5%ig) bei 10 bar hydriert. Nach dem Abfiltrieren des Katalysators wird das Lösungsmittel eingedampft, und der Rückstand aus 560 ml Essigester und 240 ml Hexan kristallisiert. Man erhält dabei 101 g (90,8%) tert.-Butyl(S)-6-aminoheptanoat-hydrochlorid, Smp. 107-109°.

89 g (374 mMol) tert.-Butyl(S)-6-aminoheptanoat-hydrochlorid werden in 1,3 l Methylenchlorid gelöst. Die Lösung wird mit Chlorwasserstoffsäure gesättigt und 4 Stunden zum Rückfluss erhitzt. Nach Abfiltrieren und Trocknen des gebildeten Niederschlags erhält man 60,8 g (89,5%) (S)-6-Aminoheptansäure-hydrochlorid, Smp. 157-160°.

Zu 30 g (166 mMol) (S)-6-Aminoheptansäure-hydrochlorid in 57 ml Wasser gibt man 57 ml 4N wässrige Natronlauge und tropft danach gleichzeitig bei 10° 92 ml 4N wässrige Natronlauge und 42 ml (294 mMol) Chlorameisensäurebenzylester so zu, dass der pH-Wert immer zwischen 10 und 12 liegt. Nach dem Ausfallen des Rohprodukts wird weitere 2 Stunden bei 0° gerührt. Danach werden 300 ml Wasser zugefügt, und das Reaktionsgemisch mit Aether extrahiert. Die wässrige Phase wird dann mit 20 ml konzentrierter Salzsäure angesäuert und mit Methylenchlorid extrahiert. Die Methylenchloridphase wird über Magnesiumsulfat getrocknet und eingedampft. Die erhaltenen Kristalle werden aus Chloroform/Hexan umkristallisiert, wobei man 33,1 g (72%) (S)-6-[1-(Benzyloxy)formamido]heptansäure erhält, Smp. 82-83°.

Zu einer Suspension von 3,05 g 55%igem Natriumhydrid (70 mMol) in 200 ml Dimethylformamid gibt man 6,5 g (23 mMol) (S)-6-[1-(Benzyloxy)formamido]heptansäure zu und lässt 30 Minuten bei 40° reagieren. Danach werden 13 g (90 mMol) Methyljodid zugetropft, und das Reaktionsgemisch 1 Stunde auf 70° erhitzt. Nach dem Eindampfen des Lösungsmittels werden 120 ml 1N wässrige Natronlauge und 120 ml Aethanol zugegeben, und das Reaktionsgemisch 30 Minuten zum Rückfluss erhitzt. Danach wird auf die

Hälfte eingedampft, 100 ml gesättigte wässrige Natriumbicarbonatlösung zugegeben und mit Essigester extrahiert. Die wässrige Phase wird angesäuert und mit Methylenchlorid extrahiert. Die organische Phase wird über Magnesiumsulfat getrocknet und eingedampft. Der Rückstand (5 g) wird an Silicagel mit einem 12:1-Gemisch von Methylenchlorid und Methanol chromatographiert, wobei man 3,5 g (52,2%) (S)-6-[1-(Benzyloxy)-N-methylformamido]heptansäure erhält, MS: $M^+$ 293.

Beispiel 10

Ein Gemisch von 1,4 g (3,33 mMol) 2-[[1S,2S]-6-Fluor-1,2,3,4-tetrahydro-1-isopropyl -2-methoxy-2-naphthyl]äthyl-p-toluolsulfonat und 1,63 g (6,66 mMol) 2-[7-(Methylamino)-heptyl]benzimidazol wird 30 Minuten auf 100° erhitzt. Das Gemisch wird danach auf 100 ml Wasser gegossen und mit 100 ml Essigester extrahiert. Die organische Phase wird über Magnesiumsulfat getrocknet, und unter vermindertem Druck eingedampft, und der Rückstand an Silicagel mit einem 6:1-Gemisch von Methylenchlorid und Methanol chromatographiert. Man erhält dabei ein gelbliches Oel (1 g), welches in 20 ml Aethanol gelöst und mit 2 ml mit Chlorwasserstoffsäure gesättigtem Aether versetzt wird. Dann wird unter vermindertem Druck eingedampft, und der Rückstand aus Essigester/Aethanol/Aether kristallisiert und getrocknet, wobei man 0,7 g (37,2%) [1S,2S]-2-[2-[[7-(2-Benzimidazolyl)heptyl]methylamino]äthyl] -6-fluor-1,2,3,4-tetrahydro-1-isopropyl-2-methoxynaphthalinol -dihydrochlorid erhält, Smp. 179-181°.

Das als Ausgangsmaterial eingesetzte 2-[[1S,2S]-6-Fluor-1,2,3,4-tetrahydro-1-isopropyl-2-methoxy -2-naphthyl]äthyl-p-toluolsulfonat wurde wie folgt hergestellt:

Ein Gemisch von 5,04 g (20 mMol) 6-Fluor-1,2,3,4-tetrahydro-2-hydroxy-1α-isopropyl-2β -naphthyläthanol, 6,13 g (22 mMol) Triphenylchlormethan und 50 ml Pyridin wird 20 Stunden bei Raumtemperatur gerührt. Das Reaktionsgemisch wird danach auf 500 ml Eiswasser gegossen und mit 400 ml Aether extrahiert. Die Aetherextrakte werden mit 400 ml 1N wässriger Salzsäure, 400 ml gesättigter wässriger Natriumbicarbonatlösung und 400 ml Wasser gewaschen. Nach dem Trocknen über Magnesiumsulfat wird der Aether unter vermindertem Druck abgedampft. Man erhält 8,25 g (83%) [1S,2S]-6-Fluor-1,2,3,4-tetrahydro-1-isopropyl -2-[2-(trityloxy)äthyll]-2-naphthalinol. 8 g (16,2 mMol) dieser Verbindung werden in 300 ml Tetrahydrofuran gelöst und bei -20° mit frisch zubereiteten 35,6 mMol Lithiumdiisopropylamid versetzt. Danach werden 9,2 g (64,8 mMol) Methyljodid zugegeben, und das Reaktionsgemisch 20 Stunden bei Raumtemperatur gerührt. Das Reaktionsgemisch wird auf Eiswasser gegossen und mit Methylenchlorid extrahiert. Die organische Phase wird über Magnesiumsulfat getrocknet, und das Lösungsmittel unter vermindertem Druck abgedampft. Nach dem Chromatographieren an Silicagel unter Verwendung eines 1:2-Gemisches von Methylenchlorid und Hexan und Umkristallisation aus Hexan erhält man 4,15 g (54,7%) [1S,2S]-6-Fluor-1,2,3,4-tetrahydro-1-isopropyl -2-methoxy-2-[2-(trityloxy)äthyl]naphthalinol, Smp. 132-134°.

4,15 g (8,16 mMol) der obigen Verbindung werden mit 15 ml mit Chlorwasserstoffsäure gesättigtem Aether bei 0° 2 Stunden stehengelassen. Nach dem Eindampfen des Lösungsmittels und Chromatographieren des Rückstandes an Silicagel unter Verwendung eines 1:2-Gemisches von Essigester und Hexan erhält man 1,0 g (47%) [1S,2S]-6-Fluor-1,2,3,4-tetrahydro-1-isopropyl -2-methoxy-2-naphthyläthanol,

$$[\alpha]_{589}^{20} = +65,6°$$

(c= 0,25%; Methanol).

0,98 g (3,7 mMol) [1S,2S]-6-Fluor-1,2,3,4-tetrahydro-1-isopropyl -2-methoxy-2-naphthyläthanol werden in 6 ml Pyridin gelöst und bei 0° 1 Stunde mit 1,06 g (5,6 mMol) Toluol-4-sulfochlorid reagieren gelassen. Das Reaktionsgemisch wird danach auf 100 ml Wasser gegossen und mit 200 ml Aether extrahiert. Der Aetherextrakt wird mit 100 ml 1N wässriger Salzsäure, 100 ml gesättigter wässriger Natriumbicarbonatlösung und 100 ml Wasser gewaschen. Nach dem Trocknen der ätherischen Lösung über Magnesiumsulfat und Eindampfen des Lösungsmittels erhält man 1,50 g (97,4%) 2-[[1S,2S]-6-Fluor-1,2,3,4-tetrahydro-1-isopropyl -2-methoxy-2-naphthyl]äthyl-p-toluolsulfonat,

$$[\alpha]_{589}^{20} = +42,6°$$

(c= 0,5%; Methanol).

Beispiel 11

Eine Suspension von 436 mg (10 mM ol) 55%igem Natriumhydrid in 20 ml Tetrahydrofuran werden 2,0 g (4,43 mMol) [1S,2S]-2-[2-[[5-(2-Benzimidazolyl)pentyl]methylamino]äthyl] -6-fluor-1,2,3,4-tetrahydro-1-isopropyl-2-naphthalinol gelöst in 30 ml Tetrahydrofuran gegeben. Nach 45-minütigem Rühren bei Raumtemperatur werden 1,42 g (10 mMol) Methyljodid zugegeben. Nach einer weiteren Stunde werden Wasser und Methylenchlorid zugegeben, und das Reaktionsgemisch kräftig geschüttelt. Die organische Phase wird über Magnesiumsulfat getrocknet und eingedampft. Der Rückstand wird an Silicagel mit einem 6:1-Gemisch von Methylenchlorid und Methanol chromatographiert, wobei man 1,2 g (60%) [1S,2S]-6-Fluor-1,2,3,4-tetrahydro-1-isopropyl-2 -[2-[methyl-[5-(1-methyl-2 -benzimidazolyl)pentyl]amino]äthyl]-2-naphthanlinol erhält, MS: $M^+$ 465.

In analoger Weise wie oben beschrieben wurde durch Umsetzen mit Dodecyljodid das [1S,2S]-2-[2-[[7-(1-Dodecyl-2-benzimidazolyl)heptyl]methylamino]äthyl]--fluor -1,2,3,4-tetrahydro-1-isopropyl-2-naphthalinol hergestellt.

Beispiel 12

Ein Gemisch von 1,2 g (2.58 mMol) [1S,2S]-6-Fluor-1,2,3,4-tetrahydro-1-isopropyl-2 -[2-[methyl-[5-(1-methyl-2-benzimidazolyl)pentyl]amino]äthyl]-2-naphthalinol, 206 mg (2.6 mMol) Pyridin und 4 ml Methoxyessigsäureanhydrid wird 2 Stunden auf 70° erhitzt. Danach gibt man 100 ml 3N wässrige Natronlauge zu und extrahiert mit 100 ml Methylenchlorid. Die organische Phase wird über Magnesiumsulfat getrocknet und eingedampft. Das Rohprodukt wird an Silicagel unter Verwendung eines 15:1-Gemisches von Methylenchlorid und Methanol chromatographiert. Die erhaltenen 550 mg eines Oels werden in 50 ml Essigester gelöst und mit 1 ml mit Chlorwasserstoffsäure gesättigtem Aether versetzt. Nach dem Eindampfen des Lösungsmittels kristallisiert man aus Essigester/Aether. wobei man 600 mg (41%) [1S,2S]-6-Fluor-1,2,3,4-tetrahydro-1-isopropyl-2-[2-[methyl-[5-(1-methyl-2-benzimidazolyl)pentyl]amino]äthyl]-2-naphthylmethoxyacetatdihydrochlorid erhält, Smp. 203-205°.

In analoger Weise wie oben beschrieben wurde das [1S,2S]-2-[2-[[7-(1-Dodecyl-2 -benzimidazolyl)-heptyl)methylamino]äthyl]-6-fluor -1,2,3,4-tetrahydro-1-isopropyl-2-naphthylmethoxyacetat -dihydrochlorid hergestellt,

$$[\alpha]^{20}_{589} = +20,4°$$

(c= 0,9%; Methanol).

Beispiel 13

0,425 g (1 mMol) [1S,2S]-2-[2-[[3-(-2 -Benzimidazolyl)propyl]methylamino]äthyl]-6-fluor -1,2,3,4-tetrahydro-1-isopropyl-2-naphthylmethoxyacetat werden in 60 ml Methanol gelöst und anschliessend mit 10 ml 6%igem Wasserstoffperoxyd und 50 mg (0.15 mMol) Natriumwolframat versetzt. Nach 20-stündigem Rühren bei Raumtemperatur gibt man 100 mg Platin auf Kohle (5%ig) in 2 ml Wasser zu und rührt eine weitere Stunde. Darauf filtriert man, konzentriert das Filtrat, verdünnt mit wenig Methylenchlorid, und chromatographiert das Gemisch an Silicagel mit einem 15:1-Gemisch von Methylenchlorid und Methanol als Eluierungsmittel. Man erhält dabei 0,18 g (35,2%) eines ersten Diastereomeren von [1S,2S]-2-[2-[[3-(2 -Benzimidazolyl)propyl]methyl-N-oxidoamino]äthyl]-6-fluor -1,2,3,4-tetrahydro-1-isopropyl-2-naphthylmethoxyacetat mit einem $R_f$-Wert von 0,33,

$$[\alpha]^{20}_{589} = +39,4°$$

(c= 0,5%; Methanol), und 0,276 g (54%) eines zweiten Diastereomeren der genannten Verbindung mit einem $R_f$-Wert von 0,26 (Methylenchlorid/Methanol 6:1),

$$[\alpha]^{20}_{589} = +34,8°$$

(c= 0,5%; Methanol).

Beispiel 14

5,0 g (12,3 mMol) 2-(6-Fluor-1,2,3,4-tetrahydro-2-hydroxy-1α-isopropyl -2β-naphthyl)äthyl-p-toluolsulfonat und 4,0 g (24,6 mMol) 2-Methylaminobenzthiazol werden 30 Minuten auf 120° erhitzt. Danach werden 50 ml eines 12:1-Gemisches von Methylenchlorid und Methanol zugegeben, und das Reaktionsgemisch mittels Säulenchromatographie an Silicagel unter Verwendung eines 1:1-Gemisches von Hexan und Essigester als Eluierungsmittel gereinigt und isoliert. Man erhält auf diese Weise 3,22 g (65,7%) [1S,2S]-2-[2-[(2-Benzthiazolyl)methylamino]äthyl]-6-fl uor -1,2,3,4-tetrahydro-1-isopropyl-2-naphthalinol, Smp. 102-103°.

Beispiel 15

6,1 g (15 mMol) [1S,2S]-2-(6-Fluor-1,2,3,4-tetrahydro-2-hydroxy-1-isopropyl -2-naphthyl)äthyl-p-toluolsulfonat und 6,8 g (30 mMol) 1-[2-(Methylamino)äthyl]-2-benzimidazolinon -hydrochlorid werden in einem Gemisch von 30 ml Dimethylformamid und 30 ml N-Aethyldiisopropylamin 4,5 Stunden bei 130° gerührt. Das Reaktionsgemisch wird auf 600 ml Eiswasser gegossen unt mit 700 ml Methylenchlorid extrahiert. Der Extrakt wird mit Wasser gewaschen, über Kaliumcarbonat getrocknet und eingedampft. Das so erhaltene Rohprodukt wird an 150 g Silicagel mit Methylenchlorid und 0-10% Isopropanol als Eluierungsmittel chromatographiert, wobei man 5,2 g (72%) 1-[2-[[2-[[1S,2S]-6-Fluor-1,2,3,4-tetrahydro-2-hydroxy -1-isopropyl-2-naphthyl]äthyl]methylamino]äthyl] -2-benzimidazolinon als Oel erhält.

Das als Ausgangsmaterial eingesetzte 1-[2-(Methylamino)äthyl]-2-benzimidazolinon-hydrochlorid wurde wie folgt hergestellt:

Zu 24,8 g (150 mMol) 2-(N-Benzyl-N-methylamino)äthanol, gelöst in 250 ml abs. Tetrahydrofuran werden bei 0-5° 93,8 ml (150 mMol) einer n-Butyllithium-Lösung (ca. 1,6M in Hexan) getropft. Nach 15-minütigem Rühren bei 0° werden 11,7 ml (150 mMol) Methansulfochlorid in 50 ml Tetrahydrofuran bei einer Temperatur zwischen 0 und 5° zugetropft, und das Reaktionsgemisch 30 Minuten bei 0° gerührt.

5,8 g (133 mMol) einer 55%igen Natriumhydrid-Dispersion in Mineralöl werden mit Hexan ölfrei gewaschen und in 40 ml Dimethylformamid suspendiert. Anschliessend werden 23,1 g (132,5 mMol) 1-(1-Methylvinyl)benzimidazolin-2-on in 90 ml Dimethylformamid bei Raumtemperatur zugetropft, und das Reaktionsgemisch 15 Minuten weiter gerührt.

Dieses Reaktionsgemisch wird bei 0° zu der weiter oben beschriebenen Reaktionslösung getropft. Danach wird auf 70° erwärmt und 3 Stunden gerührt. Anschliessend wird das Reaktionsgemisch auf 1 l Eiswasser gegossen und mit 600 ml Methylenchlorid extrahiert. Der Extrakt wird mit Wasser gewaschen, über Kaliumcarbonat getrocknet und eingedampft. Das so erhaltene Rohprodukt wird an 500 g Silicagel mit Methylenchlorid und 0-5% Isopropanol als Eluierungsmittel chromatographiert, wobei man 26,8 g (63%) 1-(1-Methylvinyl)-3-[2-(N-benzyl-N-methylamino)äthyl] -2-benzimidazolinon als Oel erhält.

26,5 g (82,5 mMol) der oben genannten Verbindung werden in 265 ml Aethanol gelöst, unter Rühren mit 26,5 ml konzentrierter wässriger Salzsäure versetzt und 1 Stunde zum Rückfluss erhitzt. Nach dem Abkühlen des Reaktionsgemisches auf 5° kristallisiert 1-[2-(N-Benzyl-N-methylamino)äthyl]-2-benzimidazolinon in Form des Hydrochlorids aus, Smp. 107-109°; Ausbeute 24,2 g (92%).

22,9 g (72 mMol) 1-[2-(N-Benzyl-N-methylamino)äthyl] -2-benzimidazolinon-hydrochlorid werden in 250 ml Methanol gelöst, mit 2,5 g Palladium auf Kohle (10%ig) versetzt und 90 Minuten bei Raumtemperatur hydriert. Der nach dem Filtrieren und Einengen erhaltene Rückstand wird aus Methanol/Aether umkristallisiert, wobei man 15,5 g (94%) 1-[2-(Methylamino)äthyl]-2-benzimidazolinon-hydrochlorid erhält, Smp. 177-180°.

Beispiel 16

4,57 g (10,7 mMol) 1-[2-[[2-[[1S,2S]-6-Fluor-1,2,3,4-tetrahydro-2-hydroxy -1-isopropyl-2-naphthyl]äthyl]-methylamino]äthyl] -2-benzimidazolinon werden in 15 ml Methylenchlorid gelöst, mit 2,2 ml Pyridin und 7,0 g (43 mMol) Methoxyessigsäureanhydrid versetzt und 20 Stunden bei Raumtemperatur gerührt. Danach wird unter Eiskühlung mit 30 ml 3N Natronlauge versetzt und 15 Minuten bei Raumtemperatur gerührt. Anschliessend wird das Reaktionsgemisch auf 400 ml Eiswasser gegossen und mit 600 ml Methylenchlorid extrahiert. Der Extrakt wird mit Wasser gewaschen, über Kaliumcarbonat getrocknet und eingeengt. Dabei erhält man 6,9 g eines Oels (N,O-diacyliertes Produkt), welche in 30 ml Methanol gelöst und bei Raumtemperatur mit 11,5 ml 1N wässriger Natronlauge versetzt werden. Nach 30-minütigem Rühren wir d das Gemisch auf 400 ml Eiswasser gegossen und mit 600 ml Methylenchlorid extrahiert. Der Extrakt wird mit Wasser gewaschen, über Kaliumcarbonat getrocknet, eingedampft, mit einem Aequivalent Chlorwasserstoffsäure in Methanol versetzt, erneut eingedampft und schliesslich aus Methanol/Aether umkristallisiert.

Dabei erhält man 3,9 g (72%) [1S,2S]-6-Fluor-1,2,3,4-tetrahydro-1-isopropyl-2-[2-[methyl[ -2-(2-oxo-1-benzimidazolinyl)äthyl]amino]äthyl]-2-naphthylmethoxyacetat-hydrochlorid, Smp. 130-133° (Zers.); $[\alpha]_D^{20}$ = +26,0° (c= 1%; Methanol).

Beispiel 17

In Analogie zu Beispiel 15 wurde durch Umsetzen von [1S,2S]-2-(6-Fluor-1,2,3,4-tetrahydro-2-hydroxy -1-iso-propyl-2-naphthyl)äthyl-p-toluolsulfonat mit 1-[6-(Methylamino)hexyl]-2-benzimidazolinon das 1-[6-[[2-[[1S,2S]-6-Fluor-1,2,3,4-tetrahydro-2-hydroxy -1-isopropyl-2-naphthyl]äthyl]methylamino]hexyl] -2-benzimidazolinon als Oel erhalten.

Das als Ausgangsmaterial eingesetzte 1-[6-(Methylamino)hexyl]-2-benzimidazolinon wurde wie folgt hergestellt:

Zu 32.7 g (150 mMol) Di-tert.-Butyldicarbonat in 100 ml Methanol werden 17,6 g (150 mMol) 6-Amino-1-hexanol gelöst in 50 ml Methanol bei Raumtemperatur getropft. Nach 4-stündigem Rühren bei Raumtemperatur wird das Reaktionsgemisch eingedampft, wobei 36,6 g tert.-Butyl (6-hydroxyhexyl)carbamat als Oel erhalten wird, das direkt in die nächste Stufe eingesetzt wird.

34,8 g tert.-Butyl (6-hydroxyhexyl)carbamat werden in 250 ml Methylenchlorid gelöst und bei 0° mit 24,0 ml (174 mMol) Triäthylamin versetzt. Anschliessend werden bei -60° innerhalb von 15 Minuten 12,9 ml (166 mMol) Methansulfochlorid in 50 ml Methylenchlorid zugetropft. und das Reaktionsgemisch anschliessend bei -60° 90 Minuten gerührt. Danach wird die Reaktionslösung auf 600 ml Eiswasser gegossen und mit 800 ml Methylenchlorid extrahiert. Der organische Extrakt wird mit Wasser gewaschen, über Magnesiumsulfat getrocknet und eingedampft. Dabei erhält man 58,6 g tert.-Butyl [6- [(methylsulfonyl)oxy]hexyl]-carbamat als Oel, das ohne Reinigung weiterverarbeitet wird.

5,9 g (135 mMol) einer 55%igen Natriumhydrid-Dispersion in Mineralöl werden mit Hexan ölfrei gewaschen und anschliessend mit 100 ml Dimethylformamid überschichtet. Zu dieser Suspension werden 22,3 g (128 mMol) 1-(1-Methylvinyl)benzimidazolin-2-on in 100 ml Dimethylformamid bei Raumtemperatur getropft. Nach 2-stündigem Rühren bei Raumtemperatur werden 55,0 g tert.-Butyl [6-[(methylsulfonyl)oxy]-hexyl]carbamat in 100 ml Dimethylformamid zugetropft, und das Reaktionsgemisch 18 Stunden bei Raumtemperatur gerührt. Danach wird das Reaktionsgemisch auf 1 l Wasser gegossen und mit 750 ml Methylenchlorid extrahiert. Der organische Extrakt wird mit Wasser gewaschen, über Kaliumcarbonat getrocknet und eingedampft. Der so erhaltene Rückstand wird an 950 g Silicagel mit Methylenchlorid/Hexan, Methylenchlorid und einem 95:5-Gemisch von Methylenchlorid und Isopropanol als Eluierungsmittel chromatographiert, wobei man 45,3 g tert.-Butyl [6-[3-(1-methylvinyl)-2-oxo -1-benzimidazolinyl]hexyl]carbamat als Oel erhält.

5,3 g (121 mMol) einer 55%igen Natriumhydrid-Dispersion in Mineralöl werden mit Hexan ölfrei gewaschen und anschliessend mit 100 ml Dimethylformamid überschichtet. Zu dieser Suspension werden 45,0 g (121 mMol) tert.-Butyl [6-[3-(1-methylvinyl)-2-oxo -1-benzimidazolinyl]hexyl]carbamat in 100 ml Dimethylformamid bei Raumtemperatur getropft, und das Reaktionsgemisch 90 Minuten bei dieser Temperatur gerührt. Anschliessend werden 9,0 ml (155 mMol) Methyljodid in 50 ml Dimethylformamid bei 10° zugetropft, und das Reaktionsgemisch 1 Stunde bei 10° und 16 Stunden bei Raumtemperatur gerührt. Danach wird die Reaktionslösung auf 800 ml Eiswasser gegossen und mit 600 ml Methylenchlorid extrahiert. Der Extrakt wird mit Wasser gewaschen, über Kaliumcarbonat getrocknet und eingedampft. Der so erhaltene Rückstand wird an 500 g Silicagel mit Hexan/Essigester (4:1 und 1:1) chromatographiert, wobei man 39,1 g tert.-Butyl methyl-[6-[3-(1-methylvinyl)-2-oxo -1-benzimidazolinyl]hexyl]carbamat als Oel erhalten werden.

38,8 g (100 mMol) der zuletzt genannten Verbindung werden in 300 ml abs. Aethanol gelöst, unter Rühren mit 40 ml konz. wässriger Salzsäure versetzt und 75 Minuten zum Rückfluss erwärmt. Nach dem Abkühlen auf 40° wird das Reaktionsgemisch unter vermindertem Druck eingeengt und auf 500 ml Eiswasser gegossen. Die wässrige Phase wird unter Zugabe von konzentrierter wässriger Ammoniaklösung auf pH 8-9 eingestellt und mit 600 ml Methylenchlorid extrahiert. Der Extrakt wird mit Wasser gewaschen und anschliessend verworfen. Die vereinigten wässrigen Phasen werden mit 3N wässriger Natronlauge auf pH 10-11 eingestellt und sechsmal mit je 150 ml Methylenchlorid/Isopropanol (4:1) extrahiert. Die vereinigten Extrakte werden über Kaliumcarbonat getrocknet und eingedampft, wobei man 21,6 g 1-[6-(Methylamino)hexyl]-2-benzimidazolinon als Oel erhält.

Beispiel 18

In Analogie zu Beispiel 17 wurde durch Umsetzen von [1S,2S]-2-(6-Fluor-1,2,3,4-tetrahydro-2-hydroxy-

1-isopropyl -2-naphthyl)äthyl-p-toluolsulfonat und 1-Methyl-3-[6-(methylamino)hexyl]-2-benzimidazolinon das 1-[6-[[2-[[1S,2S]-6-Fluor-1,2,3,4-tetrahydro-2-hydroxy -1-isopropyl-2-naphthyl]äthyl]methylamino]hexyl] -3-methyl-2-benzimidazolinon als Oel erhalten.

Das als Ausgangsmaterial eingesetzte 1-Methyl-3-[6-(methylamino)hexyl]-2-benzimidazolinon wurde wie folgt hergestellt:

Zu 10,0 g (40,4 mMol) 1-[6-(Methylamino)hexyl]-2-benzimidazolinon in 150 ml Methanol wird bei Raumtemperatur eine Lösung von 9,7 g (44,5 mMol) Di-tert.-butyldicarbonat in 50 ml Methanol getropft, und das Reaktionsgemisch 16 Stunden bei Raumtemperatur gerührt. Danach werden 6,9 ml (49,5 mMol) Triäthylamin und weitere 9,7 g Di-tert.-butyldicarbonat in 50 ml Methanol zugegeben und weitere 16 Stunden bei Raumtemperatur gerührt. Danach wird das Reaktionsgemisch auf 200 ml Wasser gegossen und mit 400 ml Methylenchlorid extrahiert. Die Extrakte werden mit Wasser gewaschen, über Kaliumcarbonat getrocknet und eingedampft, wobei man 14,1 g tert.-Butyl methyl-[6-(2-oxo-1-benzimidazolinyl)hexyl]-carbamat als Oel erhält.

2,6 g (59,6 mMol) einer 55%igen Natriumhydrid-Dispersion in Mineralöl werden mit Hexan ölfrei gewaschen und anschliessend mit 30 ml Dimethylformamid überschichtet. Zu dieser Suspension werden 13,8 g (39,7 mMol) tert.-Butyl methyl-[6-(2-oxo-1-benzimidazolinyl)hexyl]carbamat in 90 ml Dimethylformamid innerhalb von 20 Minuten bei Raumtemperatur getropft. Nach 90-minütigem Rühren bei Raumtemperatur werden 6,2 ml (99,3 mMol) Methyljodid in 30 ml Dimethylformamid bei Raumtemperatur zugetropft, und das Reaktionsgemisch weitere 16 Stunden bei dieser Temperatur gerührt. Zur Aufarbeitung wird auf 200 ml Wasser gegossen und mit 300 ml Methylenchlorid extrahiert. Der Methylenchloridextrakt wird mit Wasser gewaschen, über Kaliumcarbonat getrocknet und eingeengt. Der Rückstand wird mit Methylenchlorid und Methylenchlorid/Isopropanol (99:1 und 98:2) an 110 g Silicagel chromatographiert, wobei man 9,0 g tert.-Butyl methyl-[6-(3-methyl-2-oxo -1-benzimidazolinyl)hexyl]carbamat als Oel erhält.

In Analogie zu Beispiel 17, letzter Absatz, wurde aus der oben erhaltenen Verbindung das 1-Methyl-3-[6-(methylamino)hexyl]-2-benzimidazolinon als Oel erhalten.

Beispiel 19

In analoger Weise wie in Beispiel 17 beschrieben wurde durch Umsetzen von [1S,2S]-2-(6-Fluor-1,2,3,4-tetrahydro-2-hydroxy-1-isopropyl -2-naphthyl)äthyl-p-toluolsulfonat und 1-[p-[4-(Methylamino)butyl]phenyl]-imidazol das [1S,2S]-6-Fluor-1,2,3,4-tetrahydro-1-isopropyl-2-[2-[[4-[p -(imidazol-1-yl)phenyl]butyl]-methylamino]äthyl] -2-naphthalinol als Oel erhalten.

Das als Ausgangsmaterial eingesetzte 1-[p-[4-(Methylamino)butyl]phenyl]imidazol wurde wie folgt hergestellt:

A) 53,1 g (116 mMol) [2-(m-Dioxan-2-yl)äthyl]triphenylphosphoniumbromid werden in 160 ml Tetrahydrofuran suspendiert und bei -25° innerhalb von 15 Minuten mit 77,3 ml (116 mMol) n-Butyllithium-Lösung (ca. 1,5M in Hexan) versetzt. Danach wird 15 Minuten bei -25° gerührt. Anschliessend werden 10 ml eines Gemisches von Tetrahydrofuran und 1,3-Dimethyl-3,4,5,6-tetrahydro-2-(1H)-pyrimidinon (1:1) zugegeben, weitere 5 Minuten bei -25° gerührt und dann innerhalb von 30 Minuten bei -25° mit 20 g (116 mMol) p-Imidazol-1-yl-benzaldehyd in 180 ml Tetrahydrofuran/1,3-Dimethyl-3,4,5,6-tetrahydro -2-(1H)-pyrimidinon (1:1) versetzt. Nach beendeter Zugabe wird das Reaktionsgemisch auf Raumtemperatur erwärmt und 15 Minuten bei dieser Temperatur gerührt. Danach wird das Reaktionsgemisch auf 1 l Eiswasser gegossen und mit 600 ml Methylenchlorid extrahiert. Der Methylenchloridextrakt wird mit Wasser gewaschen, über Magnesiumsulfat getrocknet und eingedampft. Dabei werden 42,2 g eines halbkristallinen Produkts erhalten, das in 600 ml Methanol gelöst und in Gegenwart von 18 g Palladium auf Kohle (5%ig) erschöpfend hydriert wird. Nach dem Abfiltrieren des Katalysators und Eindampfen des Filtrates erhält man 36,6 g eines halbkristallinen Rückstandes, der seinerseits in 700 ml Methanol gelöst, mit 22,4 g p-Toluolsulfonsäuremonohydrat versetzt und 2,5 Stunden zum Rückfluss erhitzt wird. Nach dem Abkühlen auf Raumtemperatur wird der pH-Wert mit 36 g Natriumcarbonat auf 7 eingestellt, das Reaktionsgemisch eingedampft, der Rückstand auf 500 ml Wasser gegossen und mit 600 ml Methylenchlorid extrahiert. Der Methylenchloridextrakt wird mit Wasser und gesättigter Kochsalzlösung gewaschen, über Magnesiumsulfat getrocknet und eingedampft, wobei man 36,1 g eines halbkristallinen Rückstandes erhält. Dieser wird in 400 ml Tetrahydrofuran gelöst, mit 110 ml 3N wässriger Salzsäure versetzt, 3 Stunden bei Raumtemperatur gerührt und anschliessend unter vermindertem Druck eingeengt. Danach wird das Reaktionsgemisch auf 500 ml Eiswasser gegossen und dreimal mit je 200 ml Aether extrahiert. Die wässrige Phase wird anschliessend mit Kaliumcarbonat auf pH 9 eingestellt und mit 600 ml Methylenchlorid extrahiert. Der Methylenchloridextrakt wird mit Wasser gewaschen, über Kaliumcarbonat getrocknet und eingedampft. Der Rückstand wird an 240 g Silicagel mit Methylenchlorid

und 0-5% Isopropanol als Eluierungsmittel chromatographiert. Dabei erhält man 13,2 g (53%) 4-[p-(Imidazol-1-yl)phenyl]butanal als Oel.

B) 37,8 g (558 mMol) Methylamin-hydrochlorid werden in 200 ml Methanol gelöst und hierauf mit 45,8 g (558 mMol) Natriumacetat und 3,9 g (62,1 mMol) Natriumcyanoborhydrid versetzt. Das Reaktionsgemisch wird während 15 Minuten bei Raumtemperatur gerührt, und danach werden 12,05 g (56,24 mMol) 4-[p-(Imidazol-1-yl)phenyl]butanal in 40 ml Methanol innerhalb von 15 Minuten bei Raumtemperatur zugetropft, und das Reaktionsgemisch 3 Stunden bei Raumtemperatur gerührt. Danach wird das Reaktionsgemisch unter vermindertem Druck eingeengt, der Rückstand auf 1 l Eiswasser gegossen und mit 800 ml Methylenchlorid extrahiert. Der organische Extrakt wird mit Wasser gewaschen, über Magnesiumsulfat getrocknet und eingedampft. Der erhaltene Rückstand wird an 100 g Silicagel mit Methylenchlorid/Isopropanol/wässrigem, 25%igem Ammoniak (160:40:1 bzw. 7:3:0,3) chromatographiert, wobei man 3,8 g (29%) 1-[p-[4-(Methylamino)butyl]phenyl]imidazol als Oel erhalt.

Beispiel 20

In analoger Weise wie in Beispiel 15 beschrieben wurde durch Umsetzen von [1S,2S]-2-(6-Fluor-1,2,3,4-tetrahydro-2-hydroxy-1-isopropyl -2-naphthyl)äthyl-p-toluolsulfonat und 1-[4-(Methylamino)butyl]-2-benzimidazolinon das 1-[4-[[2-[[1S,2S]-6-Fluor-1,2,3,4-tetrahydro-2-hydroxy -1-isopropyl-2-naphthyl]äthyl]-methylamino]butyl] -2-benzimidazolinon als Oel erhalten.

Das als Ausgangsstoff eingesetzte 1-[4-(Methylamino)butyl]-2-benzimidazolinon wurde wie folgt hergestellt:

In Analogie zu Beispiel 17 wurde aus 4-(Methylamino)-1-butanol das tert.-Butyl methyl-[4-[-(methylsulfonyl)oxy]butyl]carbamat als Oel erhalten, welches dann in das tert.-Butyl methyl-[4-[3-(1-methylvinyl)-2-oxo -1-benzimidazolinyl]butyl]carbamat übergeführt wurde. Diese ebenfalls als Oel erhaltene Verbindung wurde dann wiederum in Analogie zu Beispiel 17 in das wiederum als Oel anfallende 1-[4-(Methylamino)butyl]-2-benzimidazolinon übergeführt.

Beispiel 21

In analoger Weise wie in Beispiel 17 beschrieben wurde durch Umsetzen von [1S,2S]-2-(6-Fluor-1,2,3,4-tetrahydro-2-hydroxy -1-isopropyl-2-naphthyl)äthyl-p-toluolsulfonat und 1-Isopropyl-3-[4-(methylamino)butyl]-2-benzimidazolinon das 1-[4-[[2-[[1S,2S]-6-Fluor-1,2,3,4-tetrahydro-2-hydroxy-1-isopropyl-2-na hthyl]äthyl]-methylamino]butyl] -3-isopropyl-2-benzimidazolinon als Oel erhalten.

Das als Ausgangsstoff eingesetzte 1-Isopropyl-3-[4-(methylamino)butyl]-2-benzimidazolinon wurde wie folgt hergestellt:

8,14 g (22,6 mMol) tert.-Butyl methyl-[4-[3-(1-methylvinyl)-2-oxo -1-benzimidazolinyl]butyl]carbamat werden in 80 ml Methanol gelöst und nach Zusatz von 1,6 g Palladium auf Kohle (5%ig) während 4 Stunden hydriert. Hierauf wird das Reaktionsgemisch filtriert und eingedampft, wobei man 8,5 g tert.-Butyl methyl-[4-(3-isopropyl-2-oxo -1-benzimidazolinyl)butyl]carbamat als Oel erhält. Dieses wurde in Analogie zum letzten Absatz von Beispiel 17 in das ebenfalls als Oel anfallende 1-Isopropyl-3-[4-(methylamino)butyl]-2-benzimidazolinon übergeführt.

Beispiel 22

In analoger Weise wie in Beispiel 17 beschrieben wurde durch Umsetzen von [1S,2S]-2-(6-Fluor-1,2,3,4-tetrahydro-2-hydroxy -1-isopropyl-2-naphthyl)äthyl-p-toluolsulfonat und 1-Butyl-3-[6-(methylamino)hexyl]-2-benzimidazolinon das 1-[6-[[2-[[1S,2S]-6-Fluor-1,2,3,4-tetrahydro-2-hydroxy-1-isopropyl-2-naphthyl]äthyl]-methylamino]hexyl] -3-butyl-2-benzimidazolinon als Oel erhalten.

Das als Ausgangsstoff eingesetzte 1-Butyl-3-[6-(methylamino)hexyl]-2-benzimidazolinon wurde wie folgt hergestellt:

In Analogie zu Beispiel 18 wurde aus tert.-Butyl methyl-[6-(2-oxo-1-benzimidazolinyl)hexyl]carbamat und Butyljodid das tert.-Butyl methyl-[6-(3-butyl-2-oxo-1-benzimidazolinyl)hexyl]carbamat als Oel erhalten. Diese Verbindung wurde in Analogie zu Beispiel 17 in das 1-Butyl-3-[6-(methylamino)hexyl]-2-benzimidazolinon übergeführt, welches ebenfalls als Oel erhalten wurde.

Beispiel 23

In analoger Weise wie in Beispiel 17 beschrieben wurde durch Umsetzen von [1S,2S]-2-(6-Fluor-1,2,3,4-tetrahydro-2-hydroxy -1-isopropyl-2-naphthyl)äthyl-p-toluolsulfonat und 1-(2-Morpholinoäthyl)-3-[6-(methylamino)hexyl] -2-benzimidazolinon das 1-[6-[[2-[[1S,2S]-6-Fluor-1,2,3,4-tetrahydro-2-hydroxy -1-isopropyl-2-naphthyl]äthyl]methylamino]hexyl]-3-(2-morpholinoäthyl)-2-benzimidazolinon als Oel erhalten.

Das als Ausgangsstoff eingesetzte 1-(2-Morpholinoäthyl)-3-[6-(methylamino)hexyl] -2-benzimidazolinon wurde wie folgt hergestellt:

9,0 g (25,9 mMol) tert.-Butyl methyl-[6-(2-oxo-1-benzimidazolinyl)hexyl]carbamat werden in 250 ml Methanol gelöst, mit 35 g (259 mMol) Kaliumcarbamat, 0,5 g Kaliumjodid und portionenweise mit 16,9 g (90,6 mMol) Chloräthylmorpholin-hydrochlorid versetzt. Danach wird das Reaktionsgemisch 16 Stunden zum Rückfluss erhitzt. Nach dem Abkühlen wird das Reaktionsgemisch auf 1 l Eiswasser gegossen und mit 800 ml Methylenchlorid extrahiert. Der Extrakt wird mit Wasser gewaschen, getrocknet und eingeengt. Der Rückstand wird in 50 ml Aether gelöst und je einmal mit 15 ml und 5 ml 3N Methansulfonsäure in Wasser und einmal mit 5 ml Wasser extrahiert. Die vereinigten wässrigen Phasen werden mit Ammoniak auf pH 8-9 eingestellt und dreimal mit je 100 ml Methylenchlorid extrahiert. Die vereinigten Extrakte werden mit Wasser gewaschen, über Kaliumcarbonat getrocknet und eingedampft, wobei man 7,8 g (65,4%) tert.-Butyl methyl-[6-[3-(2-morpholinoäthyl)-2-oxo -1-benzimidazolinyl]hexyl]carbamat als Oel erhält.

Dieses wurde dann ebenfalls in Analogie zu Beispiel 17 in das 1-(2-Morpholinoäthyl)-3-[6-(methylamino)hexyl]-2-benzimidazolinon-dihydrochlorid übergeführt, Smp. 229-232°.

Beispiel 24

In analoger Weise wie in Beispiel 17 beschrieben wurde durch Umsetzen von [1S,2S]-2-(6-Fluor-1,2,3,4-tetrahydro-2-hydroxy -1-isopropyl-2-naphthyl)äthyl-p-toluolsulfonat und 1-Benzyl-3-[4-(methylamino)butyl]-2-benzimidazolinon das 1-Benzyl-3-[4-[[2-[[1S,2S]-6-fluor-1,2,3,4-tetrahydro-2-hydroxy-1-isopropyl-2-naphthyl]-äthyl]methylamino]butyl]-2-benzimidazolinon als Oel erhalten.

Das als Ausgangsstoff eingesetzte 1-Benzyl-3-[4-(methylamino)butyl]-2-benzimidazolinon wurde in Analogie zu Beispiel 18 aus 1-[4-(Methylamino)butyl]-2-benzimidazolinon via tert.-Butyl methyl-[4-(2-oxo-1-benzimidazolinyl)butyl]carbamat hergestellt.

Beispiel 25

In analoger Weise wie in Beispiel 17 beschrieben wurde durch Umsetzung von [1S,2S]-2-(6-Fluor-1,2,3,4-tetrahydro-2-hydroxy -1-isopropyl-2-naphthyl)äthyl-p-toluolsulfonat und 1-[4-(Methylamino)butyl]-3-(2-pyridylmethyl) -2-benzimidazolinon das 1-[4-[[2-[[1S,2S]-6-Fluor-1,2,3,4-tetrahydro-2-hydroxy -1-isopropyl-2-naphthyl]äthyl]methylamino]butyl]-3-(2-pyridylmethyl)-2-benzimidazolinon als Oel erhalten.

Das als Ausgangsstoff eingesetzte 1-[4-(Methylamino)-butyl]-3-(2-pyridylmethyl) -2-benzimidazolinon wurde in Analogie zu Beispiel 18 aus tert.-Butyl methyl-[4-(2-oxo-1-benzimidazolinyl)butyl]carbamat hergestellt.

Beispiel 26

In analoger Weise wie in Beispiel 15 beschrieben wurde durch Umsetzen von [1S,2S]-2-(6-Fluor-1,2,3,4-tetrahydro-2-hydroxy -1-isopropyl-2-naphthyl)äthyl-p-toluolsulfonat und 1,3-Dihydro-3-[6-(methylamino)-hexyl]-2H-imidazo[4,5-c]pyridin -2-on das 3-[6-[[2-[[1S,2S]-6-Fluor-1,2,3,4-tetrahydro-2-hydroxy -1-isopropyl-2-naphthyl]äthyl]methylamino]hexyl]-1,3-dihydro-2H-imidazo 4,5-c]pyridin-2-on als Oel erhalten.

Das als Ausgangsmaterial eingesetzte 1,3-Dihydro-3-[6-(methylamino)hexyl]-2H-imidazo[4,5-c]pyridin -2-on wurde wie folgt hergestellt:

In analoger Weise wie in Beispiel 17 beschrieben wurde aus 6-(Methylamino)-1-hexanol das tert.-Butyl methyl-(6-hydroxyhexyl)carbamat als Oel erhalten, welches dann via das ebenfalls als Oel anfallende tert.-Butyl methyl-[6-[(methylsulfonyl)oxy]hexyl]carbamat in das tert.-Butyl methyl-[6-[1-(1-methylvinyl)-1,2-dihydro -2-oxo-3H-imidazo-[4,5-c]pyridin-3-yl]hexyl]carbamat übergeführt wurde; das Produkt wurde wiederum als Oel erhalten.

12,2 g (31,4 mMol) der zuletzt genannten Verbindung werden in 100 ml Aethanol gelöst, mit 13 ml konz. wässriger Salzsäure versetzt und 40 Stunden zum Rückfluss erhitzt. Danach wird unter Eiskühlung mit verdünnter wässriger Natronlauge auf pH 9-10 eingestellt, die Reaktionslösung mit Natriumchlorid gesättigt und 16 Stunden kontinuierlich mit Chloroform extrahiert. Der Extrakt wird über Kaliumcarbonat getrocknet und eingedampft, wobei man 7,2 g (92%) 1,3-Dihydro-3-[6-(methylamino)hexyl]-2H-imidazo[4,5-c]pyridin-2-on als Oel erhält, welches ohne weitere Reinigung weiterverarbeitet wird.

Beispiel 27

In analoger Weise wie in Beispiel 16 beschrieben wurden die folgenden Verbindungen hergestellt:
- [1S,2S]-6-Fluor-1,2,3,4-tetrahydro-1-isopropyl -2-[2-[methyl-[6-(2-oxo-1-benzimidazolinyl)hexyl]amino]-äthyl]-2naphthylmethoxyacetat-hydrochlorid, $[\alpha]_D^{20}$ = +27,8° (c= 1%; Methanol);
- [1S,2S]-6-Fluor-1,2,3,4-tetrahydro-1-isopropyl -2-[2-[methyl-[4-(2-oxo-1 -benzimidazolinyl)butyl]amino]-äthyl] -2-naphthylmethoxyacetat-hydrochlorid, $[\alpha]_D^{20}$ = +28,7° (c= 1%; Methanol);
- [1S,2S]-2-[2-[[6-(1,2-Dihydro-2-oxo -3H-imidazo[4,5-c]-pyridin-3-yl)hexyl]methylamino]äthyl] -6-fluor-1,2,3,4-tetrahydro-1-isopropyl -2-naphthylmethoxyacetat-dihydrochlorid, $[\alpha]_D^{20}$ = +26,0° (c=1%; Methanol).

Beispiel 28

4,6 g (9,3 mMol) 1-[6-[[2-[[1S,2S]-6-Fluor-1,2,3,4-tetrahydro-2-hydroxy -1-isopropyl-2-naphthyl]äthyl]-methylamino]hexyl] -3-methyl-2-benzimidazolinon werden in 15 ml Methylenchlorid gelöst, mit 1,9 ml Pyridin und 6,2 g (38 mMol) Methoxyessigsäureanhydrid versetzt und 20 Stunden bei Raumtemperatur gerührt. Danach wird unter Eiskühlung mit 45 ml 1N wässriger Natronlauge versetzt und 1 Stunde bei 10-15° gerührt. Anschliessend wird das Reaktionsgemisch auf 400 ml Eiswasser gegossen und mit 600 ml Methylenchlorid extrahiert. Der Extrakt wird mit Wasser gewaschen, über Kaliumcarbonat getrocknet, eingedampft, mit einem Aequivalent Chlorwasserstoffsäure in Aethanol versetzt und eingedampft. Dabei erhält man 5,3 g [1S,2S]-6-Fluor-1,2,3,4-tetrahydro-1-isopropyl-2-[2-[methyl -[6-(3-methyl-2-oxo-1-benzimidazolinyl)hexyl]amino]äthyl] -2-naphthylmethoxyacetat-hydrochlorid, $[\alpha]_D^{20}$ = +27,1° (c= 1%; Methanol).

Beispiel 29

In Analogie zu Beispiel 28 wurden die folgenden Verbindungen hergestellt:
- [1S,2S]-6-Fluor-1,2,3,4-tetrahydro -1-isopropyl-2-[2-[[4-[p-(imidazol-1 -yl)phenyl]butyl]methylamino]-äthyl]-2-naphthylmethoxyacetat-oxalat (1:1), $[\alpha]_D^{20}$ = +27,6° (c= 1%; Methanol);
- [1S,2S]-6-Fluor-1,2,3,4-tetrahydro -1-isopropyl-2-[2-[methyl-[4-(3-isopropyl-2-oxo -1-benzimidazolinyl)-butyl]amino]äthyl] -2-naphthylmethoxyacetat-hydrochlorid, $[\alpha]_D^{20}$ = +27,6° (c= 1%; Methanol);
- [1S,2S]-6-Fluor-1,2,3,4-tetrahydro -1-isopropyl-2-[2-[methyl-[6-(3-butyl-2-oxo -1-benzimidazolinyl)-hexyl]-amino]äthyl] -2-naphthylmethoxyacetat-hydrochlorid, $[\alpha]_D^{20}$ = +26,4° (c= 1%; Methanol);
- [1S,2S]-6-Fluor-1,2,3,4-tetrahydro -1-isopropyl-2-[2-[methyl-[6-[3-(2-morpholinoäthyl) -2-oxo-1-benzimidazolinyl]hexyl]amino]äthyl] -2-naphthylmethoxyacetat-dihydrochlorid, $[\alpha]_D^{20}$ = +22,4° (c= 1%; Methanol);
- [1S,2S]-2-[2-[[4-(3-Benzyl-2-oxo -1-benzimidazolinyl)-butyl]methylamino]äthyl]-6-fluor -1,2,3,4-tetrahydro-1-isopropyl -2-naphthylmethoxyacetat-hydrochlorid, $[\alpha]_D^{20}$ = +25,6° (c= 1%; Methanol);
- [1S,2S]-6-Fluor-1,2,3,4-tetrahydro -1-isopropyl-2-[2-[methyl-[4-[2-oxo-3-(2-pyridylmethyl) -1-benzimidazolinyl]butyl]amino]äthyl] -2-naphthylmethoxyacetatdihydrochlorid, $[\alpha]_D^{20}$ = +23,3° (c= 1%; Methanol).

Beispiel 30

1,3 g (3,1 mMol) [1S,2S]-2-[2-[[3-(2-Benzimidazolyl)propyl]methylamino]äthyl] -6-fluor-1,2,3,4-tetrahydro-1-isopropyl-2-naphthalinol in 10 ml Dimethylformamid werden bei Raumtemperatur mit 0,19 g (1,53 mMol) 4-Dimethylaminopyridin, 1,7 ml (12,3 mMol) Triäthylamin und einer Lösung von 0,96 ml (9,24 mMol) Isobuttersäurechlorid in 5 ml Dimethylformamid versetzt und 2 Stunden bei Raumtemperatur gerührt. Danach wird das Reaktionsgemisch auf 20 ml Eiswasser gegossen, mit 10 ml 1N wässriger Natronlauge versetzt, 10 Minuten bei 0° gerührt und mit 100 ml Methylenchlorid extrahiert. Der Extrakt wird mit Wasser gewaschen, über Kaliumcarbonat getrocknet und eingedampft. Das so erhaltene Rohprodukt wird in 20 ml Methanol gelöst, mit 1,5 ml 1N wässriger Natronlauge versetzt, 1 Stunde bei Raumtemperatur gerührt, auf 50 ml Wasser gegossen und mit 100 ml Methylenchlorid extrahiert. Der Extrakt wird mit Wasser gewaschen, über Magnesiumsulfat getrocknet und eingedampft. Der Rückstand wird an 30 g Silicagel mit Methylenchlorid und 1-20% Isopropanol und an 20 g Silicagel mit Methylenchlorid/Isopropanol/25%igem wässrigem Ammoniak (9:1:0,1) chromatographiert. Dabei erhält man 360 mg (21%) [1S,2S]-2-[2-[[3-(2-Benzimidazolyl)propyl]methylamino]äthyl] -6-fluor-1,2,3,4-tetrahydro-1-isopropyl -2-naphthylisobutyrat-dihydrochlorid.

Beispiel 31

Eine Lösung von 2,32 g (0,005 Mol) [1S,2S]-6-Fluor-1,2,3,4-tetrahydro-1-isopropyl-2-[2-[methyl [5-(1-methyl-2-benzimidazolyl)pentyl]amino]äthyl] -2-naphtalinol und 0,6 g (0,005 Mol) Phenylisocyanat in 5 ml Toluol wird mit 7,5 mg Zinn(II)-2-äthylhexanoat versetzt und 15 Stunden auf 100° erhitzt. Nach dem Einengen unter vermindertem Druck wird der ölige Rückstand an 160 g Silicagel mit Methanol/Methylenchlorid (3:2) als Eluier ungsmittel chromatographiert. Das erhaltene ölige Produkt wird in Methylenchlorid gelöst und mit einem Ueberschuss von Chlorwasserstoff in Aether versetzt. Man erhält 2,15 g (65%) [1S,2S]-6-Fluor-1,2,3,4-tetrahydro-1-isopropyl-2 -[2-[methyl-[5-(1-methyl-2-benzimidazolyl)pentyl]-amino]äthyl] -2-naphthylcarbanilat-dihydrochlorid, Smp. 157-160°, als farbloses Kristallpulver.

Beispiel 32

In analoger Weise wie in Beispiel 31 beschrieben wurden die folgenden Verbindungen erhalten:

- [1S,2S]-6-Fluor-1,2,3,4-tetrahydro -1-isopropyl-2-[2-[methyl[5-(1-methyl -2-benzimidazolyl)pentyl]-amino]äthyl] -2-naphthylbutylcarbamat-dihydrochlorid, Smp. 156-158°;
- [1S,2S]-6-Fluor-1,2,3,4-tetrahydro -1-isopropyl-2-[2-[methyl[5-(1-methyl -2-benzimidazolyl)pentyl]-amino]äthyl] -2-naphthylbenzylcarbamat-dihydrochlorid, Smp. 132-136°;
- [1S,2S]-6-Fluor-1,2,3,4-tetrahydro -1-isopropyl-2-[2-[methyl[5-(1-methyl -2-benzimidazolyl)pentyl]-amino]äthyl] -2-naphthyl-p-chlorcarbanilat-dihydrochlorid, Smp. 159-163°.

Beispiel 33

Eine Mischung von 4,67 g (11,5 mMol) [1S,2S]-2-(6-Fluor-1,2,3,4-tetrahydro-2-hydroxy-1-isopropyl -2-naphthyl)äthyl-p-toluolsulfonat, 3,5 g (11,5 mMol) 1-Methyl-2-[3-(methylamino)propyl]-4,5-diphenylimidazol und 1,5 g (11,5 mMol) N-Aethyldiisopropylamin wird 1 Stunde bei 100° gerührt. Die abgekühlte Masse wird zwischen Wasser und Methylenchlorid verteilt, und die organische Phase mit einer gesättigten wässrigen Lösung von Natriumchlorid gewaschen, über Natriumsulfat getrocknet und zur Trockene eingeengt. Das verbleibende Oel wird an 400 g Silicagel mit Methylenchlorid/Methanol (4:1) als Eluierungsmittel chromatographiert. Das gereinigte Kondensationsprodukt (5,3 g Oel) wird in 15 ml Methoxyessigsäureanhydrid gelöst, mit 0,85 ml Pyridin versetzt, und die Lösung 2 Stunden bei 70° gerührt. Das abgekühlte Reaktionsgemisch wird zwischen 400 ml Methylen chlorid und 400 ml 3N wässrige Natronlauge verteilt, und das Gemisch 15 Minuten bei Raumtemperatur intensiv gerührt. Die abgetrennte wässrige Phase wird wiederum mit 400 ml Methylenchlorid extrahiert, die vereinigten Extrakte mit einer gesättigten wässrigen Lösung von Natriumchlorid gewaschen, über Natriumsulfat getrocknet und zur Trockene eingeengt. Der ölige Rückstand wird an 350 g Silicagel mit Methylenchlorid/Methanol (9:1) als Eluierungsmittel chromatographiert. Das aus den homogenen Fraktionen erhaltene Oel wird in Essigester gelöst und mit einem Ueberschuss von Chlorwasserstoff in Aether versetzt. Das Kristallisat wird abfiltriert, mit Aether gewaschen und getrocknet. Man erhält 4,0 g (51%) [1S,2S]-6-Fluor-1,2,3,4-tetrahydro-1-isopropyl-2-[2-[[3 -(1-methyl-4,5-diphenylimidazol-2-yl)propyl]-methylamino]äthyl] -2-naphthylmethoxyacetat-dihydrochlorid, Smp. 185-189°, als fast farbloses Kristallpulver.

Das als Ausgangsmaterial verwendete 1-Methyl-2-[3-(methylamino)propyl]-4,5-diphenylimidazol wurde wie folgt hergestellt:

Zur Lösung von 7,0 g (0,024 Mol) 4,5-Diphenylimidazol-2-propionsäure und 3,36 ml (0,024 Mol) Triäthylamin in 80 ml Dimethylformamid werden bei -5° 3,2 ml (0,024 Mol) Chlorameisensäureisobutylester getropft. Nach 30-minütigem Rühren bei 0-5° werden 1,64 g (0,024 Mol) Methylamin-hydrochlorid und 3,36 ml (0,024 Mol) Triäthylamin in 32 ml Dimethylformamid und 1,65 ml Wasser gegeben. Man lässt dann die Temperatur auf Raumtemperatur steigen und rührt 20 Stunden weiter. Nach dem Einengen unter vermindertem Druck wird der Rückstand in 250 ml Methanol aufgekocht und mit 3,6 ml (0,024 Mol) 1,8-Diazabicyclo-[5.4.0]undec-7-en (DBU) versetzt, wobei eine klare Lösung entsteht. Durch Abkühlen im Eisbad kristallisieren 4,8 g N-Methyl-4,5-diphenylimidazol-2-propionamid, Smp. 195-200° (Zers.). Aus der Mutterlauge erhält man durch Einengen und Behandeln mit Wasser weitere 2 g desselben Produktes, Smp. 195-200°. Gesamtausbeute; 6,8 g (93%).

Zu einer gerührten Suspension von 2,3 g (0,06 Mol) Lithiumaluminiumhydrid in 160 ml Tetrahydrofuran gibt man portionenweise 9,15 g (0,03 Mol) N-Methyl-4,5-diphenylimidazol-2-propionamid und erhitzt anschliessend 4 Stunden zum Rückfluss. Bei 5-10° werden tropfenweise 6 ml Wasser, dann 9 ml einer 10%igen Lösung von Kaliumhydroxyd und wiederum 6 ml Wasser zugegeben. Der Niederschlag wird abfiltriert und dreimal mit je 50 ml Tetrahydrofuran ausgekocht. Die vereinigten Filtrate werden mit einer

gesättigten wässrigen Lösung von Natriumchlorid gewaschen, über Natriumsulfat getrocknet und unter vermindertem Druck zur Trockene eingeengt. Der ölige Rückstand wird an 200 g Silicagel zuerst mit Chloroform/Aethanol (9:1), dann mit Methanol als Eluierungsmittel chromatographiert. Die ersten eluierten homogenen Fraktionen ergeben nach Eindampfen und Anreiben mit Aether 1,2 g Ausgangsmaterial. Die folgenden eluierten homogenen Fraktionen liefern nach gleicher Behandlung 5,5 g (73%) 2-[3-(Methylamino)propyl]-4,5-diphenylimidazol in Form von farblosen Kristallen, Smp. 110-113°.

Eine Lösung von 5,25 g (0,018 Mol) 2-[3-(Methylamino)propyl]-4,5-diphenylimidazol und 3,8 ml (0,028 Mol) Chlorameisensäurebenzylester in 38 ml Dimethylformamid wird mit 5 g feingeriebenem trockenem Kaliumcarbonat versetzt und hierauf während 1 Stunde bei Raumtemperatur intensiv gerührt. Die anorganischen Salze werden dann abfiltriert, mit Methylenchlorid gewaschen, und das Filtrat unter vermindertem Druck zur Trockene eingeengt. Der ölige Rückstand wird an 500 g Silicagel mit Essigester als Eluierungsmittel chromatographiert. Die homogenen Fraktionen ergeben nach Eindampfen und Anreiben des Rückstandes mit Hexan 6,5 g (85%) 2-[3-(N-Benzyloxycarbonylmethylamino)propyl]-4,5-diphenylimidazol in Form von farblosen Kristallen, Smp. 105-108°.

Eine Lösung von 6,4 g (0,015 Mol) 2-[3-(N-Benzyloxycarbonylmethylamino)propyl] -4,5-diphenylimidazol in 120 ml Dimethylformamid wird unter Argon bei 15-20° mit 0,018 Mol Natriumhydrid (0,8 g einer 55%igen Dispersion in Mineralöl) versetzt und hierauf 30 Minuten bei Raumtemperatur weitergerührt. Man gibt bei 15-20° innert 15 Minuten eine Lösung von 1,85 ml (0,03 Mol) Methyljodid in 10 ml Dimethylformamid zu und rührt 3 Stunden bei Raumtemperatur weiter. Nach dem Einengen unter vermindertem Druck wird der Rückstand zwischen Eiswasser und Essigester verteilt. Die über Natriumsulfat getrocknete organische Phase wird eingedampft, und das verbleibende Oel an 100 g Silicagel mit Essigester als Eluierungsmittel chromatographiert. Das erhaltene 2-[3-(N-Benzyloxycarbonylmethylamino)propyl]-1-methyl-4,5-diphenylimidazol (6,4 g Oel) wird in 300 ml Methanol gelöst und bei Raumtemperatur und Normaldruck in Gegenwart von 1 g 5%igem Palladium auf Aktivkohle hydriert. Das in üblicher Weise isolierte Rohprodukt wird an 70 g Silicagel mit Methanol/konz. Ammoniumhydroxyd (100:1) als Eluierungsmittel chromatographiert. Man erhält 2,95 g (64%) 1-Methyl-2-[3-(methylamino)propyl]-4,5-diphenylimidazol als dickes Oel.

Beispiel 34

In analoger Weise wie in Beispiel 33 beschrieben wird [1S,2S]-2-(6-Fluor-1,2,3,4-tetrahydro-2-hydroxy-1-isopropyl-2-naphthyl) thyl-p-toluolsulfonat zuerst mit 2-[3-(Methylamino)propyl]-4,5-diphenylimidazol und dann mit Methoxyessigsäureanhydrid umgesetzt. Man erhält [1S,2S]-6-Fluor-1,2,3,4-tetrahydro-1-isopropyl-2-[2-[[3 -(4,5-diphenylimidazol-2-yl)propyl]methylamino]äthyl] -2-naphthylmethoxyacetat-dihydrochlorid, Smp. 160-164°, als farbloses Kristallpulver.

Beispiel 35

In analoger Weise wie in Beispiel 33 beschrieben wird [1S,2S]-2-(6-Fluor-1,2,3,4-tetrahydro-2-hydroxy-1-isopropyl-2-naphthyl) thyl-p-toluolsulfonat zuerst mit 2-[4-[(Methylamino)methyl]benzyl]-1-methyl-benzimidazol und dann mit Methoxyessigsäureanhydrid umgesetzt. Man erhält [1S,2S]-6-Fluor-1,2,3,4-tetrahydro-1-isopropyl-2-[2-[[4 -[(1-methyl-2-benzimidazolyl)methyl]benzyl]methylamino]äthyl]2-naphthyl ethoxyacetat-dihydrochlorid, Smp. 130-134°, als fast farbloses Kristallpulver.

Das als Ausgang smaterial verwendete 2-[4-[(Methylamino)methyl]benzyl]-1-methylbenzimidazol wurde wie folgt hergestellt:

Eine Mischung von 30 g (0,277 Mol) o-Phenylendiamin und 150 g Polyphosphorsäureester (PPE) wird auf 120° erhitzt. Wenn das Diamin gelöst ist, gibt man auf einmal 33 g (0,205 Mol) p-Cyanphenylessigsäure zu und erhitzt 20 Minuten weiter auf 120°. Nach dem Abkühlen auf Raumtemperatur wird die zähe Masse mit ca. 1 l Wasser versetzt und mit festem Natriumhydrogencarbonat schwach basisch gestellt. Das Gemisch wird mit Methylenchlorid extrahiert, und der Extrakt mit Wasser gewaschen, über Natriumsulfat getrocknet und zur Trockene eingedampft. Umkristallisation des Rückstandes aus Methylenchlorid/Essigester ergibt 29 g (60%) 2-(p-Cyanbenzyl)benzimidazol, Smp. 201-203°, als farbloses Kristallpulver.

Eine Lösung von 21,9 g 2-(p-Cyanbenzyl)benzimidazol in einem Gemisch von 140 ml Methanol und 140 ml flüssigem Ammoniak wird bei Raumtemperatur und 30 bar in Gegenwart von 5 g Raney-Nickel hydriert. Das in üblicher Weise isolierte Rohprodukt wird an 400 g Silicagel mit Methanol als Eluierungsmittel chromatographiert. Die homogenen Fraktionen ergeben nach Eindampfen und Anreiben des Rückstandes mit Aether 14,7 g (66%) 2-[p-(Aminomethyl)benzyl]benzimidazol, Smp. 133-136°, als hellbraunes Kristallpulver.

Eine Lösung von 9,2 g (0,04 Mol) 2-[p-(Aminomethyl)benzyl]benzimidazol und 8,4 ml (0,06 Mol) Chlorameisensäurebenzylester in 80 ml Dimethylformamid wird mit 10 g feingeriebenem trockenem Kaliumcarbonat versetzt und hierauf 30 Minuten bei Raumtemperatur intensiv gerührt. Man gibt dann 12 ml (0,08 Mol) 1,8-Diazabicyclo[5.4.0]undec-7-en (DBU) zu und rührt bei Raumtemperatur 30 Minuten weiter. Die anorganischen Salze werden abfiltriert, mit Methylenchlorid nachgewaschen, und das Filtrat unter vermindertem Druck zur Trockene eingeengt. Der ölige Rückstand wird an 600 g Silicagel zuerst mit Methylenchlorid/Essigester (4:1) und dann mit Chloroform/Aethanol (9:1) als Eluierungsmittel chromatographiert. Die mit Chloroform/Aethanol eluierten Fraktionen ergeben nach Eindampfen und Anreiben mit Essigester 10,8 g (73%) [4-[2-(Benzimidazolyl)methyl]benzyl]carbamidsäurebenzylester, Smp. 190-194°, als farbloses Kristallpulver.

Eine Lösung von 8,9 g (0,024 Mol) [4-[2-(Benzimidazolyl)methyl]benzyl]carbamidsäurebenzylester in 210 ml Dimethylformamid wird unter Argon bei 15-20° mit 0,056 Mol Natriumhydrid (2,5 g einer 55%igen Dispersion in Mineralöl) versetzt und hierauf 30 Minuten bei Raumtemperatur weitergerührt. Man gibt bei 15-20° innert 20 Minuten eine Lösung von 7,4 ml (0,12 Mol) Methyljodid in 22 ml Dimethylformamid zu und rührt 10 Minuten bei Raumtemperatur weiter. Nach dem Einengen unter vermindertem Druck wird der Rückstand zwischen Eiswasser und Essigester verteilt. Die über Natriumsulfat getrocknete organische Phase wird eingedampft, und das verbleibende Oel an 300 g Silicagel mit Methylenchlorid/Essigester (1:1) als Eluierungsmittel chromatographiert. Die ersten eluierten homogenen Fraktionen liefern nach Eindampfen und Anreiben mit Aether 4,5 g (45%) [4-[1-(1-Methyl-2 -benzimidazolyl)äthyl]benzyl]-methylcarbamidsäurebenzylester, Smp. 131-133°, als farbloses Kristallpulver. Die folgenden eluierten homogenen Fraktionen ergeben nach Einengen 3,5 g (37%) [4-[(1-Methyl-2 -benzimidazolyl)methyl]benzyl]-methylcarbamidsäurebenzylester, als dickes Oel.

3,5 g [4-[(1-Methyl-2 -benzimidazolyl)methyl]benzyl]methylcarbamidsäurebenzylester werden in 600 ml Methanol gelöst und bei Raumtemperatur und Normaldruck nach Zusatz von 1 g 5%igem Palladium auf Aktivkohle hydriert. Das in üblicher Weise isolierte Rohprodukt wird an 150 g Silicagel mit Methanol/konz. Ammoniumhydroxyd (100:1) als Eluierungsmittel chromatographiert. Man erhält 2,1 g (90%) 2-[4-[(Methylamino)methyl]benzyl]-1-methylbenzimidazol als dickes Oel.

Beispiel 36

In analoger Weise wie in Beispiel 33 beschrieben wird [1S,2S]-2-(6-Fluor-1,2,3,4-tetrahydro-2-hydroxy-1-is opropyl-2-naphthyl)äthyl-p-toluolsulfonat zuerst mit 2-[1-[4-[(Methylamino)methyl]phenyl]äthyl] -1-methyl-benzimidazol und dann mit Methoxyessigsäureanhydrid umgesetzt. Man erhält [1S,2S]-6-Fluor-1,2,3,4-tetrahydro-1-isopropyl-2-[2-[[4-[1-(1-methyl-2-enzimidazolyl)äthyl]benzyl]methylamino]äthyl]2-naphthylmethoxyacetat-dihydrochlorid (Gemisch von 2 Epimeren), Smp: 95-105°, als fast farbloses Kristallpulver.

Das als Ausgangsmaterial verwendete 2-[1-[4-[(Methylamino)methyl]phenyl]äthyl] -1-methylbenzimidazol wurde in analoger Weise in Beispiel 35 angegeben durch Hydrierung von [4-[1-(1-Methyl-2 -benzimidazolyl)äthyl]benzyl]methylcarbamidsäurebenzylester hergestellt.

Beispiel 37

In analoger Weise wie in Beispiel 33 beschrieben wird [1S,2S]-2-(6-Fluor-1,2,3,4-tetrahydro-2-hydroxy-1-isopropyl-2-naphthyl) thyl-p-toluolsulfonat zuerst mit 2-[4-[(Methylamino)methyl]benzyl]benzimidazol, und dann mit Methoxyessigsäureanhydrid umgesetzt. Man erhält [1S,2S]-6-Fluor-1,2,3,4-tetrahydro-1-isopropyl-2-[2 -[[4-[(2-benzimidazolyl)methyl]benzyl]methylamino]äthyl] -2-naphthylmethoxyacetat-dihydrochlorid, Smp. 146-150°, als fast farbloses Kristallpulver.

Das als Ausgangsmaterial verwendete 2-[4-[(Methylamino)methyl]benzyl]benzimidazol wurde wie folgt hergestellt:

6,6 g (0,028 Mol) 2-(p-Cyanbenzyl)benzimidazol werden in 110 ml 1N Natronlauge 2 Stunden zum Rückfluss erhitzt. Die erhaltene Lösung wird abgekühlt und mit zweimal 100 ml Essigester und zweimal 100 ml Methylenchlorid extrahiert. Die wässerige Phase wird mit 2N Salzsäure auf pH 6,0 gestellt und 30 Minuten im Eisbad stehen gelassen. Der Niederschlag wird abgenutscht und mit Aether gewaschen. Man erhält 5,8 g (83%) p-[(2-Benzimidazolyl)methyl]benzoesäure, Smp. 265-267°, als farbloses Pulver.

Zur Lösung von 5,0 g (0,020 Mol) p-[(2-Benzimidazolyl)methyl]benzoesäure und 2,8 ml (0,020 Mol) Triäthylamin in 68 ml Dimethylformamid werden bei -5° 2,8 ml Chlorameisensäureisobutylester getropft. Nach 30-minütigem Rühren bei 0-5° werden 1,32 g (0,020 Mol) Methylamin-hydrochlorid und 2,8 ml (0,020 Mol) Triäthylamin in 28 ml Dimethylformamid und 1,4 ml Wasser zugegeben. Man lässt dann die

Temperatur auf Raumtemperatur steigen und rührt 18 Stunden weiter. Nach dem Einengen unter vermindertem Druck wird der Rückstand an 400 g Silicagel mit Chloroform/Aethanol (4:1) als Eluierungsmittel chromatographiert. Die einheitlichen Fraktionen ergeben 2,0 g (38%) N-Methyl-p-[(2-benzimidazolyl)methyl]-benzamid, Smp. 250-255° (Zers.), als farbloses Pulver.

Zu einer gerührten Suspension von 0,58 g (0,0075 Mol) Lithiumaluminiumhydrid in 40 ml Tetrahydrofuran gibt man portionenweise 1,98 g (0,0075 Mol) N-Methyl-p-[(2-benzimidazolyl)methyl]benzamid und erhitzt anschliessend 4 Stunden zum Rückfluss. Bei 5-10° werden tropfenweise 1,5 ml Wasser, dann 2,3 ml einer 10%igen Lösung von Kaliumhydroxy und widerum 1,5 ml Wasser zugegeben. Der Niederschlag wird abfiltriert und dreimal mit je 20 ml Tetrahydrofuran ausgekocht. Die vereinigten Filtrate werden mit einer gesättigten wässrigen Lösung von Natriumchlorid gewaschen, über Natriumsulfat getrocknet und unter vermindertem Druck zur Trockene eingeengt. Der Rückstand wird an 150 g Silicagel mit Methanol/konz. Ammoniumhydroxyd (100:1) als Eluierungsmittel chromatographiert. Man erhält 1,58 g (84%) 2-[4-[-(Methylamino)methyl]benzyl]benzimidazol, Smp. 157-160°, als farbloses Kristallpulver.

Beispiel 38

In analoger Weise wie in Beispiel 33 beschrieben wird [1S,2S]-2-(6-Fluor-1,2,3,4-tetrahydro-2-hydroxy-1-isopropyl-2-naphthyl) thyl-p-toluolsulfonat zuerst mit 2-[trans-4-[(Methylamino)methyl]cyclohexyl]-benzimidazol und dann mit Methoxyessigsäureanhydrid umgesetzt. Man erhält [1S,2S]-6-Fluor-1,2,3,4-tetrahydro-1-isopropyl-2-[2 -[methyl[trans-4-(2 -benzimidazolyl)cyclohexyl]methylamino]äthyl] -2-naphthylmethoxyacetat-dihydrochlorid, Smp. 150-153°, als farbloses Kistallpulver.

Das als Ausgangsmateri al verwendete 2-[trans-4-[(Methylamino)methyl]cyclohexyl]benzimidazol wurde wie folgt hergestellt:

Eine Lösung von 20,3 g (0,07 Mol) trans-4-(N-Benzyloxycarbonyl-aminomethyl)cyclohexancarbonsäure in 380 ml Dimethylformamid wird unter Argon bei 15-20° mit 0,21 Mol Natriumhydrid (9,35 g einer 55%igen Dispersion in Mineralöl) versetzt und hierauf 30 Minuten bei Raumtemperatur weitergerührt. Man gibt bei 25-30° innert 20 Minuten eine Lösung von 17,5 ml (0,28 Mol) Methyljodid in 20 ml Dimethylformamid zu und rührt 1 Stunde bei 70° weiter. Nach dem Einengen unter vermindertem Druck wird der Rückstand zwischen Wasser und Methylenchlorid verteilt. Die organische Phase wird eingedampft, und das verbleibende Oel in einem Gemisch von 350 ml Aethanol und 350 ml 1N Natronlauge gelöst. Man erhitzt 1 Stunde zum Rückfluss, kühlt ab und giesst in 700 ml Eiswasser. Die Lösung wird mit Essigester extrahiert und dann mit 6N Salzsäure angesäuert. Die freigesetzte Säure wird mit Methylenchlorid extrahiert, und der Extrakt über Natriumsulfat getrocknet und eingedampft. Das verbleibende Oel wird an 270 g Silicagel mit einem Gemisch Methylenchlorid/Essigester (4:1) als Eluierungsmittel chromatographiert. Man erhält 13,6 g (64%) trans-4-(N-Benzyloxycarbonyl-N-methyl -aminomethyl)cyclohexancarbonsäure als dickes Oel.

Eine Lösung von 13,6 g (0,044 Mol) trans-4-(N-Benzyloxycarbonyl-N-methyl -aminomethyl)-cyclohexancarbonsäure und 9,5 ml (0,068 Mol) Triäthylamin in 110 ml Tetrahydrofuran wird bei -15° innert 30 Minuten mit 6,5 ml (0,049 Mol) Chlorameisensäureisobutylester versetzt. Dann wird bei -15° innert 45 Minuten eine Lösung von 5,8 g (0,053 Mol) o-Phenylendiamin zugetropft. Man rührt 1 Stunde bei Raumtemperatur weiter und lässt 20 Stunden stehen. Nach dem Einengen unter vermindertem Druck wird der Rückstand zwischen Wasser und Essigester verteilt, und die organische Phase mit einer 5%igen Lösung von Natriumhydrogencarbonat, dann mit einer gesättigten wässrigen Lösung von Natriumchlorid und schliesslich mit Wasser gewaschen. Die über Magnesiumsulfat getrocknete Lösung wird eingedampft und mit Aether angerieben. Man löst das erhaltene feste Produkt (9,3 g) in 200 ml Toluol, gibt 3 g p-Toluolsulfonsäure zu und erhitzt 4 Stunden zum Rückfluss mit einem Wasserabscheider. Die Lösung wird abgekühlt, mit einer 2N Natriumcarbonatlösung und einer gesättigten wässerigen Lösung von Natriumchlorid gewaschen, über Magnesiumsulfat getrocknet und unter vermindertem Druck zur Trockene eingeengt. Der feste Rückstand wird aus Essigester umkristallisiert. Man erhält 5,6 g [[trans-4-(2 -Benzimidazolyl)-cyclohexyl]methyl]methylcarbamidsäurebenzylester, Smp. 146-148°, als farbloses Kristallpulver. Die Mutterlauge ergibt nach Chromatographie an 250 g Silicagel mit Essigester/Methylenchlorid (9:1) als Eluierungsmittel weitere 1,1 g desselben Produktes, Smp. 146-148°. Gesamtausbeute: 6,7 g (40%).

6,0 g [[trans-4-(2 -Benzyimidazolyl)cyclohexyl]methyl]methylcarbamidsäurebenzylester werden in 600 ml Aethanol gelöst und bei Raumtemperatur und Normaldruck nach Zusatz von 1 g 5%igem Palladium auf Aktivkohle hydriert. Das in üblicher Weise isolierte Rohprodukt wird aus Methylenchlorid/Aether umkristallisiert. Man erhält 3,0 g (78%) 2-[trans-4-[(Methylamino)methyl]cyclohexyl]benzimidazol, Smp. 232-235°, als farbloses Kristallpulver.

Beispiel 39

In analoger Weise wie in Beispiel 33 beschrieben wird [1S,2S]-2-(6-Fluor-1,2,3,4-tetrahydro-2-hydroxy-1-isopropyl2-naphthyl)ä hyl-p-toluolsulfonat zuerst mit 2-[trans-4-[(Methylamino)methyl]cyclohexyl] -1-methyl-benzimidazol und dann mit Methoxyessigsäureanhydrid umgesetzt. Man erhält [1S,2S]-6-Fluor-1,2,3,4-tetrahydro-1-isopropyl-2-[2[methyl[trans-4-(1-methyl-2 -benzimidazolyl)cyclohexyl]methylamino]-äthyl] -2-naphthylmethoxyacetat-dihydrochlorid, Smp. 148-152°, als farbloses Kristallpulver.

Das als Ausgangsmaterial verwendete 2-[trans-4-[(Methylamino)methyl]cyclohexyl] -1-methyl-benzimidazol wurde wie folgt hergestellt:

Eine Lösung von 7,2 g (0,019 Mol) [[trans-4-(2-Benzimidazolyl)cyclohexyl]methyl]-methylcarbamidsäurebenzyl ster in 160 ml Dimethylformamid wird unter Argon bei 15-20° mit 0,023 Mol Natriumhydrid (1,0 g einer 55%igen Dispersion in Mineralöl) versetzt und hierauf 30 Minuten bei Raumtemperatur weitergerührt. Man gibt bei 15-20° innert 15 Minuten einer Lösung von 2,3 ml (0,038 Mol) Methyljodid in 10 ml Dimethylformamid zu und rührt 3 Stunden bei Raumtemperatur weiter. Nach dem Einengen unter vermindertem Druck wird der Rückstand zwischen Eiswasser und Essigester verteilt. Die über Natriumsulfat getrocknete organische Phase wird eingedampft, und der feste Rückstand aus Essigester/Aether umkristallisiert. Man erhält 5,9 g (79%) [[trans-4-(1-Mehyl-2 -benzimidazolyl)cyclohexyl]-methyl]methylcarbamidsäurebenzylester, Smp. 141-142°, als farbloses Kristallpulver.

5,9 g [[trans-4-(1-Methyl-2 -benzimidazolyl)cyclohexyl]methyl]methylcarbamidsäurebenzylester werden in 600 ml Aethanol gelöst und bei Raumtemperatur und Normaldruck nach Zusatz von 1 g 5%igem Palladium auf Aktivkohle hydriert. Das in üblicher Weise isolierte Rohprodukt wird an 250 g Silicagel zuerst mit Methylenchlorid/Methanol (1:1) und dann mit Methanol/konz. Ammoniumhydroxyd (100:1) als Elutionsmittel chromatographiert. Man erhält 3,3 g (85%) 2-[trans-4-[(Methylamino)methyl]cyclohexyl] -1-methyl-benzimidazol, als dickes Oel.

Beispiel 40

In analoger Weise wie in Beispiel 7 beschrieben wurde durch Umsetzen von 2-(6-Fluor-1,2,3,4-tetrahydro-2-hydroxy-1α-isopropyl-2β -naphthyl)äthyl]-p-toluolsulfonat und 3,4-Dihydro-4-methyl-1-[4-(methylamino)butyl]-2H -1,4-benzodiazepin-2,5-(1H)-dion das 1-[4-[[2-[[1S,2S]-6-Fluor-1,2,3,4-tetrahydro-2-hydroxy -1-isopropyl-2-naphthyl]äthyl]methylamino]butyl] -3,4-dihydro-4-methyl-2H-1,4-benzodiazepin-2,5-(1H)-dion erhalten, MS: $M^+$ 509.

In analoger Weise wie oben beschrieben wurde ausgehend von 2-(6-Fluor-1,2,3,4-tetrahydro-2-hydroxy-1α-isopropyl-2β -naphthyl)äthyl]-p-toluolsulfonat und (S)-6-Chlor-1,2,3,11a-tetrahydro-5H -pyrrolo[2,1-c][1,4]-benzodiazepin-5,11-(10H)-dion das (S)-6-Chlor-10-[4-[[2-[[1S,2S]-6-fluor-1,2,3,4-tetrahydro -2-hydroxy-1-isopropyl-2-naphthyl]äthyl]methylamino]butyl] -1,2,3,11a-tetrahydro-5H-pyrrolo2,1-c][1,4]benzodiazepin -5,11-(10H)-dion hergestellt, MS: $M^+$ 570.

Das als Ausgangsstoff eingesetzte 3,4-Dihydro-4-methyl-1-[4-(methylamino)butyl]-2H -1,4-benzodiazepin-2,5-(1H)-dion wurde wie folgt hergestellt:

10 g (40 mMol) 4-[1-(Benzyloxy)-N-methylformamido]-buttersäure werden in 200 ml Aethanol gelöst und mit 1 ml konz. Schwefelsäure versetzt. Danach erhitzt man das Reaktionsgemisch 4 Stunden zum Rückfluss und dampft anschliessend das Lösungsmittel ab. Dann wird das Reaktionsprodukt mit Methylenchlorid/gesättigter Natriumbicarbonatlösung extrahiert. Nach dem Trocknen und Eindampfen des Extraktes erhält man 9,24 g eines braunes Oels, welches in 200 ml Tetrahydrofuran gelöst, mit 7,1 ml 10M Bormethylsulfidkomplex versetzt und 2 Stunden zum Rückfluss erhitzt wird. Danach lässt man das Reaktionsgemisch über Nacht bei Raumtemperatur stehen und gibt dann langsam soviel Methanol zu, dass keine Gasentwicklung mehr stattfindet. Man erhält auf diese Weise eine klare Lösung, welche eingedampft wird. Der erhaltene Rückstand (8,09 g) wird mit einem 1:1-Gemisch von Essigester und Hexan an Silicagel chromatographiert, wobei man 6,82 g (72%) Benzyl (4-hydroxyabutyl)methylcarbamat erhält, welches direkt in die nächste Stufe eingesetzt wird.

6,75 g (28,4 mMol) des oben erhaltenen Carbamates und 10,0 g (52,5 mMol) p-Toluolsulfonsäurechlorid werden bei 0° in 25 ml Pyridin gelöst. Nach 6 stündigem Stehenlassen wird Eis zugegeben und mit Aether extrahiert. Der Aetherextrakt wird mit 4N Salzsäure, gesättigter Natriumbicarbonatlösung und gesättigter Kochsalzlösung gewaschen, getrocknet und eingedampft. Man erhält auf diese Weise 9,38 g (84%) eines gelblichen Oels von Benzyl methyl-[4-[(p-toluolsulfonyl)oxy]butyl]carbamat, welches direkt weiterverarbeitet wird.

1,9 g (10 mMol) 4-Methyl-3H-1,4-benzodiazepin-2,5-(1H,4H)-dion werden in 20 ml Dimethylformamid gelöst und zu einer Suspension von 430 mg (10 mMol) 55%igem Natriumhydrid in 50 ml Dimethylformamid gegeben. 30 Minuten nach der Zugabe wird eine Lösung von 3,91 g (10 mMol) Benzyl methyl-[4-[(p-toluolsulfonyl)oxy]butyl]carbamat in 20 ml Dimethylformamid zugegeben, und das ganze Reaktionsgemisch

20 Stunden bei Raumtemperatur gerührt. Danach wird unter vermindertem Druck bei 50° das Lösungsmittel abgedampft und anschliessend Wasser zugegeben. Nach zweimaligem Extrahieren mit Methylenchlorid wird das Lösungsmittel erneut abgedampft, und der Rückstand mit einem 20:1-Gemisch von Methylenchlorid und Methanol an Silicagel chromatographiert, wobei man 3,92 g (95,8%) Benzyl methyl-[4-(2,3,4,5-tetrahydro-4-methyl-2,5 -dioxo-1H-1,4-benzodiazepin-1-yl)butyl]carbamat erhält, MS: $M^+$ 409.

Das obige Carbamat wird in analoger Weise wie in Beispiel 7, letzter Absatz, beschrieben in das erwünschte 3,4-Dihydro-4-methyl-1-[4-(methylamino)butyl]-2H -1,4-benzodiazepin-2,5-(1H)-dion übergeführt, das direkt in die nächste Stufe eingesetzt wird.

In analoger Weise wie oben beschrieben wurde ausgehend von Benzyl methyl-[4-[(p-toluolsulfonyl)oxy]-butyl]carbamat durch Umsetzen mit dem entsprechenden Benzodiazepin das (S)-6-Chlor-1,2,3,11a-tetrahydro-10-[4-(methylamino)butyl]-5H-pyrrolo[2 1-c][1,4]benzodiazepin-5,11-(10H)-dion hergestellt.

Beispiel 41

In analoger Weise wie in Beispiel 8 beschrieben wurden durch Methoxyacetylierung der entsprechenden Hydroxyderivate die folgenden Verbindungen hergestellt:

- [1S,2S]-6-Fluor-1,2,3,4-tetrahydro -1-isopropyl-2-[2-[[4-(2,3,4,5-tetrahydro-4-methyl -2,5-dioxo-1H-1,4-benzodiazepin -1-yl)butyl]methylamino]äthyl] -2-naphthylmethoxyacetat-hydrochlorid,

$$[\alpha]_{589}^{20} = +28,2°$$

(c = 0,5%; Methanol);

- [1S,2S]-2-[2-[[4-[(S)-6-Chlor -2,3,11,11a-tetrahydro-5,11-dioxo-1H-pyrrolo[2,1-c][1,4]benzodiazepin-10-(5H)-yl]butyl]methylamino]äthyl]-6-fluor -1,2,3,4-tetrahydro-1-isopropyl -2-naphthylmethoxyacetat-hydrochlorid,

$$[\alpha]_{589}^{20} = +215,2°$$

(c = 0,5%; Methanol).

Beispiel A

| Tabletten | |
|---|---|
| Zusammensetzung: | |
| 1) 2-[2-[[3-(2-Benzimidazolyl)propyl]methylamino]äthyl]-6-fluor-1,2,3,4-tetrahydro-1α-isopropyl-2α-naphthylmethoxyacetathydrochlorid | 75 mg |
| 2) Milchzucker pulv. | 135 mg |
| 3) Maisstärke weiss | 55 mg |
| 4) Povidone K 30 | 15 mg |
| 5) Maisstärke weiss | 15 mg |
| 6) Talk | 3 mg |
| 7) Magnesiumstearat | 2 mg |
| Tablettengewicht | 300 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel B

**Tabletten**

| Zusammensetzung: | | |
|---|---|---|
| 1) 2-[2-[[3-(2-Benzimidazolyl)propyl]methylamino]äthyl]-6-fluor-1,2,3,4-tetrahydro-1α-isopropyl-2α-naphthylmethoxyacetathydrochlorid | 75 mg | 60 mg |
| 2) Milchzucker pulv. | 100 mg | 100 mg |
| 3) Maisstärke | 60 mg | 60 mg |
| 4) Povidone K 30 | 5 mg | 5 mg |
| 5) Maisstärke | 15 mg | 15 mg |
| 6) Natriumcarboxymethylstärke | 5 mg | 5 mg |
| 7) Talk | 3 mg | 3 mg |
| 8) Magnesiumstearat | 2 mg | 2 mg |
| Tablettengewicht | 265 mg | 250 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-8 gemischt und zu Tabletten geeigneter Grösse verpresst.

Beispiel C

| Tabletten | | |
|---|---|---|
| Zusammensetzung: | | |
| 1) 2-[2-[[3-(2-Benzimidazolyl)propyl]methylamino]äthyl]-6-fluor-1,2,3,4-tetrahydro-1α-isopropyl-2α-naphthylmethoxyacetathydrochlorid | 75 mg | 90 mg |
| 2) Milchzucker pulv. | 46 mg | 46 mg |
| 3) Cellulose mikrokristallin | 60 mg | 60 mg |
| 4) Povidone K 30 | 10 mg | 10 mg |
| 5) Natriumcarboxymethylstärke | 4 mg | 4 mg |
| 6) Talk | 3 mg | 3 mg |
| 7) Magnesiumstearat | 2 mg | 2 mg |
| Tablettengewicht | 200 mg | 215 mg |

Herstellungsverfahren:

1-3 werden intensiv gemischt. Die Mischung wird danach mit einer wässrigen Lösung von 4 befeuchtet und geknetet, und die resultierende Masse granuliert, getrocknet und gesiebt. Das Granulat wird mit 5-7 gemischt und zu Tabletten geeigneter Grösse verpresst.

Beispiel D

| Kapseln | |
|---|---|
| Zusammensetzung: | |
| 1) 2-[2-[[3-(2-Benzimidazolyl)propyl]methylamino]äthyl]-6-fluor-1,2,3,4-tetrahydro1α-isopropyl-2α-naphthylmethoxyacetathydrochlorid | 75 mg |
| 2) Milchzucker krist. | 100 mg |
| 3) Maisstärke weiss | 20 mg |
| 4) Talk | 9 mg |
| 5) Magnesiumstearat | 1 mg |
| Kapselfüllgewicht | 205 mg |

Herstellungsverfahren:

Der Wirkstoff wird mit dem Milchzucker intensiv gemischt. Dieser Mischung wird danach die Maisstärke, der Talk und das Magnesiumstearat zugemischt, und das Gemisch in Kapseln geeigneter Grösse abgefüllt.

Beispiel E

| Kapseln | |
|---|---|
| Zusammensetzung: | |
| 1) 2-[2-[[3-(2-Benzimidazolyl)propyl]methylamino]äthyl]-6-fluor-1,2,3,4-tetrahydro-1α-isopropyl-2α-naphthylmethoxyacetathydrochlorid | 75 mg |
| 2) Cellulose mikrokristallin | 100 mg |
| 3) Natriumcarboxymethylstärke | 5 mg |
| 4) Talk | 9 mg |
| 5) Magnesiumstearat | 1 mg |
| Kapselfüllgewicht | 190 mg |

Herstellungsverfahren:

Der Wirkstoff wird mit der Cellulose intensiv gemischt. Dieser Mischung wird danach die Natriumcarboxymethylstärke, der Talk und das Magnesiumstearat zugemischt, und das Gemisch in Kapseln geeigneter Grösse abgefüllt.

Beispiel F

| Injektionslösung | |
|---|---|
| | 1 ml |
| 2-[2-[[3-(2-Benzimidazolyl)propyl]methylamino]äthyl]-6-fluor-1,2,3,4-tetrahydro-1α-isopropyl-2α-naphthylmethoxyacetathydrochlorid | 8 mg |
| Natriumchlorid krist. rein | 8,5 mg |
| Wasser zu Injektionszwecken ad | 1 ml |

Beispiel G

Wenn man nach den in den Beispielen A-F beschriebenen Verfahren arbeitet, können aus den folgenden, ebenfalls bevorzugten Verbindungen und ihren pharmazeutisch verwendbaren Salzen Tabletten, Kapseln und Injektionspräparate hergestellt werden:

- [1S,2S]-2-[2-[[3-(2-Benzyimidazolyl)propyl]methylamino]äthyl]-6-fluor-1,2,3,4-tetrahydro-1-isopropyl-2-naphthylmethoxyacetat-hydrochlorid.
- [1S,2S]-2-[2-[[5-(2-Benzthiazolyl)pentyl]methylamino]äthyl]-6-fluor-1,2,3,4-tetrahydro-1-isopropyl-2-naphthylmethoxyacetat-hydrochlorid.

**Patentansprüche**

**Patentansprüche für folgende Vertragsstaaten : AT, BE, CH, DE, FR, GB, IT, LI, LU, NL, SE**

1. Tetrahydronaphthalinderivate der allgemeinen Formel

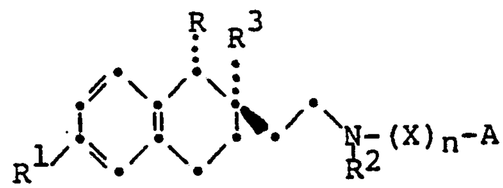

I

worin R nieder-Alkyl, $R^1$ Halogen, $R^2$ $C_1$-$C_{12}$--Alkyl, $R^3$ Hydroxy, nieder-Alkoxy, nieder-Alkylcarbonyloxy, nieder-Alkoxy-nieder-alkylcarbonyloxy, nieder--Alkylaminocarbonyloxy, Arylaminocarbonyloxy oder Aryl-nieder-alkylaminocarbonyloxy, X $C_1$-$C_{18}$-Alkylen, welches gegebenenfalls durch 1,4-Phenylen unterbrochen oder durch 1,4-Cyclohexylen unterbrochen oder verlängert sein kann, A 2-Benzimidazolyl, 1-Methyl-2-benzimidazolyl, 1-Dodecyl-2-benzimidazolyl, Benzimidazolonyl, 3-Methylbenzimidazolonyl, 3-Isopropylbenzimidazolonyl, 3-Butylbenzimidazolonyl, 3-Morpholinoäthylbenzimidazolonyl, 3-Benzylbenzimidazolonyl, 2-Pyridylmethylbenzimidazolonyl, 2-Imidazo-(4,5-c)pyridinyl, Imidazo(4,5-c)-pyridinonyl, 2-Benzthiazolyl, 2,3,4,5-Tetrahydro-4-methylbenzodiazepin-2,5-dion-1-yl, 6-Chlor-2,3,11,11a-tetrahydro-pyrrolo(2,1-c)(1,4)-benzodiazepin-5,11-dion-10-yl, 5,6-Dimethyl-2-benzimidazolyl, 1-Methyl-4,5-diphenyl-2-imidazolyläthyl oder 4,5-Diphenyl-2-imidazolyläthyl und n die Zahl 0 oder 1 bedeuten, wobei "Aryl" gegebenenfalls durch Halogen, Trifluormethyl, nieder Alkyl, nieder Alkoxy, Nitro oder Amino ein- oder mehrfach substituiertes Phenyl und "Aryl-nieder-alkyl" nieder-Alkylgruppen bedeuten, worin ein oder mehrere Wasserstoffatome durch "Aryl" ersetzt sind, und die als "nieder" bezeichneten Reste 1-6 Kohlenstoffatom aufweisen,
in Form von Racematen und optischen Antipoden, sowie N-Oxyde und pharmazeutisch verwendbare Säureadditionssalze davon.

2. Verbindungen gemäss Anspruch 1, worin R Isopropyl bedeutet.

3. Verbindungen gemäss Anspruch 1 oder 2, worin $R^3$ Hydroxy, nieder-Alkylcarbonyloxy, nieder-Alkoxy-nieder--alkylcarbonyloxy oder nieder-Alkylaminocarbonyloxy bedeutet.

4. Verbindungen gemäss Anspruch 3, worin $R^3$ Isobutyryloxy, Methoxyacetyloxy oder Butylaminocarbonyloxy bedeutet.

5. Verbindungen gemäss einem der Ansprüche 1-4, worin n die Zahl 1 bedeutet.

6. Verbindungen gemäss einem der Ansprüche 1-5, worin $R^1$ Fluor bedeutet.

7. Verbindungen gemäss einem der Ansprüche 1-6, worin $R^2$ Methyl bedeutet.

8. Verbindungen gemäss einem der Ansprüche 1-7, worin X $C_3$-$C_7$-Alkylen bedeutet.

9. Verbindungen gemäss Anspruch 8, worin X Propylen, Butylen, Pentamethylen oder Hexamethylen bedeutet.

10. Verbindungen gemäss einem der Ansprüche 1-9, worin A 2-Benzimidazolyl, 2-Benzthiazolyl, 1-Methyl-2-benzimidazolyl, 1-Dodecyl-2-benzimidazolyl, Benzimidazolonyl, 2,3,4,5-Tetrahydro-4-methylbenzodiazepin-2,5-dion-1-yl, 6-Chlor-2,3,11,11a-tetrahydro-pyrrolo[2,1-c][1,4]benzodiazepin -5,11-dion-10-yl oder 1-Methyl-4,5-diphenyl-2-imidazolyl bedeutet.

11. Verbindungen gemäss Anspruch 10, worin A 2-Benzimidazolyl oder 2-Benzthiazolyl bedeutet.

12. Verbindungen gemäss einem der Ansprüche 1-11, worin R Isopropyl, $R^3$ Hydroxy, Isobutyryloxy, Methoxyacetyloxy oder Butylaminocarbonyloxy, $R^1$ Fluor, $R^2$ Methyl, X Propylen, Butylen, Pentamethylen oder Hexamethylen, A 2-Benzimidazolyl oder 2-Benzthiazolyl und n die Zahl 1 bedeuten.

13. 2-[2-[[3-(2-Benzimidazolyl)propyl]methylamino]-äthyl]-6-fluor-1,2,3,4-tetrahydro-1α-isopropyl-2α-naphthylmethoxyacetat.

14. [1S,2S]-2-[2-[[5-(2-Benzthiazolyl)pentyl]methylamino]äthyl]-6-fluor-1,2,3,4-tetrahydro-1-isopropyl-2-naphthylmethoxyacetat.

15. [1S,2S]-2-[2-[[3-(2-Benzimidazolyl)propyl]methylamino]äthyl]-6-fluor-1,2,3,4-tetrahydro-1-isopropyl-2-naphthylmethoxyacetat.

16. Tetrahydronaphthalinderivate gemäss einem der Ansprüche 1-15 zur Anwendung als therapeutische Wirkstoffe.

17. Tetrahydronaphthalinderivte gemäss einem der Ansprüche 1-15 zur Anwendung bei der Bekämpfung bzw. Verhütung von Angina pectoris, Ischämie, Arryhthmien, Bluthochdruck und Herzinsuffizienz.

18. [1S,2S]-2-[2-[[3-(2-Benzimidazolyl)propyl]methylamino]äthyl]-6-fluor-1,2,3,4-tetrahydro-1-isopropyl-2-naphthylmethoxyacetat oder dessen Racemat zur Anwendung als therapeutischer Wirkstoff.

19. [1S,2S]-2-[2-[[3-(2-Benzimidazolyl)propyl]methylamino]äthyl]-6-fluor-1,2,3,4-tetrahydro-1-isopropyl-2-naphthylmethoxyacetat oder dessen Racemat zur Anwendung bei der Bekämpfung bzw. Verhütung von Angina pectoris, Ischämie, Arryhthmien, Bluthochdruck und Herzinsuffizienz.

20. Verfahren zur Herstellung einer Verbindung gemäss einem der Ansprüche 1-15, dadurch gekennzeichnet, dass man

a) zur Herstellung von Verbindungen der Formel I, worin $R^3$ Hydroxy oder nieder-Alkoxy bedeutet und die übrigen Symbole die in Anspruch 1 angegebene Bedeutung besitzen, eine Verbindung der allgemeinen Formel

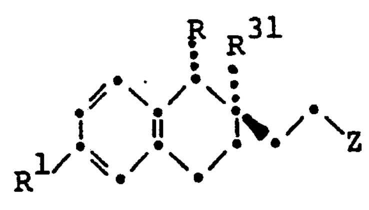

II

worin $R^{31}$ Hydroxy oder nieder-Alkoxy und Z eine Abgangsgruppe bedeuten und R und $R^1$ die in Anspruch 1 angegebene Bedeutung besitzen, mit einem Amin der allgemeinen Formel

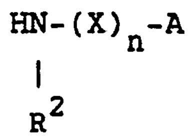 III

worin $R^2$, A, X und n die in Anspruch 1 angegebene Bedeutung besitzen, umsetzt, oder

b) zur Herstellung von Verbindungen der Formel I, worin $R^3$ nieder-Alkylcarbonyloxy oder nieder-Alkoxy-nieder-alkylcarbonyloxy bedeutet und die übrigen Symbole die in Anspruch 1 angegebene Bedeutung besitzen, eine Verbindung der allgemeinen Formel

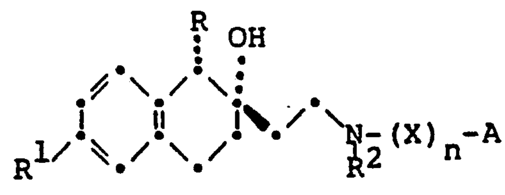 Ia

worin R, $R^1$, $R^2$, A, X und n die in Anspruch 1 angegebene Bedeutung besitzen, mit einem eine nieder-Alkylcarbonyl- oder nieder-Alkoxy-nieder-alkylcarbonylgruppe abgebenden Acylierungsmittel umsetzt, oder

c) zur Herstellung von Verbindungen der Formel I, worin $R^3$ nieder-Alkylaminocarbonyloxy, Arylaminocarbonyloxy oder Aryl-nieder-alkylaminocarbonyloxy bedeutet und die übrigen Symbole die in Anspruch 1 angegebene Bedeutung besitzen, eine Verbindung der obigen Formel Ia mit einem nieder-Alkyl-, Aryl- oder Aryl-nieder-alkyl-isocyanat umsetzt, und erwünschtenfalls

d) eine erhaltene Verbindung zum entsprechenden N-Oxyd oxydiert, und/oder

e) ein erhaltenes Racemat in die optischen Antipoden auftrennt, und/oder

f) eine erhaltene Verbindung in ein pharmazeutisch verwendbares Säureadditionssalz überführt.

**21.** Arzneimittel, enthaltend ein Tetrahydronaphthalinderivat gemäss einem der Ansprüche 1-15 und ein therapeutisch inertes Excipiens.

**22.** Mittel zur Bekämpfung bzw. Verhütung von Angina pectoris, Ischämie, Arryhthmien, Bluthochdruck und Herzinsuffizienz, enthaltend ein Tetrahydronaphthalinderivat gemäss einem der Ansprüche 1-15 und ein therapeutisch inertes Excipiens.

**23.** Arzneimittel, enthaltend [1S,2S]-2-[2-[[3-(2-Benzimidazolyl)propyl]methylamino]äthyl]-6-fluor-1, ,3,4-tetrahydro-1-isopropyl-2-naphthylmethoxyacetat oder dessen Racemat und ein therapeuutisch inertes Excipiens.

**24.** Mittel zur Bekämpfung bzw. Verhütung von Angina pectoris, Ischämie, Arryhthmien, Bluthochdruck und Herzinsuffizienz, enthaltend [1S,2S]-2-[2-[[3-(2-Benzimidazolyl)propyl]methylamino]äthyl]-6-fluor-1, ,3,4-tetrahydro-1-isopropyl-2-naphthylmethoxyacetat oder dessen Racemat und ein therapeuutisch inertes Excipiens.

**25.** Verwendung eines Tetrahydronaphthalinderivates gemäss einem der Ansprüche 1-15 zur Herstellung von Mitteln gegen Angina pectoris, Ischämie, Arryhthmien, Bluthochdruck und/oder Herzinsuffizienz.

**26.** Verwendung von [1S,2S]-2-[2-[[3-(2-Benzimidazolyl)propyl]methylamino]äthyl]-6-fluor-1,2,3,4-tetrahydro-1-isopropyl-2-naphthylmethoxyacetat oder von dessen Racemat nach Anspruch 25.

**Patentansprüche für folgende Vertragsstaaten : ES, GR**

**1.** Verfahren zur Herstellung von Tetrahydronaphthalinderivaten der allgemeinen Formel

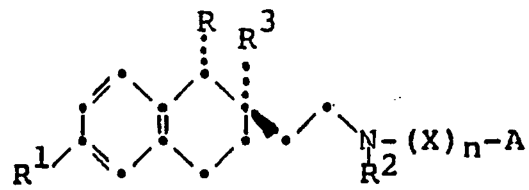

I

worin R nieder-Alkyl, $R^1$ Halogen, $R^2$ $C_1$-$C_{12}$--Alkyl, $R^3$ Hydroxy, nieder-Alkoxy, nieder-Alkylcarbonyloxy, nieder-Alkoxy-nieder-alkylcarbonyloxy, nieder--Alkylaminocarbonyloxy, Arylaminocarbonyloxy oder Aryl--nieder-alkylaminocarbonyloxy, X $C_1$-$C_{18}$-Alkylen, welches gegebenenfalls durch 1,4-Phenylen unterbrochen oder durch 1,4-Cyclohrexylen unterbrochen oder verlängert sein kann, A 2-Benzimidazolyl, 1-Methyl-2-benzimidazolyl, 1-Dodecyl-2-benzimidazolyl, Benzimidazolonyl, 3-Methylbenzimidazolonyl, 3-Isopropylbenzimidazolonyl, 3-Butylbenzimidazolonyl, 3-Morpholinoäthylbenzimidazolonyl, 3-Benzylbenzimidazolonyl, 2-Pyridylmethylbenzimidazolonyl, 2-Imidazo-(4,5-c)pyridinyl, Imidazo(4,5-c)-pyridinonyl, 2-Benzthiazolyl, 2,3,4,5-Tetrahydro-4-methylbenzodiazepin-2,5-dion-1-yl, 6-Chlor-2,3,11,11a-tetrahydro-pyrrolo(2,1-c)(1,4)-benzodiazepin-5,11-dion-10-yl, 5,6-Dimethyl-2-benzimidazolyl, 1-Methyl-4,5-diphenyl-2-imidazolyläthyl oder 4,5-Diphenyl-2-imidazolyläthyl und n die Zahl 0 oder 1 bedeuten, wobei "Aryl" gegebenenfalls durch Halogen, Trifluormethyl, nieder Alkyl, nieder Alkoxy, Nitro oder Amino eine- oder mehrfach substituiertes Phenyl und "Aryl-nieder-alkyl" nieder-Alkylgruppen bedeuten, worin ein oder mehrere Wasserstoffatome durch "Aryl" ersetzt sind, und die als "nieder" bezeichneten Reste 1-6 Kohlenstoffatom aufweisen,
in Form von Racematen und optischen Antipoden. sowie N-Oxyde und pharmazeutisch verwendbare Säureadditionssalze davon, dadurch gekennzeichnet, dass man

a) zur Herstellung von Verbindungen der Formel I, worin $R^3$ Hydroxy oder nieder-Alkoxy bedeutet und die übrigen Symbole die oben angegebene Bedeutung besitzen, eine Verbindung der allgemeinen Formel

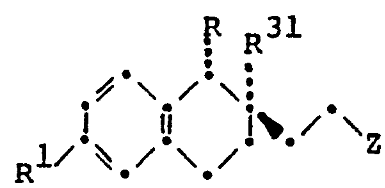

II

worin $R^{31}$ Hydroxy oder nieder-Alkoxy und Z eine Abgangsgruppe bedeuten und R und $R^1$ die oben angegebene Bedeutung besitzen,
mit einem Amin der allgemeinen Formel

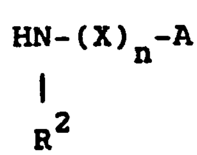

III

worin $R^2$, A, X und n die oben angegebene Bedeutung besitzen,
umsetzt, oder
b) zur Herstellung von Verbindungen der Formel I, worin $R^3$ nieder-Alkylcarbonyloxy oder nieder-Alkoxy-nieder-alkylcarbonyloxy bedeutet und die übrigen Symbole die oben angegebene Bedeutung besitzen, eine Verbindung der allgemeinen Formel

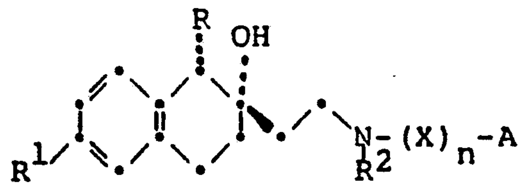

Ia

worin R, $R^1$, $R^2$, A, X und n die oben 1 angegebene Bedeutung besitzen, mit einem eine nieder-Alkylcarbonyl- oder nieder-Alkoxy-nieder-alkylcarbonylgruppe abgebenden Acylierungsmittel umsetzt, oder

c) zur Herstellung von Verbindungen der Formel I, worin $R^3$ nieder-Alkylaminocarbonyloxy, Arylaminocarbonyloxy oder Aryl-nieder-alkylaminocarbonyloxy bedeutet und die übrigen Symbole die oben angegebene Bedeutung besitzen, eine Verbindung der obigen Formel Ia mit einem nieder-Alkyl-, Aryl- oder Aryl-nieder-alkyl-isocyanat umsetzt, und erwünschtenfalls

d) eine erhaltene Verbindung zum entsprechenden N-Oxyd oxydiert, und/oder

e) ein erhaltenes Racemat in die optischen Antipoden auftrennt, und/oder

f) eine erhaltene Verbindung in ein pharmazeutisch verwendbares Säureadditionssalz überführt.

2. Verfahren gemäss Anspruch 1, worin R Isopropyl bedeutet.

3. Verfahren gemäss Anspruch 1 oder 2, worin $R^3$ Hydroxy, nieder-Alkylcarbonyloxy, nieder-Alkoxy-nieder-alkylcarbonyloxy oder nieder-Alkylaminocarbonyloxy bedeutet.

4. Verfahren gemäss Anspruch 3, worin $R^3$ Isobutyryloxy, Methoxyacetyloxy oder Butylaminocarbonylxoy bedeutet.

5. Verfahren gemäss einem der Ansprüche 1-4, worin n die Zahl 1 bedeutet.

6. Verfahren gemäss einem der Ansprüche 1-5, worin $R^1$ Fluor bedeutet.

7. Verfahren gemäss einem der Ansprüche 1-6, worin $R^2$ Methyl bedeutet.

8. Verfahren gemäss einem der Ansprüche 1-7, worin X $C_3$-$C_7$-Alkylen bedeutet.

9. Verfahren gemäss Anspruch 8, worin X Propylen, Butylen, Pentamethylen oder Hexamethylen bedeutet.

10. Verfahren gemäss einem der Ansprüche 1-9, worin A 2-Benzimidazolyl, 2-Benzthiazolyl, 1-Methyl-2-benzimidazolyl, 1-Dodecyl-2-benzimidazolyl, Benzimidazolonyl, 2,3,4,5-Tetrahydro-4-methylbenzodiazepin-2,5-dion-1-yl, 6-Chlor-2,3,11,11a -tetrahydro-pyrrolo[2,1-c][1,4]benzodiazepin-5,11-dion-10-yl oder 1-Methyl-4,5-diphenyl-2-imidazolyl bedeutet.

11. Verfahren gemäss Anspruch 10, worin A 2-Benzimidazolyl oder 2-Benzthiazolyl bedeutet.

12. Verfahren gemäss einem der Ansprüche 1-11, worin R Isopropyl, $R^3$ Hydroxy, Isobutyryloxy, Methoxyacetyloxy oder Butylaminocarbonyloxy, $R^1$ Fluor, $R^2$ Methyl, X Propylen, Butylen, Pentamethylen oder Hexamethylen, A 2-Benzimidazolyl oder 2-Benzthiazolyl und n die Zahl 1 bedeuten.

13. Verfahren gemäss Anspruch 1, dadurch gekennzeichnet, dass man 2-[2-[[3-(2-Benzimidazolyl)propyl]-methylamino]äthyl]-6-fluor-1,2,3,4-te rahydro-1α-isopropyl-2α-naphthylmethoxyacetat herstellt.

14. Verfahren gemäss Anspruch 1, dadurch gekennzeichnet, dass man [1S,2S]-2-[2-[[5-(2-Benzthiazolyl)-pentyl]methylamino]äthyl]-6-fluor-1,2 3,4-tetrahydro-1-isopropyl-2-naphthylmethoxyacetat herstellt.

15. Verfahren gemäss Anspruch 1, dadurch gekennzeichnet,.dass man [1S,2S]-2-[2-[[3-(2-Benzimidazolyl)-propyl]methylamino]äthyl]-6-fluor-1, ,3,4-tetrahydro-1-isopropyl-2-naphthylmethoxyacetat herstellt.

16. Verwendung eines Tetrahydronaphthalinderivates gemäss einem der Ansprüche 1-15 zur Herstellung von Mitteln gegen Angina pectoris, Ischämie, Arryhthmien, Bluthochdruck und/oder Herzinsuffizienz.

**Claims**

**Claims for the following Contracting States : AT, BE, CH, DE, FR, GB, IT, LI, LU, NL, SE**

1. Tetrahydronaphthalene derivatives of the general formula

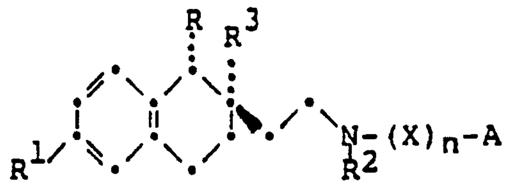

I

wherein R signifies lower-alkyl, $R^1$ signifies halogen, $R^2$ signifies $C_1$-$C_{12}$-alkyl, $R^3$ signifies hydroxy, lower-alkoxy, lower-alkylcarbonyloxy, lower-alkoxy-lower-alkylcarbonyloxy, lower-alkylaminocarbonyloxy, arylaminocarbonyloxy or aryl-lower-alkylaminocarbonyloxy, X signifies $C_1$-$C_{18}$-alkylene which optionally can be interrupted by 1,4-phenylene or interrupted or lengthened by 1,4-cyclohexylene, A signifies 2-benzimidazolyl, 1-methyl-2-benzimidazolyl, 1-dodecyl-2-benzimidazolyl, benzimidazolonyl, 3-methylbenzimidazolonyl, 3-isopropylbenzimidazolonyl,3-butylbenzimidazolonyl, 3-morpholinoethylbenzimidazolonyl, 3-benzylbenzimidazolonyl, 2-pyridylmethylbenzimidazolonyl, 2-imidazo-(4,5-c)pyridinyl, imidazo(4,5-c)pyridinonyl, 2-benzthiazolyl, 2,3,4,5-tetrahydro-4-methylbenzodiazepine--2,5-dion-1-yl, 6-chloro-2,3,11,11a-tetrahydro-pyrrolo-(2,1-c)(1,4)benzodiazepine-5,11-dion-10-yl, 5,6-dimethyl-2-benzimidazolyl, 1-methyl-4,5-diphenyl -2-imidazolylethyl or 4,5-diphenyl-2-imidazolylethyl and n signifies the number 0 or 1, whereby "aryl" signifies phenyl optionally mono- or multiply-substituted by halogen, trifluoromethyl,lower alkyl,lower alkoxy, nitro or amino and "aryl-lower-alkyl" signifies lower alkyl groups in which one or more hydrogen atoms is/are replaced by "aryl", and the residues denoted as "lower" have 1-6 carbon atoms,
in the form of racemates and optical antipodes, as well as N-oxides and pharmaceutically usable acid addition salts thereof.

2. Compounds in accordance with claim 1, wherein R signifies isopropyl.

3. Compounds in accordance with claim 1 or 2, wherein $R^3$ signifies hydroxy, lower-alkylcarbonyloxy, lower-alkoxy-lower-alkylcarbonyloxy or lower-alkylaminocarbonyloxy.

4. Compounds in accordance with claim 3, wherein $R^3$ signifies isobutyryloxy, methoxyacetyloxy or butylaminocarbonyloxy.

5. Compounds in accordance with any one of claims 1-4, wherein n signifies the number 1.

6. Compounds in accordance with any one of claims 1-5, wherein $R^1$ signifies fluorine.

7. Compounds in accordance with any one of claims 1-6, wherein $R^2$ signifies methyl.

8. Compounds in accordance with any one of claims 1-7, wherein X signifies $C_3$-$C_7$-alkylene.

9. Compounds in accordance with claim 8, wherein X signifies propylene, butylene, pentamethylene or hexamethylene.

10. Compounds in accordance with any one of claims 1-9, wherein A signifies 2-benzimidazolyl, 2-benzthiazolyl, 1-methyl-2-benzimidazolyl, 1-dodecyl-2-benzimidazolyl, bezimidazolonyl, 2,3,4,5-tetrahydro-4 -methylbenzodiazepine-2,5-dion-1-yl, 6-chloro-2,3,11,11a--teirahydro-pyrrolo[2,1-c][1,4]-benzodiazepine-5,11-dion-10--yl or 1-methyl-4,5-diphenyl-2-imidazolyl.

11. Compounds in accordance with claim 10, wherein A signifies 2-benzimidazolyl or 2-benzthiazolyl.

12. Compounds in accordance with any one of claims 1-11, wherein R signifies isopropyl, $R^3$ signifies hydroxy, isobutyryloxy, methoxyacetyloxy or butylaminocarbonyloxy, $R^1$ signifies fluorine, $R^2$ signifies methyl, X signifies propylene, butylene, pentamethylene or hexamethylene, A signifies 2-benzimidazolyl or 2-benzthiazolyl and n signifies the number 1.

13. 2-[2-[[3-(2-Benzimidazolyl)propyl]methylamino]-ethyl]-6-fluoro-1,2,3,4-tetrahydro-1α-isopropyl-2α--naphthyl methoxyacetate.

14. [1S,2S]-2-[2-[[5-(2-Benzthiazolyl)pentyl]methylamino]ethyl]-6-fluoro-1,2,3,4-tetrahydro-1-isopropyl-2--naphthyl methoxyacetate.

15. [1S.2S]-2-[2-[[3-(2-Benzimidazolyl)propyl]-methylamino]ethyl]-6-fluoro-1,2,3,4-tetrahydro-1--isopropyl-2-naphthyl methoxyacetate.

16. Tetrahydronaphthalene derivatives in accordance with any one of claims 1-15 for use as therapeutically active substances.

17. Tetrahydronaphthalene derivatives in accordance with any one of claims 1-15 for use in the control or prevention of angina pectoris, ischaemia, arryhthmias, high blood pressure and cardiac insufficiency.

18. [1S,2S]-2-[2-[[3-(2-Benzimidazolyl)propyl]-methylamino]ethyl]-6-fluoro-1,2,3,4-tetrahydro-1--isopropyl-2-naphthyl methoxyacetate or its racemate for use as a therapeutically active substance.

19. [1S,2S]-2-[2-[[3-(2-Benzimidazolyl)propyl]-methylamino]ethyl]-6-fluoro-1,2,3,4-tetrahydro-1--isopropyl-2-naphthyl methoxyacetate or its racemate for use in the control or prevention of angina pectoris, ischaemia, arryhthmias, high blood pressure and cardiac insufficiency.

20. A process for the manufacture of a compound in accordance with any one of claims 1-15, characterized by

a) for the manufacture of compounds of formula I in which $R^3$ signifies hydroxy or lower-alkoxy and the remaining symbols have the significance given in claim 1, reacting a compound of the general formula

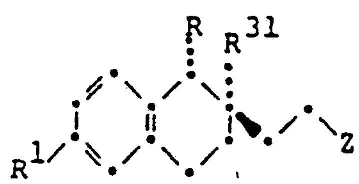

II

wherein $R^{31}$ signifies hydroxy or lower-alkoxy and Z signifies a leaving group and R and $R^1$ have the significance given in claim 1,
with an amine of the general formula

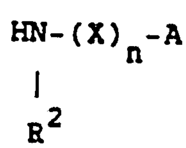

III

wherein $R^2$, A, X and n have the significance given in claim 1,
or

b) for the manufacture of compounds of formula I in which $R^3$ signifies lower-alkylcarbonyloxy or lower-alkoxy-lower-alkylcarbonyloxy and the remaining symbols have the significance given in claim 1, reacting a compound of the general formula

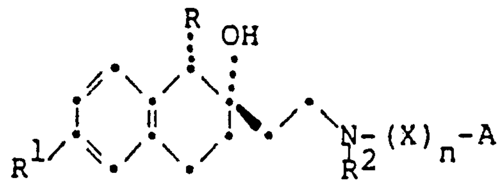

Ia

wherein R, $R^1$, $R^2$, A, X and n have the significance given in claim 1,
with an acylating agent yielding a lower-alkylcarbonyl or lower-alkoxy-lower-alkylcarbonyl group, or
c) for the manufacture of compounds of formula I in which $R^3$ signifies lower-alkylaminocarbonyloxy, arylaminocarbonyloxy or aryl-lower-alkylaminocarbonyloxy and the remaining symbols have the significance given in claim 1, reacting a compound of formula Ia above with a lower-alkyl, aryl or aryl-lower-alkyl isocyanate, and, if desired,
d) oxidizing a compound obtained to the corresponding N-oxide, and/or
e) separating a racemate obtained into the optical antipodes, and/or
f) converting a compound obtained into a pharmaceutically usable acid addition salt.

**21.** A medicament containing a tetrahydronaphthalene derivative in accordance with any one of claims 1-15 and a therapeutically inert excipient.

**22.** A medicament for the control or prevention of angina pectoris, ischaemia, arrythmias, high blood pressure and cardiac insufficiency, containing a tetrahydronaphthalene derivative in accordance with any one of claims 1-15 and a therapeutically inert excipient.

**23.** A medicament containing [1S,2S]-2-[2-[[3-(2-benzimidazolyl)propyl]methylamino]ethyl)-6-fluoro-1,2,3,4--tetrahydro-1-isopropyl-2-naphthyl methoxyacetate or its racemate and a therapeutically inert excipient.

**24.** A medicament for the control or prevention of angina pectoris, ischaemia, arrythmias, high blood pressure and cardiac insufficiency, containing [1S,25]-2-[2--[[3-(2-benzimidazolyl)propyl]methylamino]-ethyl]-6-fluoro--1,2,3,4-tetrahydro-1-isopropyl-2-naphthyl methoxyacetate or its racemate and a therapeutically inert excipient.

**25.** The use of a tetrahydronaphthalene derivative in accordance with any one of claims 1-15 for the manufacture of medicaments against angina pectoris, ischaemia, arrhythmias, high blood pressure and/or cardiac insufficiency.

**26.** The use of [1S,2S]-2-[2-[[3-(2-benzimidazolyl)-propyl]methylamino]ethyl]-6-fluoro-1,2,3,4-tetrahydro-1--isopropyl-2-naphthyl methoxyacetate or its racemate according to claim 25.

**Claims for the following Contracting States: ES, GR**

**1.** A process for the manufacture of tetrahydronaphthalene derivatives of the general formula

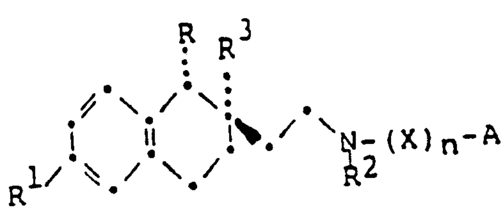

I

wherein R signifies lower-alkyl, $R^1$ signifies halogen, $R^2$ signifies $C_1$-$C_{12}$-alkyl, $R^3$ signifies hydroxy,

lower-alkoxy, lower-alkylcarbonyloxy, lower-alkoxy-lower-alkylcarbonyloxy, lower-alkylaminocarbonyloxy, arylaminocarbonyloxy or aryl-lower-alkylaminocarbonyloxy, X signifies $C_1$-$C_{18}$-alkylene which optionally can be interrupted by 1,4-phenylene or interrupted or lengthened by 1,4-cyclohexylene, A signifies 2-benzimidazolyl, 1-methyl-2-benzimidazolyl, 1-dodecyl-2-benzimidazolyl, benzimidazolonyl, 3-methylbenzimidazolonyl, 3-isopropylbenzimidazolonyl, 3-butylbenzimidazolonyl, 3-morpholinoethylbenzimidazolonyl, 3-benzylbenzimidazolonyl, 2-pyridylmethylbenzimidazolonyl, 2-imidazo-(4,5-c)pyridinyl, imidazo(4,5-c)pyridinonyl, 2-benzthiazolyl, 2,3,4,5-tetrahydro-4-methylbenzodiazepine--2,5-dion-l-yl, 6-chloro-2,3,11,11a-tetrahydropyrrolo(2,1-c)(1,4)benzodiazepine,-5,11-dion-10-yl, 5,6-dimethyl-2-benzimidazolyl, 1-methyl-4,5-diphenyl-2-imidazolylethyl or 4,5-diphenyl-2-imidazolylethyl and n signifies the number 0 or 1, whereby "aryl" signifies phenyl optionally mono- or multiply-substituted by halogen, trifluoromethyl, lower alkyl, lower alkoxy, nitro or amino and "aryl-lower-alkyl" signifies lower alkyl groups in which one or more hydrogen atoms is/are replaced by "aryl", and the residues denoted as "lower" have 1-6 carbon atoms,

in the form of racemates and optical antipodes, as well as N-oxides and pharmaceutically usable acid addition salts thereof, characterized by

a) for the manufacture of compounds of formula I in which $R^3$ signifies hydroxy or lower-alkoxy and the remaining symbols have the significance given above, reacting a compound of the general formula

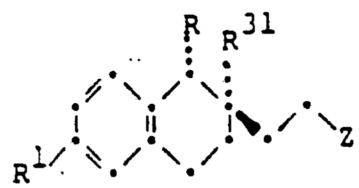

II

wherein $R^{31}$ signifies hydroxy or lower-alkoxy and Z signifies a leaving group and R and $R^1$ have the significance given above,

with an amine of the general formula

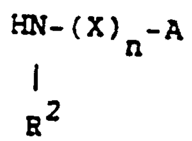

III

wherein $R^2$, A, X and n have the significance given above,

or

b) for the manufacture of compounds of formula I in which $R^3$ signifies lower-alkylcarbonyloxy or lower-alkoxy-lower-alkylcarbonyloxy and the remaining symbols have the significance given above, reacting a compound of the general formula

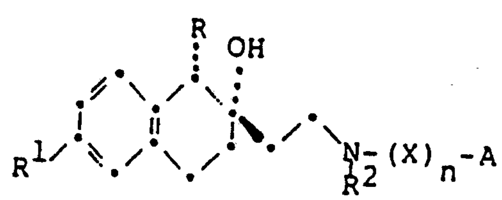

Ia

wherein R, $R^1$, $R^2$, A, X and n have the significance given above,

with an acylating agent yielding a lower-alkylcarbonyl or lower-alkoxy-lower-alkylcarbonyl group, or

c) for the manufacture of compounds of formula I in which $R^3$ signifies lower-alkylaminocarbonyloxy, arylaminocarbonyloxy or aryl-lower-alkylaminocarbonyloxy and the remaining symbols have the significance given above, reacting a compound of formula Ia above with a lower-alkyl, aryl or aryl-lower-alkyl isocyanate, and, if desired,

d) oxidizing a compound obtained to the corresponding N-oxide, and/or

e) separating a racemate obtained into the optical antipodes, and/or
f) converting a compound obtained into a pharmaceutically usable acid addition salt.

**2.** A process in accordance with claim 1, wherein R signifies isopropyl.

**3.** A process in accordance with claim 1 or 2, wherein $R^3$ signifies hydroxy, lower-alkylcarbonyloxy, lower-alkoxy-lower-alkylcarbonyloxy or lower-alkylaminocarbonyloxy.

**4.** A process in accordance with claim 3, wherein $R^3$ signifies isobutyryloxy, methoxyacetyloxy or butylaminocarbonyloxy.

**5.** A process in accordance with any one of claims 1-4, wherein n signifies the number 1.

**6.** A process in accordance with any one of claims 1-5, wherein $R^1$ signifies fluorine.

**7.** A process in accordance with any one of claims 1-6, wherein $R^2$ signifies methyl.

**8.** A process in accordance with any one of claims 1-7, wherein X signifies $C_3$-$C_7$-alkylene.

**9.** A process in accordance with claim 8, wherein X signifies propylene, butylene, pentamethylene or hexamethylene.

**10.** A process in accordance with any one of claims 1-9, wherein A signifies 2-benzimidazolyl, 2-benzthiazolyl, 1-methyl-2-benzimidazolyl, 1-dodecyl-2-benzimidazolyl, benzimidazolonyl, 2,3,4,5-tetrahydro-4-methylbenzodiazepine-2,5-dion-1-yl, 6-chloro-2,3,11,11a-tetrahydro-pyrrolo[2,1-c][1,4]-benzodiazepine-5,11-dion-10-yl or 1-methyl-4,5-diphenyl-2-imidazolyl.

**11.** A process in accordance with claim 10, wherein A signifies 2-benzimidazolyl or 2-benzthiazolyl.

**12.** A process in accordance with any one of claims 1-11, wherein R signifies isopropyl, $R^3$ signifies hydroxy, isobutyryloxy, methoxyacetyloxy or butylaminocarbonyloxy, $R^1$ signifies fluorine, $R^2$ signifies methyl, X signifies propylene, butylene, pentamethylene or hexamethylene, A signifies 2-benzimidazolyl or 2-benzthiazolyl and n signifies the number 1.

**13.** A process in accordance with claim 1, wherein 2-[2-[[3-(2-benzimidazolyl)propyl]methylamino]ethyl]-6-fluoro-1,2,3,4-tetrahydro-1$\alpha$-isopropyl-2$\alpha$-naphthyl methoxyacetate is manufactured.

**14.** A process in accordance with claim 1, wherein [1S,2S]-2-[2-[[5-(2-benzthiazolyl)pentyl]methylamino]-ethyl]-6-fluoro-1,2,3,4-tetrahydro-1-isopropyl-2-naphthyl methoxyacetate is manufactured.

**15.** A process in accordance with claim 1, wherein [1S,2S]-2-[2-[[3-(2-benzimidazolyl)propyl]methylamino]-ethyl] -6-fluoro-1,2,3,4-tetrahydro-1-isopropyl-2-naphthyl methoxyacetate is manufactured.

**16.** The use of a tetrahydronaphthalene derivative in accordance with any one of claims 1-15 for the manufacture of medicaments against angina pectoris, ischaemia, arrhythmias, high blood pressure and/or cardiac insufficiency.

**Revendications**
**Revendications pour les Etats contractants suivants : AT, BE, CH, DE, FR, GB, IT, LI, LU, NL, SE**

**1.** Dérivés du tétrahydronaphtalène, de formule générale

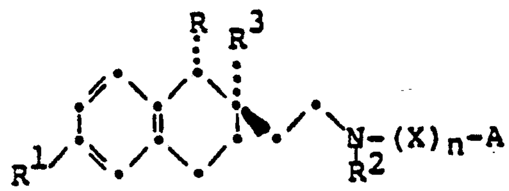

I

dans laquelle R représente un groupe alkyle inférieur, $R^1$ représente un halogène, $R^2$ représente un groupe alkyle en C 1-C 12, $R^3$ représente un groupe hydroxy, alcoxy inférieur, (alkyle inférieur)-carbonyloxy, (alcoxy inférieur)-(alkyle inférieur)-carbonyloxy, (alkyle inférieur)-aminocarbonyloxy, arylaminocarbonyloxy ou aryl-(alkyle inférieur)-aminocarbonyloxy, X représente un groupe alkylène en C 1-C 18 éventuellement interrompu par un groupe 1,4-phénylène ou interrompu ou prolongé par un groupe 1,4-cyclohexylène, A représente un groupe 2-benzimidazolyle, 1-méthyl-2-benzimidazolyle, 1-dodécyl-2-benzimidazolyle, benzimidazolonyle, 3-méthylbenzimidazolonyle, 3-isopropylbenzimidazolonyle, 3-butylbenzimidazolonyle, 3-morpholinoéthylbenzimidazolonyle, 3-benzylbenzimidazolonyle, 2-pyridylméthylbenzimidazolonyle, 2-imidazo(4,5-c)pyridinyle, imidazo(4,5-c)pyridinonyle, 2-benzothiazolyle, 2,3,4,5-tétrahydro-4-méthylbenzodiazépine-2,5-dione-1-yle, 6-chloro-2,3,11,11a-tétrahydropyrrolo(2,1-c)-(1,4)benzodiazépine-5,11-dione-10-yle, 5,6-diméthyl-2-benzimidazolyle, 1-méthyl-4,5-diphényl-2-imidazolyléthyle ou 4,5-diphényl-2-imidazolyléthyle, et n est égal à 0 ou 1, les groupes"aryle"en question consistant en groupes phényle éventuellement mono- ou polysubstitués par des halogènes, des groupes trifluorométhyle, alkyle inférieurs, alcoxy inférieurs, nitro ou amino, les groupes "aryl-alkyle inférieurs" consistant en groupes alkyle inférieurs dans lesquels un ou plusieurs atomes d'hydrogène sont remplacés par des groupes aryle, et l'expression "inférieurs" s'appliquant à des groupes qui contiennent de 1 à 6 atomes de carbone,

à l'état de racémates et d'antipodes optiques, ainsi que leurs N-oxydes et leurs sels formés par addition avec des acides acceptables pour l'usage pharmaceutique.

2. Composés selon la revendication 1, dans lesquels R représente un groupe isopropyle.

3. Composés selon la revendication 1 ou 2, dans lesquels $R^3$ représente un groupe hydroxy, (alkyle inférieur)-carbonyloxy, (alcoxy inférieur)-(alkyle inférieur)carbonyloxy ou (alkyle inférieur)-aminocarbonyloxy.

4. Composés selon la revendication 3, dans lesquels $R^3$ représente un groupe isobutyryloxy, méthoxyacétyloxy ou butylaminocarbonyloxy.

5. Composés selon l'une des revendications 1 à 4, dans lesquels n est égal à 1.

6. Composés selon l'une des revendications 1 à 5, dans lesquels $R^1$ représente le fluor.

7. Composés selon l'une des revendications 1 à 6, dans lesquels $R^2$ représente un groupe méthyle.

8. Composés selon l'une des revendications 1 à 7, dans lesquels X représente un groupe alkylène en C 3-C 7.

9. Composés selon la revendication 8, dans lesquels X représente un groupe propylène, butylène, pentaméthylène ou hexaméthylène.

10. Composés selon l'une des revendications 1 à 9, dans lesquels A représente un groupe 2-benzimidazolyle, 2-benzothiazolyle, 1-méthyl-2-benzimidazolyle, 1-dodécyl-2-benzimidazolyle, benzimidazolonyle, 2,3,4,5-tétrahydro-4-méthylbenzodiazépine-2,5-dione-1-yle, 6-chloro-2,3,11,11a-tétrahydro-pyrrolo[2,1-c][1,4]benzodiazépine-5,11-dione-10-yle ou 1-méthyl-4,5-diphényl-2-imidazolyle.

11. Composés selon la revendication 10, dans lesquels A représente un-groupe 2-benzimidazolyle ou 2-benzothiazolyle.

12. Composés selon l'une des revendications 1 à 11, dans lesquels R représente un groupe isopropyle, $R^3$

un groupe hydroxy, isobutyryloxy, méthoxyacétyloxy ou butylaminocarbonyloxy, $R^1$ représente le fluor, $R^2$ un groupe méthyle, X un groupe propylène, butylène, pentaméthylène ou hexaméthylène, A un groupe 2-benzimidazolyle ou 2-benzothiazolyle et n est égal à 1.

**13.** Le méthoxyacétate de 2-[2-[[3-(2-benzimidazolyl)propyl]méthylamino]éthyl]-6-fluoro-1,2,3,4-tétrahydro-1 alpha-isopropyl-2 alpha-naphtyle.

**14.** Le méthoxyacétate de [1S,2S]-2-[2-[[5-(2-benzothiazolyl)pentyl]méthylamino]éthyl]-6-fluoro-1,2,3,4-tétrahydro-1-isopropyl-2-naphtyle.

**15.** Le méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle.

**16.** Dérivés du tétrahydronaphtalène selon une des revendications 1 à 15, pour l'utilisation en tant que substances actives thérapeutiques.

**17.** Dérivés du tétrahydronaphtalène selon une des revendications 1 à 15, pour l'utilisation dans le traitement et la prévention de l'angine de poitrine, des ischémies, des arythmies, de l'hypertension sanguine et de l'insuffisance cardiaque.

**18.** Le méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle ou son racémate pour l'utilisation en tant que substances actives thérapeutiques.

**19.** Le méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle ou son racémate pour l'utilisation dans le traitement et la prévention de l'angine de poitrine, des ischémies, des arythmies, de l'hypertension sanguine et de l'insuffisance cardiaque.

**20.** Procédé de préparation d'un composé selon l'une des revendications 1 à 15, caractérisé en ce que :

a) pour la préparation des composés de formule I dans laquelle $R^3$ représente un groupe hydroxy ou alcoxy inférieur et les autres symboles ont les significations indiquées dans la revendication 1,
on fait réagir un composé de formule générale

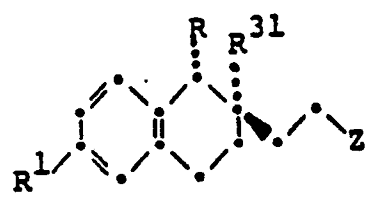

II

dans laquelle $R^{31}$ représente un groupe hydroxy ou alcoxy inférieur et Z un groupe éliminable, et R et $R^1$ ont les significations indiquées dans la revendication 1,
avec une amine de formule générale

$$HN-(X)_n-A \quad \text{III}$$
$$|$$
$$R^2$$

dans laquelle $R^2$, A, X et n ont les significations indiquées dans la revendication 1,
ou bien

b) pour la préparation des composés de formule I dans laquelle $R^3$ représente un groupe (alkyle inférieur)-carbonyloxy ou (alcoxy inférieur)-(alkyle inférieur)-carbonyloxy et les autres symboles ont

les significations indiquées dans la revendication 1, on fait réagir un composé de formule générale

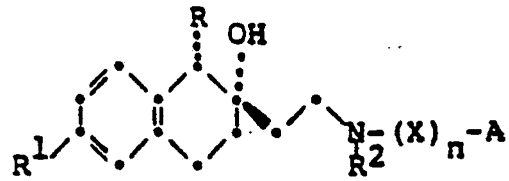

Ia

dans laquelle R, $R^1$, $R^2$, A, X et n ont les significations indiquées dans la revendication 1, avec un agent acylant apportant un groupe (alkyle inférieur)-carbonyle ou (alcoxy inférieur)-(alkyle inférieur)-carbonyle, ou bien

c) pour préparer les composés de formule I dans laquelle $R^3$ représente un groupe (alkyle inférieur)-aminocarbonyloxy, arylaminocarbonyloxy ou aryl-(alkyle inférieur)-aminocarbonyloxy et les autres symboles ont les significations indiquées dans la revendication 1, on fait réagir un composé de formule Ia ci-dessus avec un isocyanate d'alkyle inférieur, d'aryle, ou d'aryl-alkyle inférieur, et si on le désire,

d) on oxyde un composé ainsi obtenu en le N-oxyde correspondant, et/ou

e) on résout un racémate ainsi obtenu en les antipodes optiques, et/ou

f) on convertit un composé ainsi obtenu en un sel formé par addition avec un acide acceptable pour l'usage pharmaceutique.

21. Médicament contenant un dérivé du tétrahydronaphtalène selon une des revendications 1 à 15 et un excipient inerte du point de vue thérapeutique.

22. Produit pour le traitement et la prévention de l'angine de poitrine, des ischémies, des arythmies, de l'hypertension sanguine et de l'insuffisance cardiaque, contenant un dérivé du tétrahydronaphtalène selon une des revendications 1 à 15 et un excipient inerte du point de vue thérapeutique.

23. Médicament contenant du méthoxyacétate de [1S,2S]-2-[2-[[3-(2-benzimidazolylepropyl]méthylamino]-éthyl]-6-fluoro-1,2,3,4-tétrahydro-1-isopropyl-2-naphtyle ou son racémate et un excipient inerte du point de vue thérapeutique.

24. Produit pour le traitement et la prévention de l'angine de poitrine, des ischémies, des arythmies, de l'hypertension sanguine et de l'insuffisance cardiaque, contenant du méthoxyacétate de [1S,2S]-2-[2-[-[3-(2-benzimidazolyl)propyl]méthylamino]éthyl]-6-fluoro-1,2,3,4-tétrahydro-1-isopropyl-2-naphtyle ou son racémate et un excipient inerte du point de vue thérapeutique.

25. Utilisation d'un dérivé du tétrahydronaphtalène selon une des revendications 1 à 15 pour la préparation de produits contre l'angine de poitrine, les ischémies, les arythmies, l'hypertension sanguine et l'insuffisance cardiaque.

26. Utilisation du méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle ou de son racémate selon la revendication 25.

**Revendications pour les Etats contractants suivants : ES, GR**

1. Procédé de préparation de dérivés du tétrahydronaphtalène de formule générale

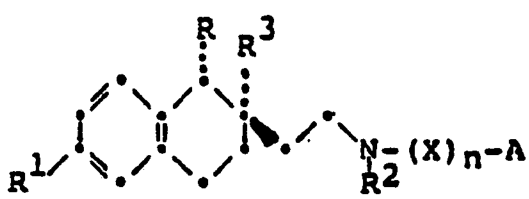

I

dans laquelle R représente un groupe alkyle inférieur, $R^1$ représente un halogène, $R^2$ un groupe alkyle

en C 1-C 12, $R^3$ un groupe hydroxy, alcoxy inférieur, (alkyle inférieur)-carbonyloxy, (alcoxy inférieur)-(alkyle inférieur)-carbonyloxy, (alkyle inférieur)-aminocarbonyloxy, arylaminocarbonyloxy ou aryl-(alkyle inférieur)-aminocarbonyloxy, X représente un groupe alkylène en C 1-C 18 éventuellement interrompu par un groupe 1,4-phénylène ou interrompu ou prolongé par un groupe 1,4-cyclohexylène, A représente un groupe 2-benzimidazolyle, 1-méthyl-2-benzimidazolyle, 1-dodécyl-2-benzimidazolyle, benzimidazolonyle, 3-méthylbenzimidazolonyle, 3-isopropylbenzimidazolonyle, 3-butylbenzimidazolonyle, 3-morpholinoéthylbenzimidazolonyle, 3-benzylbenzimidazolonyle, 2-pyridylméthylbenzimidazolonyle, 2-imidazo-(4,5-c)pyridinyle, imidazo-(4,5-c)pyridinonyle, 2-benzothiazolyle, 2,3,4,5-tétrahydro-4-méthylbenzodiazépine-2,5-dione-1-yle, 6-chloro-2,3,11,11a-tétrahydro-pyrrolo(2,1-c)(1,4)benzodiazépine-5,11-dione-10-yle, 5,6-diméthyl-2-benzimidazolyle, 1-méthyl-4,5-diphényl-2-imidazolyléthyle ou 4,5-diphényl-2-imidazolyléthyle, et n est égal à 0 ou 1, les groupes "aryle" en question consistant en groupes phényle éventuellement mono- ou poly-substitués par des halogènes, des groupes trifluorométhyle, alkyle inférieurs, alcoxy inférieurs, nitro ou amino et les groupes "aryl-alkyle inférieurs" consistant en groupes alkyle inférieurs dans lesquels un ou plusieurs atomes d'hydrogène sont remplacés par des groupes aryle, l'expression "inférieurs" s'appliquant à des groupes qui contiennent 1 à 6 atomes de carbone,

à l'état de racémates et d'antipodes optiques, et de leurs N-oxydes et sels formés par addition avec des acides acceptables pour l'usage pharmaceutique, caractérisé en ce que :

a) pour préparer les composés de formule I dans laquelle $R^3$ représente un groupe hydroxy ou alcoxy inférieur, les autres symboles ayant les significations indiquées ci-dessus, on fait réagir un composé de formule générale

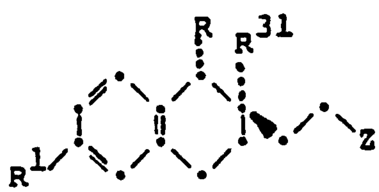

II

dans laquelle $R^{31}$ représente un groupe hydroxy ou alcoxy inférieur et Z un groupe éliminable, et R et $R^1$ ont les significations indiquées ci-dessus,

avec une amine de formule générale

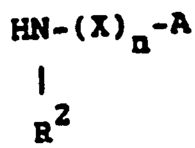

III

dans laquelle $R^2$, A, X et n ont les significations indiquées ci-dessus, ou bien

b) pour préparer les composés de formule I dans laquelle $R^3$ représente un groupe (alkyle inférieur)-carbonyloxy ou (alcoxy inférieur)-(alkyle inférieur)-carbonyloxy, les autres symboles ayant les significations indiquées ci-dessus, on fait réagir un composé de formule générale

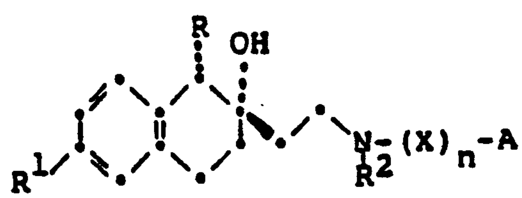

Ia

dans laquelle R, $R^1$, $R^2$, A, X et n ont les significations indiquées ci-dessus,

avec un agent acylant apportant un groupe (alkyle inférieur)-carbonyle ou (alcoxy inférieur)-(alkyle

inférieur)-carbonyle, ou bien

c) pour préparer les composés de formule I dans laquelle $R^3$ représente un groupe (alkyle inférieur)-aminocarbonyloxy, arylaminocarbonyloxy ou aryl-(alkyle inférieur)-aminocarbonyloxy, les autres symboles ayant les significations indiquées ci-dessus, on fait réagir un composé de formule Ia ci-dessus avec un isocyanate d'alkyle inférieur, d'aryle ou d'aryl-alkyle inférieur, et si on le désire,

d) on oxyde un composé ainsi obtenu en le N-oxyde correspondant, et/ou

e) on résout un racémate ainsi obtenu en les antipodes optiques, et/ou

f) on convertit un composé ainsi obtenu en un sel formé par addition avec un acide acceptable pour l'usage pharmaceutique.

2. Procédé selon la revendication 1, dans lequel R représente un groupe isopropyle.

3. Procédé selon la revendication 1 ou 2, dans lequel $R^3$ représente un groupe hydroxy, (alkyle inférieur)-carbonyloxy, (alcoxy inférieur)-(alkyle inférieur)-carbonyloxy ou (alkyle inférieur)-aminocarbonyloxy.

4. Procédé selon la revendication 3, dans lequel $R^3$ représente un groupe isobutyryloxy, méthoxyacétyloxy ou butylaminocarbonyloxy.

5. Procédé selon l'une des revendications 1 à 4, dans lequel n est égal à 1.

6. Procédé selon l'une des revendications 1 à 5, dans lequel $R^1$ représente le fluor.

7. Procédé selon l'une des revendications 1 à 6, dans lequel $R^2$ représente un groupe méthyle.

8. Procédé selon l'une des revendications 1 à 7, dans lequel X représente un groupe alkylène en C 3-C 7.

9. Procédé selon la revendication 8, dans lequel X représente un groupe propylène, butylène, pentaméthylène ou hexaméthylène.

10. Procédé selon l'une des revendications 1 à 9, dans lequel A représente un groupe 2-benzimidazolyle, 2-benzothiazolyle, 1-méthyl-2-benzimidazolyle, 1-dodécyl-2-benzimidazolyle, benzimidazolonyle, 2,3,4,5-tétrahydro-4-méthylbenzodiazépine-2,5-dione-1-yle, 6-chloro-2,3,11, 11a-tétrahydro-pyrrolo[2,1-c][1,4]benzodiazépine-5,11-dione-10-yle ou 1-méthyl-4,5-diphényl-2-imidazolyle.

11. Procédé selon la revendication 10, dans lequel A représente un groupe 2-benzimidazolyle ou 2-benzothiazolyle.

12. Procédé selon l'une des revendications 1 à 11, dans lequel R représente un groupe isopropyle, $R^3$ un groupe hydroxy, isobutyryloxy, méthoxyacétyloxy ou butylaminocarbonyloxy, $R^1$ représente le fluor, $R^2$ un groupe méthyle, X un groupe propylène, butylène, pentaméthylène ou hexaméthylène, A un groupe 2-benzimidazolyle ou 2-benzothiazolyle et n est égal à 1.

13. Procédé selon la revendication 1, caractérisé en ce que l'on prépare le méthoxyacétate de 2-[2-[[3-(2-benzimidazolyl)propyl]méthylamino]éthyl]-6-fluoro-1,2,3,4-tétrahydro-1-alpha-isopropyl-2 alphanaphtyle.

14. Procédé selon la revendication 1, caractérisé en ce que l'on prépare le méthoxyacétate de [1S,2S]-2-[2-[[5-(2-benzothiazolyl)pentyl]méthylamino]-éthyl]-6-fluoro-1,2,3,4-tétrahydro-1-isopropyl-2-naphtyle.

15. Procédé selon la revendication 1, caractérisé en ce que l'on prépare le méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle.

16. Utilisation d'un dérivé du tétrahydronaphtalène selon une des revendications 1 à 15 pour la préparation de produits contre l'angine de poitrine, les ischémies, les arythmies, l'hypertension sanguine et/ou l'insuffisance cardiaque.